(19) Europäisches Patentamt
European Patent Office
Office européen des brevets

(11) **EP 4 644 560 A2**

(12) **EUROPEAN PATENT APPLICATION**

(43) Date of publication:
**05.11.2025 Bulletin 2025/45**

(21) Application number: **25201503.7**

(22) Date of filing: **18.12.2017**

(51) International Patent Classification (IPC):
***C12P 7/64*** *(2022.01)*

(52) Cooperative Patent Classification (CPC):
**C12C 7/04; C12C 5/004; C12N 9/52; C12P 7/06; C12P 7/64; C12P 19/02; C12P 21/06; C12Y 304/21;** C12P 2203/00; Y02E 50/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2016 US 201662437340 P**

(62) Document number(s) of the earlier application(s) in accordance with Art. 76 EPC:
**17832424.0 / 3 558 026**

(71) Applicant: **International N&H Denmark ApS**
**2800 Kongens Lyngby (DK)**

(72) Inventors:
- **CRAMER, Jacob Flyvholm**
  **Højbjerg (DK)**
- **KOLKMAN, Marc Anton Bernhard**
  **Palo Alto (US)**
- **MA, Zhen**
  **Garnet Valley (US)**
- **SCHEFFERS, Martijn**
  **ET Warmond (NL)**
- **SHIPOVSKOV, Stepan**
  **Egå (DK)**
- **VAN BRUSSEL-ZWIJNEN, Marco**
  **PV Lemele (NL)**
- **YU, Shukun**
  **Malmo (SE)**

(74) Representative: **Mewburn Ellis LLP**
**Aurora Building**
**Counterslip**
**Bristol BS1 6BX (GB)**

Remarks:
•The complete document including Reference Table(s) and the Sequence Listing(s) can be downloaded from the EPO website
•This application was filed on 10.09.2025 as a divisional application to the application mentioned under INID code 62.

# (54) METHODS OF USING THERMOSTABLE SERINE PROTEASES

(57) Methods of using thermostable serine proteases are described herein.

EP 4 644 560 A2

## Description

CROSS-REFERENCE TO RELATED APPLICATION

The present application claims priority to U.S. Provisional Patent Application Serial No. 62/437340, filed on December 21, 2016 which is hereby incorporated by reference in its entirety.

INCORPORATION BY REFERENCE OF THE SEQUENCE LISTING

The sequence listing provided in the file named NB40835WOPCT_SequenceListing_ST.txt" with a size of 23 KB which was created on December 14, 2016 and which is filed herewith, is incorporated by reference herein in its entirety.

FIELD

The field relates to methods of using thermostable serine proteases.

BACKGROUND

Proteases (also called peptidases or proteinases) are enzymes capable of cleaving peptide bonds. Proteases have evolved multiple times, and different classes of proteases can perform the same reaction by completely different catalytic mechanisms. Proteases can be found in animals, plants, fungi, bacteria, archaea and viruses.

Proteolysis can be achieved by enzymes currently classified into six broad groups: aspartyl proteases, cysteine proteases, serine proteases, threonine proteases, glutamic proteases, and metalloproteases.

Serine proteases are a subgroup of carbonyl hydrolases comprising a diverse class of enzymes having a wide range of specificities and biological functions. Notwithstanding this functional diversity, the catalytic machinery of serine proteases has been approached by at least two genetically distinct families of enzymes: 1) the subtilisins; and 2) chymotrypsin-related serine proteases (e.g. trypsin).

These two families of serine proteases or serine endopeptidases have very similar catalytic mechanisms. The tertiary structure of these two enzyme families brings together a conserved catalytic triad of amino acids consisting of serine, histidine and aspartate.

Much research has been conducted on the serine proteases, due largely to their useful in industrial applications. Additional work has been focused on adverse environmental conditions (e.g., exposure to oxidative agents, chelating agents, extremes of temperature and/or pH) which can adversely impact the functionality of these enzymes in a variety of applications.

Thus, there is a continuing need to develop applications in which proteases can be used under adverse conditions and retain or have improved activity.

SUMMARY

In one embodiment, there is disclosed a method for producing a thermostable serine protease comprising:

(a) transforming a production host with a recombinant construct encoding a thermostable serine protease having at least 80% sequence identity to SEQ ID NOs: 3, 8, or 10; and

(b) culturing the production host of step (a) at a temperature above 37.5°C to increase expression level of the thermostable serine protease.

In a second embodiment, the thermostable serine protease can be optionally recovered from the production host.

In a third embodiment, a thermostable serine protease-containing culture supernatant obtained by any of the methods described herein.

In a fourth embodiment, there is disclosed a feed, feedstuff, feed additive composition, premix, food or grain product comprising a thermostable serine protease having at least 80% sequence identity to SEQ ID NOs:3, 8, or 10 wherein said thermostable serine protease can be used alone or in combination with at least one direct fed microbial.

In a fifth embodiment, there is disclosed an animal feed comprising a thermostable serine protease having at least 80% sequence identity to SEQ ID NOs: 3, 8 or 10 wherein the thermostable serine protease is present in an amount from 1-20g/ton feed and further wherein said thermostable serine protease can be used alone or in combination with at least one direct fed microbial.

In a sixth embodiment, there is disclosed a feed additive composition as described herein further comprising at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

In a seventh embodiment, there is disclosed a granulated feed additive composition for use in animal feed comprising a thermostable serine protease having at least 80% sequence identity to SEQ ID NOs: 3, 8, or 10 used either alone or in combination with at least one direct fed microbial, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

In an eighth embodiment, there is disclosed a granulated feed additive composition of claim 7, wherein the mean diameter of the particles is greater than 50 microns and less than 2000 microns. Furthermore, this granulated feed additive composition of claim can be in a liquid form. Even further, this liquid form can be suitable for spray-drying on a feed pellet.

In a ninth embodiment, there is disclosed a method for hydrolyzing starch-containing material comprising:

(a) contacting starch-containing material with a liquid to form a mash; and
(b) hydrolyzing starch in the mash to form a liquefact by contacting the mash with an enzyme cocktail comprising a thermostable serine protease having at least 80% sequence identity to SEQ ID NOs: 3, 8, or 10. In addition, the enzyme cocktail can further comprise at least one enzyme selected from the group consisting of alpha-amylase, amyloglucosidase, phytase, pullulanase, beta-glucanase, cellulase and xylanase.

In a tenth embodiment, there is disclosed a method for producing fermentation products from starch-containing material comprising:

(a) liquefying the starch-containing material with an enzyme cocktail comprising a thermostable serine protease having at least 80% sequence identity to SEQ ID NOs: 3, 8, or 10;
(b) saccharifying the product of step (a);
(c) fermenting with a suitable organism; and
(d) optionally, recovering the product produced in step (c).

Steps (b) and (c) can be performed sequentially or can be performed simultaneously.

When steps (b) and (c) are performed simultaneously, addition of a nitrogen source (such as but not limiting to urea or ammonia) is either eliminated or reduced by at least 30%, 40%, 50%, 65%, 70%, 75%, 80%, 85%, 90%, 95%, 96%, 97%, 98%, or 99% by using at least 1, 2, 3, 4, 5, 6, 7, 8, 9, 10, 11, 12, 13, 14, 15,16, 17, 18, 19, or 20 g thermostable serine protease/metric ton (MT) starch-containing material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10 is used.

When steps (b) and (c) are performed simultaneously, addition of a nitrogen source (such as but not limiting to urea) is either eliminated (100% reduction) or reduced by at least 30%, 40%, 50%, 60%, 65%, 70%, 75%, 80%, 85%, 90%, 95%, 96%, 97%, 98%, or 99% by using at least 3, 4, 5, or 6 g thermostable serine protease/MT dry solid material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 is used.

When steps (b) and (c) are performed simultaneously, addition of a nitrogen source, such as but not limiting to, urea is either eliminated (100% reduction) or reduced by at least 30%, 40%, 50%, 60%, 65%, 70%, 75%, 80%, 85%, 90%, 95%, 96%, 97%, 98%, or 99% by using at least 1, 2, 3, 4, 5, 6, 7, 8, 9, 10, 11, 12, 13, 14, 15,16, 17, 18, 19, or 20 g thermostable serine protease/MTstarch-containing material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10 and, furthermore, when the fermentation product is ethanol then no acid proteolytic enzyme is needed in step (c).

In an eleventh embodiment, there is a method for reducing viscosity of a liquefied starch-containing material which comprises contacting a slurry of starch-containing material with a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10. Contacting of the thermostable serine protease described herein with the slurry can be in combination with other enzymes, or to a slurry comprising enzymes other than the thermostable serine protease described herein.

In a twelfth embodiment, there is a method for extracting oil from an oilseed crop comprising:

(a) contacting an oil-containing oilseed fraction with an aqueous extractant to form an extract oilseed fraction; and

(b) separating the extracted oilseed fraction into an aqueous phase, an oil-in-water emulsion, and an insoluble phase;
(c) contacting the oil-in-water emulsion with at least a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10, either alone or in combination with a phospholipase, under conditions permitting enzyme activity for a time sufficient to destabilize the emulsion; and
(d) separating the destabilizing emulsion of step (c) into an aqueous phase, an oil phase, and an insoluble phase to obtain oil from the oilseed.

Furthermore, the oilseed fraction can be from soybean, corn seed, rape seed, palm kernel, sunflower seed, safflower seed, coconut, peanut, cotton seed, sesame seed, flax seed, poppy seed, almond, hazelnut, walnut, evening primrose seed, grape seed, hemp seed, black currant seed, red raspberry seed, carrot seed, cumin seed, blueberry seed, cranberry seed, parsley seed, onion seed, pumpkin seed, apricot kernel, mustard seed, linseed, castor seed or jatropha.
Even further, the oilseed fraction can comprise a protein fraction that is useful as a food, food ingredient, a food additive or food supplement.
In a thirteenth embodiment, there is disclosed a feed, feedstuff, feed additive composition, premix, food or grain product comprising oil obtained by the method described herein.
In a fourteenth embodiment, there is disclosed a method for preparing a wort comprising:

a) contacting starch-containing material comprising a mix of milled grain with water and a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 to form a mash;
b) slowly heating the mash of step (a) to convert the starch to sugars; and
c) separating the heated mash of step (b) into residual grain and liquid wort

wherein the thermostable protease is present in an amount from 1-30 g/ton milled grain.
Furthermore, an alpha-amylase can be added in step (a) along with the thermostable serine protease.
In a fifteenth embodiment, there is disclosed a method for hydrolyzing at least one food or animal by-product comprising:

(a) contacting the food or animal by-product with a liquid; and
(b) hydrolyzing food or animal by-product to form a liquefact by contacting with an enzyme cocktail comprising a thermostable serine protease having at least 80% sequence identity to SEQ ID NOs:3, 8, or 10.

Furthermore, the food by-product is keratin-rich material selected from the group consisting of feather, hair and wool.
In a sixteenth embodiment, there is disclosed a method for hydrolyzing proteins in lignocellulosic biomass comprising

(a) contacting the biomass with a liquid; and
(b) hydrolyzing the proteins in the biomass by contacting the biomass with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99%sequence identity to SEQ ID NOs:3, 8, or 10.

BRIEF DESCRIPTION OF THE DRAWINGS AND SEQUENCES

Figure 1. Physical map of the pHYT-Tce01961plasmid for the expression of ME-3 protease in *Bacillus subtilis.*
Figure 2. Dose response of corn soy feed protein hydrolysis treated with ME-3 protease in the presence of pepsin and pancreatin.
Figure 3. Thermostability of ME-3 protease incubated at pH 10.
Figure 4. Effect of pH on the enzymatic activity of ME-3 protease evaluated between pH 3.75 and 7.5.
Figure 5. ME-3 protease without any coating retained more than 70% activity after pelleting at 90°C to 100°C for 60 seconds, compared to mash.
Figure 6. Cumulative pressure plot of simultaneous saccharification and fermentation performed on Ankom system. The light grey line represents no FERMGEN™ 2.5x, 240 ppm urea, no ME-3 added condition; medium-grey line represents 0.178 SAPU/g DS FERMGEN™, 600 ppm urea, no ME-3 added condition; and the black line represents no FERMGEN™ 2. 5x, 240 ppm urea plus ME-3 protease added condition.

Figure 7. FAN content (mg/L) in wort after high temperature mashing with ME-3 protease, using a mix of 50% malt and 50% corn in a single vessel.

Figure 8. Multiple amino acid sequence alignment of the mature chains of WP_017594871 with the Thermobifida proteases: ME-3, Tfpa and Thpa, *and M. thermotolerans*_WP_078763344, *A. marina* _WP_091675221, *A. hymeniacidonis*_WP_069846166, *N. trehalosi*_WP_067965505 *and Saccharothrix sp.*_WP_033441214

The following sequences comply with 37 C.F.R. §§ 1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (2009) and the sequence listing requirements of the European Patent Convention (EPC) and the Patent Cooperation Treaty (PCT) Rules 5.2 and 49.5(a-bis), and Section 208 and Annex C of the Administrative Instructions. The symbols and format used for nucleotide and amino acid sequence data comply with the rules set forth in 37 C.F.R. § 1.822.

SEQ ID NO:1 sets forth the nucleotide sequence of *Tce01961n,* encoding the protease ME-3.

SEQ ID NO:2 sets forth amino acid sequence of the ME-3 preproenzyme encoded by *Tce01961n*.

SEQ ID NO:3 sets forth the amino acid sequence of the fully processed mature enzyme, ME-3 (186 amino acids).

SEQ ID NO:4 sets forth the sequence of the modified *aprE* signal peptide sequence fused to the codon-optimized DNA sequence encoding the pro-mature sequence of ME-3 protease.

SEQ ID NO:5 sets forth the terminator sequence added after the tetracycline resistance gene using the *Bst*EII and *Eco*RI sites to make the pHYT vector derived from pHY300PLK (Takara Bio Inc.).

SEQ ID NO:6 sets forth the linker sequence cloned into the *Bam*HI and *Hind*III sites of pHY300PLK.

SEQ ID NO:7 sets forth the amino acid sequence of *Thermobifida fusca* Tfpa preproenzyme.

SEQ ID NO:8 sets forth the amino acid sequence of the fully processed mature enzyme, Tfpa (186 amino acids).

SEQ ID NO:9 sets forth the amino acid sequence of *Thermobifida halotolerans* WP_068687914 peptidase S1, Thpa.

SEQ ID NO: 10 sets forth the amino acid sequence of the predicted fully processed mature enzyme, *Thermobifida halotolerans* WP_068687914 peptidase S1, or Thpa (186 amino acids).

SEQ ID NO: 11 sets forth the amino acid sequence of *Nocardiopsis potens* WP_017594871.

SEQ ID NO: 12 sets forth the amino acid sequence of the predicted fully processed mature enzyme, *Nocardiopsis potens* WP_017594871 serine protease (185 amino acids).

## DETAILED DESCRIPTION

All patents, patent applications, and publications cited are incorporated herein by reference in their entirety.

In this disclosure, a number of terms and abbreviations are used. The following definitions apply unless specifically stated otherwise.

The articles "a", "an", and "the" preceding an element or component are intended to be nonrestrictive regarding the number of instances (i.e., occurrences) of the element or component. Therefore "a", "an", and "the" should be read to include one or at least one, and the singular word form of the element or component also includes the plural unless the number is obviously meant to be singular.

The term "comprising" means the presence of the stated features, integers, steps, or components as referred to in the claims, but that it does not preclude the presence or addition of one or more other features, integers, steps, components or groups thereof. The term "comprising" is intended to include embodiments encompassed by the terms "consisting essentially of" and "consisting of". Similarly, the term "consisting essentially of" is intended to include embodiments encompassed by the term "consisting of".

Where present, all ranges are inclusive and combinable. For example, when a range of "1 to 5" is recited, the recited range should be construed as including ranges "1 to 4", "1 to 3", "1-2", "1-2 & 4-5", "1-3 & 5", and the like.

As used herein in connection with a numerical value, the term "about" refers to a range of +/- 0.5 of the numerical value, unless the term is otherwise specifically defined in context. For instance, the phrase a "pH value of about 6" refers to pH values of from 5.5 to 6.5, unless the pH value is specifically defined otherwise.

It is intended that every maximum numerical limitation given throughout this Specification includes every lower numerical limitation, as if such lower numerical limitations were expressly written herein. Every minimum numerical limitation given throughout this Specification will include every higher numerical limitation, as if such higher numerical limitations were expressly written herein. Every numerical range given throughout this Specification will include every narrower numerical range that falls within such broader numerical range, as if such narrower numerical ranges were all expressly written herein.

The term "protease" means a protein or polypeptide domain of derived from a microorganism, e.g., a fungus, bacterium, or from a plant or animal, and that has the ability to catalyze cleavage of peptide bonds at one or more of various positions of a protein backbone (e.g., E.C. 3.4). The terms "protease", "peptidase" and "proteinase" can be used interchangeably. Proteases can be found in animals, plants, fungi, bacteria, archaea and viruses. Proteolysis can be achieved by enzymes currently classified into six broad groups: aspartyl proteases, cysteine proteases, serine proteases, threonine proteases, glutamic proteases, and metalloproteases.

The term "thermostable serine protease" means a serine protease that is heat-stable.

The term "starch" refers to any material comprised of the complex polysaccharide carbohydrates of plants. This term refers to any plant-based material including, but not limited to, grains, grasses, tubers, and roots and, more specifically, to wheat, barley, corn, rye, rice, sorghum, brans, cassaya, millet, potato, sweet potato, and tapioca.

The term "slurry" refers to a mixture of a starch-containing material (e.g., milled corn) and an aqueous component, which can include water, de-ionized water or process water (e.g., backset, steam, condensate), or any combination thereof. The terms "slurry" and "mash" can be used interchangeably herein.

The terms "liquefy", "liquefaction", "liquefact" and variations thereof refer to the process or product of converting starch to soluble dextrinized substrates (e.g., smaller polysaccharides).

"Liquefact" can be referred to as "mash" or may also be called a soluble starch substrate or a liquefied substrate. In some cases, it may be a whole ground grain slurry containing a thermostable alpha amylase that has been subjected to high temperature liquefaction resulting in a soluble substrate for saccharification and fermentation or simultaneous saccharification and fermentation (SSF). High temperature is a temperature higher than the gelatinization temperature of the grain polysaccharides.

The term "milled" is used herein to refer to plant material that has been reduced in size, such as by grinding, crushing, fractionating or any other means of particle size reduction. Milling includes dry or wet milling. "Dry milling" refers to the milling of whole dry grain. "Wet milling" refers to a process whereby grain is first soaked (steeped) in water to soften the grain.

The term "hydrolysis" refers to a chemical reaction or process in which a chemical compound is broken down by reaction with water. Starch digesting enzymes hydrolyze starch into smaller units, i.e, smaller polysaccharides.

The term "lignocellulosic" refers to a composition comprising both lignin and cellulose. It may also contain hemicellulose.

The term "lignocellulosic biomass" refers to any lignocellulosic material and includes materials comprising cellulose, hemicellulose, lignin, starch, oligosaccharides and/or monosaccharides. Biomass can also comprise additional components, such as protein and/or lipid. Biomass can be derived from a single source, or biomass can comprise a mixture derived from more than one source; 25 for example, biomass could comprise a mixture of corn cobs and corn stover, or a mixture of grass and leaves. Lignocellulosic biomass includes, but is not limited to, bioenergy crops, agricultural residues, municipal solid waste, industrial solid waste, sludge from paper manufacture, yard waste, wood and forestry waste. Examples of biomass include, but are not limited to, corn cobs, crop residues 30 such as corn husks, corn stover, grasses (including Miscanthus), wheat straw, barley straw, hay, rice straw, switchgrass, waste paper, sugar cane bagasse, sorghum material, soybean plant material, components obtained from milling of grains or from using grains in production processes (such as DDGS: dried distillers grains with solubles), trees, branches, roots, leaves, wood chips, sawdust, shrubs and bushes, vegetables, fruits, flowers, empty palm fruit bunch, and energy cane. The term "energy cane" refers to sugar cane that is bred for use in energy production. It is selected for a higher percentage of fiber than sugar.

The term "pretreated lignocellulosic biomass" refers to biomass which has been subjected to a physical, thermal and/or chemical treatment prior to saccharification. The term "ammonia pretreated lignocellulosic biomass" refers to biomass which has been subjected at least to a pretreatment process employing ammonia. In one embodiment ammonia pretreatment is a low ammonia pretreatment where biomass is contacted with an aqueous solution comprising ammonia to form a biomass-aqueous ammonia mixture where the ammonia concentration is sufficient to maintain alkaline pH of the biomassaqueous ammonia mixture but is less than about 12 weight percent relative to dry weight of biomass, and where dry weight of biomass is at least about 15 weight percent solids relative to the weight of the biomass-aqueous ammonia mixture, as disclosed in the U.S. Patent No. 7,932,063, which is herein incorporated by reference. The term "lignocellulosic biomass hydrolysate" refers to the product resulting from saccharification of lignocellulosic biomass. The biomass may also be pretreated or pre-processed prior to saccharification. The terms "saccharification" and "saccharifying" refer to the process of converting polysaccharides to dextrose monomers using enzymes. Saccharification can refer to the conversion of polysaccharides in a liquefact. Saccharification products are, for example, glucose and other small (low molecular weight) oligosaccharides such as disaccharides and trisaccharides.

The term "SSF" refers to simultaneous saccharification and fermentation.

The term "enzyme cocktail" refers to a mixture or combination of at least two different enzymes, which make it more efficient and effective for any catalytic reaction.

The terms "fermentation" or "fermenting" refer to the process of transforming sugars from reduced plant material to produce as fermentation product.

The term "fermentation product" means a product produced by a process including a fermentation step using a fermenting organism.

The term "viscosity" means the resistance of a fluid to flow, i.e., the internal friction of a fluid.

The term "phospholipase" refers to an enzyme that hydrolyzes phospholipids into fatty acids and other lipophilic substances.

The term "emulsion" refers to a mixture of two or more liquids that are normally immiscible (unmixable or unblendable). In an emulsion, one liquid (the dispersed phase) is dispersed in the other (continuous phase). Examples of emulsions include vinaigrettes, homogenized milk, mayonnaise, and the like.

The term "grist" refers to grain or a quantity of grain to be ground; ground grain or meal produced from a grinding. The term "wort" refers to the unfermented liquor run-off following extracting the grist during mashing.

The term "mash" refers to an aqueous slurry of any starch and/or sugar containing plant material such as grist, e.g. comprising crushed barley malt, crushed barley, and/or other adjunct or a combination hereof, mixed with water later to be separated into wort and spent grains.

The term "beer" is meant to comprise any fermented wort, produced by fermentation/brewing of a starch-containing plant material, thus in particular also beer produced exclusively from malt or adjunct, or any combination of malt and adjunct.

Beer can be made from a variety of starch and/or sugar containing plant material, often cereal grains and/or malt by essentially the same process. Grain starches are believed to be glucose homopolymers in which the glucose residues are linked by either alpha-1, 4- or alpha-1,6-bonds, with the former predominating.

The term "by-product" refers to a secondary product derived from a manufacturing process or chemical reaction. It is not the primary product or service being produced.

The terms "animal" and "subject" are used interchangeably herein. An animal includes all non-ruminant (including humans) and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

The term "pathogen" as used herein means any causative agent of disease. Such causative agents can include, but are not limited to, bacterial, viral, fungal causative agents and the like.

A "feed" and a "food," respectively, means any natural or artificial diet, meal or the like or components of such meals intended or suitable for being eaten, taken in, digested, by a non-human animal and a human being, respectively.

As used herein, the term "food" is used in a broad sense and covers food and food products for humans as well as food for non-human animals (i.e. a feed).

The term "feed" is used with reference to products that are fed to animals in the rearing of livestock. The terms "feed" and "animal feed" are used interchangeably.

The term "direct-fed microbial" ("DFM") as used herein is source of live (viable) naturally occurring microorganisms. A DFM can comprise one or more of such naturally occurring microorganisms such as bacterial strains. Categories of DFMs include Bacillus, Lactic Acid Bacteria and Yeasts. Thus, the term DFM encompasses one or more of the following: direct fed bacteria, direct fed yeast, direct fed yeast and combinations thereof.

Bacilli are unique, gram-positive rods that form spores. These spores are very stable and can withstand environmental conditions such as heat, moisture and a range of pH. These spores germinate into active vegetative cells when ingested by an animal and can be used in meal and pelleted diets. Lactic Acid Bacteria are gram-positive cocci that produce lactic acid which are antagonistic to pathogens. Since Lactic Acid Bacteria appear to be somewhat heat-sensitive, they are not used in pelleted diets. Types of Lactic Acid Bacteria include *Bifidobacterium, Lactobacillus* and *Streptococcus.*

The term "prebiotic" means a non-digestible food ingredient that beneficially affects the host by selectively stimulating the growth and/or the activity of one or a limited number of beneficial bacteria.

The term "probiotic culture" as used herein defines live microorganisms (including bacteria or yeasts for example) which, when for example ingested or locally applied in sufficient numbers, beneficially affects the host organism, i.e. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut. Whilst there are no lower or upper limits for probiotic intake, it has been suggested that at least $10^6$-$10^{12}$, preferably at least $10^6$-$10^{10}$, preferably $10^8$-$10^9$, cfu as a daily dose will be effective to achieve the beneficial health effects in a subject.

The term "CFU" as used herein means "colony forming units" and is a measure of viable cells in which a colony represents an aggregate of cells derived from a single progenitor cell.

The term "isolated" means a substance in a form or environment that does not occur in nature. Non-limiting examples of isolated substances include (1 ) any non- naturally occurring substance, (2) any substance including, but not limited to, any host cell, enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated. The terms "isolated nucleic acid molecule", "isolated polynucleotide", and "isolated nucleic acid fragment" will be used interchangeably and refer to a polymer of RNA or DNA that is single- or double-stranded, optionally containing synthetic, non-natural or altered nucleotide bases. An isolated nucleic acid molecule in the form of a polymer of DNA may be comprised of one or more segments of cDNA, genomic DNA or synthetic DNA.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, the term "functional assay" refers to an assay that provides an indication of a protein's activity. In some embodiments, the term refers to assay systems in which a protein is analyzed for its ability to function in its usual capacity. For example, in the case of a protease, a functional assay involves determining the effectiveness of the protease to hydrolyze a proteinaceous substrate.

The terms "peptides", "proteins" and "polypeptides are used interchangeably herein and refer to a polymer of amino acids joined together by peptide bonds. A "protein" or "polypeptide" comprises a polymeric sequence of amino acid residues. The single and 3-letter code for amino acids as defined in conformity with the IUPAC-IUB Joint Commission on Biochemical Nomenclature (JCBN) is used throughout this disclosure. The single letter X refers to any of the twenty amino acids. It is also understood that a polypeptide may be coded for by more than one nucleotide sequence due to the degeneracy of the genetic code. Mutations can be named by the one letter code for the parent amino acid, followed by a position number and then the one letter code for the variant amino acid. For example, mutating glycine (G) at position 87 to serine (S) is represented as "G087S" or "G87S". When describing modifications, a position followed by amino acids listed in parentheses indicates a list of substitutions at that position by any of the listed amino acids. For example, 6(L,I) means position 6 can be substituted with a leucine or isoleucine. At times, in a sequence, a slash (/) is used to define substitutions, e.g. F/V, indicates that the particular position may have a phenylalanine or valine at that position.

A "prosequence" or "propeptide sequence" refers to an amino acid sequence between the signal peptide sequence and mature protease sequence that is necessary for the proper folding and secretion of the protease; they are sometimes referred to as intramolecular chaperones. Cleavage of the prosequence or propeptide sequence results in a mature active protease. Proteases are often expressed as pro-enzymes.

The terms "signal sequence" and "signal peptide" refer to a sequence of amino acid residues that may participate in the secretion or direct transport of the mature or precursor form of a protein. The signal sequence is typically located N-terminal to the precursor or mature protein sequence. The signal sequence may be endogenous or exogenous. A signal sequence is normally absent from the mature protein. A signal sequence is typically cleaved from the protein by a signal peptidase after the protein is transported.

The term "mature" form of a protein, polypeptide, or peptide refers to the functional form of the protein, polypeptide, or enzyme without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked to the amino terminus of the prosequence. The precursor may also have additional polypeptides that are involved in post-translational activity (e.g., polypeptides cleaved therefrom to leave the mature form of a protein or peptide).

The term "wild-type" in reference to an amino acid sequence or nucleic acid sequence indicates that the amino acid sequence or nucleic acid sequence is a native or naturally-occurring sequence. As used herein, the term "naturally-occurring" refers to anything (e.g., proteins, amino acids, or nucleic acid sequences) that is found in nature. Conversely, the term "non-naturally occurring" refers to anything that is not found in nature (e.g., recombinant nucleic acids and protein sequences produced in the laboratory or modification of the wild-type sequence).

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position in a related proteins or a reference protein.

The terms "derived from" and "obtained from" refer to not only a protein produced or producible by a strain of the organism in question, but also a protein encoded by a DNA sequence isolated from such strain and produced in a host organism containing such DNA sequence. Additionally, the term refers to a protein which is encoded by a DNA sequence of synthetic and/or cDNA origin and which has the identifying characteristics of the protein in question.

The term "amino acid" refers to the basic chemical structural unit of a protein or polypeptide. Abbreviations used herein to identify specific amino acids may use the three or one-letter code as known in the art, and additionally, Xaa or X can be used to denote any amino acid.

It would be recognized by one of ordinary skill in the art that modifications of amino acid sequences disclosed herein can be made while retaining the function associated with the disclosed amino acid sequences. For example, it is well known in the art that alterations in a gene which result in the production of a chemically equivalent amino acid at a given site, but do not affect the functional properties of the encoded protein are common. For example, any particular amino acid in an amino acid sequence disclosed herein may be substituted for another functionally equivalent amino acid. For the purposes of this disclosure, substitutions are defined as exchanges within one of the following five groups:

1. Small aliphatic, nonpolar or slightly polar residues: Ala, Ser, Thr (Pro, Gly);
2. Polar, negatively charged residues and their amides: Asp, Asn, Glu, Gln;
3. Polar, positively charged residues: His, Arg, Lys;
4. Large aliphatic, nonpolar residues: Met, Leu, Ile, Val (Cys); and
5. Large aromatic residues: Phe, Tyr, and Trp.

Thus, a codon for the amino acid alanine, a hydrophobic amino acid, may be substituted by a codon encoding another less hydrophobic residue (such as glycine) or a more hydrophobic residue (such as valine, leucine, or isoleucine). Similarly, changes which result in substitution of one negatively charged residue for another (such as thermostable serine acid for glutamic acid) or one positively charged residue for another (such as lysine for arginine) can also be expected to produce a functionally equivalent product. In many cases, nucleotide changes which result in alteration of the N-terminal and C-terminal portions of the protein molecule would also not be expected to alter the activity of the protein. Each of the proposed modifications is well within the routine skill in the art, as is determination of retention of biological activity of the encoded products.

The term "codon optimized", as it refers to genes or coding regions of nucleic acid molecules for transformation of various hosts, refers to the alteration of codons in the gene or coding regions of the nucleic acid molecules to reflect the typical codon usage of the host organism without altering the polypeptide for which the DNA codes.

The term "gene" refers to a nucleic acid molecule that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different from that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

The term "coding sequence" refers to a nucleotide sequence which codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, RNA processing site, effector binding sites, and stem-loop structures.

The term "operably linked" refers to the association of nucleic acid sequences on a single nucleic acid molecule so that the function of one is affected by the other. For example, a promoter is operably linked with a coding sequence when it is capable of affecting the expression of that coding sequence, *i.e.,* the coding sequence is under the transcriptional control of the promoter. Coding sequences can be operably linked to regulatory sequences in sense or antisense orientation.

The terms "regulatory sequence" or "control sequence" are used interchangeably herein and refer to a segment of a nucleotide sequence which is capable of increasing or decreasing expression of specific genes within an organism. Examples of regulatory sequences include, but are not limited to, promoters, signal sequence, operators and the like. As noted above, regulatory sequences can be operably linked in sense or antisense orientation to the coding sequence/gene of interest.

"Promoter" or "promoter sequences" refer to DNA sequences that define where transcription of a gene by RNA polymerase begins. Promoter sequences are typically located directly upstream or at the 5' end of the transcription initiation site. Promoters may be derived in their entirety from a native or naturally occurring sequence, or be composed of different elements derived from different promoters found in nature, or even comprise synthetic DNA segments. It is understood by those skilled in the art that different promoters may direct the expression of a gene in different tissues or cell type or at different stages of development, or in response to different environmental or physiological conditions ("inducible promoters").

The "3' non-coding sequences" refer to DNA sequences located downstream of a coding sequence and include sequences encoding regulatory signals capable of affecting mRNA processing or gene expression, such as termination of transcription.

The term "transformation" as used herein refers to the transfer or introduction of a nucleic acid molecule into a host organism. The nucleic acid molecule may be introduced as a linear or circular form of DNA. The nucleic acid molecule may be a plasmid that replicates autonomously, or it may integrate into the genome of a production host. Production hosts containing the transformed nucleic acid are referred to as "transformed" or "recombinant" or "transgenic" organisms or "transformants".

The term "recombinant" as used herein refers to an artificial combination of two otherwise separated segments of nucleic acid sequences, e.g., by chemical synthesis or by the manipulation of isolated segments of nucleic acids by genetic engineering techniques. For example, DNA in which one or more segments or genes have been inserted, either naturally or by laboratory manipulation, from a different molecule, from another part of the same molecule, or an artificial sequence, resulting in the introduction of a new sequence in a gene and subsequently in an organism. The terms "recombinant", "transgenic", "transformed", "engineered" or "modified for exogenous gene expression" are used interchangeably herein.

The terms "recombinant construct", "expression construct", "recombinant expression construct" and "expression cassette" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The terms "production host", "host" and "host cell" are used interchangeably herein and refer to any organism, or cell thereof, whether human or non-human into which a recombinant construct can be stably or transiently introduced in order to express a gene. This term encompasses any progeny of a parent cell, which is not identical to the parent cell due to mutations that occur during propagation.

The term "percent identity" is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the number of matching nucleotides or amino acids between strings of such sequences. "Identity" and "similarity" can be readily calculated by known methods, including but not limited to those described in: Computational Molecular Biology (Lesk, A. M., ed.) Oxford University Press, NY (1988); Biocomputing: Informatics and Genome Projects (Smith, D. W., ed.) Academic Press, NY (1993); Computer Analysis of Sequence Data, Part I (Griffin, A. M., and Griffin, H. G., eds.) Humana Press, NJ (1994); Sequence Analysis in Molecular Biology (von Heinje, G., ed.) Academic Press (1987); and Sequence Analysis Primer (Gribskov, M. and Devereux, J., eds.) Stockton Press, NY (1991). Methods to determine identity and similarity are codified in publicly available computer programs.

As used herein, "% identity" or percent identity" or "PID" refers to protein sequence identity. Percent identity may be determined using standard techniques known in the art. Useful algorithms include the BLAST algorithms (*See,* Altschul et al., J Mol Biol, 215:403-410, 1990; and Karlin and Altschul, Proc Natl Acad Sci USA, 90:5873-5787, 1993). The BLAST program uses several search parameters, most of which are set to the default values. The NCBI BLAST algorithm finds the most relevant sequences in terms of biological similarity but is not recommended for query sequences of less than 20 residues (Altschul et al., Nucleic Acids Res, 25:3389-3402, 1997; and Schaffer et al., Nucleic Acids Res, 29:2994-3005, 2001). Exemplary default BLAST parameters for a nucleic acid sequence searches include: Neighboring words threshold = 11; E-value cutoff = 10; Scoring Matrix = NUC.3.1 (match = 1, mismatch = -3);Gap Opening = 5; and Gap Extension = 2.

Exemplary default BLAST parameters for amino acid sequence searches include: Word size = 3; E-value cutoff = 10; Scoring Matrix = BLOSUM62; Gap Opening = 11; and Gap extension = 1. A percent (%) amino acid sequence identity value is determined by the number of matching identical residues divided by the total number of residues of the "reference" sequence including any gaps created by the program for optimal/maximum alignment. BLAST algorithms refer to the "reference" sequence as the "query" sequence.
As used herein, "homologous proteins" or "homologous proteases" refers to proteins that have distinct similarity in primary, secondary, and/or tertiary structure. Protein homology can refer to the similarity in linear amino acid sequence when proteins are aligned. Homologous search of protein sequences can be done using BLASTP and PSI-BLAST from NCBI BLAST with threshold (E-value cut-off) at 0.001. (Altschul SF, Madde TL, Shaffer AA, Zhang J, Zhang Z, Miller W, Lipman DJ. Gapped BLAST and PSI BLAST a new generation of protein database search programs. Nucleic Acids Res 1997 Set 1;25(17):3389-402). Using this information, proteins sequences can be grouped. A phylogenetic tree can be built using the amino acid sequences.

Sequence alignments and percent identity calculations may be performed using the Megalign program of the LASERGENE bioinformatics computing suite (DNASTAR Inc., Madison, WI), the AlignX program of Vector NTI v. 7.0 (Informax, Inc., Bethesda, MD), or the EMBOSS Open Software Suite (EMBL-EBI; Rice et al., Trends in Genetics 16, (6):276-277 (2000)). Multiple alignment of the sequences can be performed using the CLUSTAL method (such as CLUSTALW; for example, version 1.83) of alignment (Higgins and Sharp, CABIOS, 5:151-153 (1989); Higgins et al., Nucleic Acids Res. 22:4673-4680 (1994); and Chenna et al., Nucleic Acids Res 31 (13):3497-500 (2003)), available from the European Molecular Biology Laboratory via the European Bioinformatics Institute) with the default parameters. Suitable parameters for CLUSTALW protein alignments include GAP Existence penalty=15, GAP extension =0.2, matrix = Gonnet (*e.g.*, Gonnet250), protein ENDGAP = -1, protein GAPDIST=4, and KTUPLE=1. In one embodiment, a fast or slow alignment is used with the default settings where a slow alignment. Alternatively, the parameters using the CLUSTALW method (*e.g.*, version 1.83) may be modified to also use KTUPLE =1, GAP PENALTY=10, GAP extension =1, matrix = BLOSUM (*e.g.*, BLOSUM64), WINDOW=5, and TOP DIAGONALS SAVED=5. Sequences can also be multiply aligned using the MUSCLE program from Geneious software (Biomatters Ltd.) (Robert C. Edgar. MUSCLE: multiple sequence alignment with high accuracy and high throughput Nucl. Acids Res. (2004) 32 (5): 1792-1797).

Various polypeptide amino acid sequences and polynucleotide sequences are disclosed herein as features of certain aspects. Variants of these sequences that are at least about 70-85%, 85-90%, or 90%-95% identical to the sequences disclosed herein may be used in certain embodiments. Alternatively, a variant polypeptide sequence or polynucleotide sequence in certain embodiments can have at least 60%, 61%, 62%,63%,64%, 65%, 66%, 67%, 68%,69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with a sequence disclosed herein. The variant amino acid sequence or polynucleotide sequence has the same function of the disclosed sequence, or at least about 85%, 86%, 87%, 88%, 89%, 90%, 91%, 92%, 93%, 94%, 95%, 96%, 97%, 98%, or 99% of the function of the disclosed sequence.

The term "variant", with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. The terms "expression cassette" and "expression vector are used interchangeably herein and refer to a specific vector containing a gene and having elements in addition to the gene that allow for expression of that gene in a host.

The term "expression", as used herein, refers to the production of a functional end-product (e.g., an mRNA or a protein) in either precursor or mature form. Expression may also refer to translation of mRNA into a polypeptide.

Expression of a gene involves transcription of the gene and translation of the mRNA into a precursor or mature protein. "Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals. "Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms

The expression vector can be one of any number of vectors or cassettes useful for the transformation of suitable production hosts known in the art. Typically, the vector or cassette will include sequences directing transcription and translation of the relevant gene, a selectable marker, and sequences allowing autonomous replication or chromosomal integration. Suitable vectors generally include a region 5' of the gene which harbors transcriptional initiation controls and a region 3' of the DNA fragment which controls transcriptional termination. Both control regions can be derived from homologous genes to genes of a transformed production host cell and/or genes native to the production host, although such control regions need not be so derived.

Possible initiation control regions or promoters that can be included in the expression vector are numerous and familiar to those skilled in the art. Virtually any promoter capable of driving these genes is suitable, including but not limited to, *CYC1, HIS3, GAL1, GAL10, ADH1, PGK, PHOS, GAPDH, ADC1, TRP1, URA3, LEU2, ENO, TPI*(useful for expression in *Saccharomyces); AOX1* (useful for expression in *Pichia*); and *lac, araB, tet, trp,* $IP_L$*,* $IP_R$*, T7, tac,* and *trc* (useful for expression in *Escherichia coli*) as well as the *amy, apr, npr* promoters and various phage promoters useful for expression in *Bacillus.* In some embodiments, the promoter is a constitutive or inducible promoter. A "constitutive promoter" is a promoter that is active under most environmental and developmental conditions. An "inducible" or "repressible" promoter is a promoter that is active under environmental or developmental regulation. In some embodiments, promoters are inducible or repressible due to changes in environmental factors including but not limited to, carbon, nitrogen or other nutrient availability, temperature, pH, osmolarity, the presence of heavy metal(s), the concentration of inhibitor(s), stress, or a combination of the foregoing, as is known in the art. In some embodiments, the inducible or repressible promoters are inducible or repressible by metabolic factors, such as the level of certain carbon sources, the level of certain energy sources, the level of certain catabolites, or a combination of the foregoing as is known in the art. In one embodiment, the promoter is one that is native to the host cell. For example, when *Trichoderma reesei* is the host, the promoter is a native *T. reesei* promoter such as the *cbhl* promoter which is deposited in GenBank under Accession Number D86235.

Suitable non-limiting examples of promoters include *cbh1, cbh2, egll, egl2, egl3, egl4, egl5, xynl,* and *xyn2,* repressible acid phosphatase gene (phoA) promoter of *P. chrysogenus* (see e.g., Graessle et al., (1997) Appl. Environ. Microbiol., 63 :753-756), glucose repressible PCK1 promoter (see e.g., Leuker et al., (1997), Gene, 192:235-240), maltose inducible, glucose-repressible MET3 promoter (see Liu et al., (2006), Eukary. Cell, 5:638-649), pKi promoter and cpc1 promoter. Other examples of useful promoters include promoters from *A. awamori* and *A. niger* glucoamylase genes (see e.g., Nunberg et al., (1984) Mol. Cell Biol. 15 4:2306-2315 and Boel et al., (1984) EMBO J. 3:1581-1585). Also, the promoters of the *T. reesei xln1* gene may be useful (see e.g., EPA 137280Al).

DNA fragments which control transcriptional termination may also be derived from various genes native to a preferred production host cell. In certain embodiments, the inclusion of a termination control region is optional. In certain embodiments, the expression vector includes a termination control region derived from the preferred host cell.

The expression vector can be included in the production host, particularly in the cells of microbial production hosts. The production host cells can be microbial hosts found within the fungal or bacterial families and which grow over a wide range of temperature, pH values, and solvent tolerances. For example, it is contemplated that any of bacteria, algae, and fungi such as filamentous fungi and yeast may suitably host the expression vector.

Inclusion of the expression vector in the production host cell may be used to express the protein of interest so that it may reside intracellularly, extracellularly, or a combination of both inside and outside the cell. Extracellular expression renders recovery of the desired protein from a fermentation product more facile than methods for recovery of protein produced by intracellular expression.

Certain embodiments of the present disclosure relate to a method for producing a thermostable serine protease comprising:

(a) transforming a production host with a recombinant construct encoding a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10; and

(b) culturing the production host of step (a) at a temperature above 37.5°C to increase expression level of the thermostable serine protease.

In another aspect, the thermostable serine protease optionally can be recovered from the production host.

In still another aspect, a thermostable serine protease-containing culture supernatant can be obtained by any of the methods described herein.

Expression will be understood to include any step involved in production of a thermostable serine protease including, but not limited to, transcription, post-transcriptional modification, translation, post-translation modification and secretion.

Techniques for modifying nucleic acid sequences utilizing cloning methods are well known in the art.

A polynucleotide encoding a thermostable serine protease can be manipulated in a variety of ways to provide for expression of the polynucleotide in a *Bacillus* host cell. Manipulation of the polynucleotide sequence prior to its insertion into a nucleic acid construct or vector may be desirable or necessary depending on the nucleic acid construct or vector or the *Bacillus* host cell. The techniques for modifying nucleotide sequences utilizing cloning methods are well known in the art.

Regulatory sequences are defined above. They include all components, which are necessary or advantageous for the expression of a thermostable serine protease. Each control sequence may be native or foreign to the nucleotide sequence encoding the thermostable serine protease. Such regulatory sequences include, but are not limited to, a leader, a polyadenylation sequence, a propeptide sequence, a promoter, a signal sequence and a transcription terminator. Regulatory sequences may be provided with linkers for the purpose of introducing specific restriction sites facilitating ligation or the regulatory sequences with the coding region of the nucleotide sequence encoding a thermostable serine protease.

A nucleic acid construct comprising a polynucleotide encoding a thermostable serine protease may be operably linked to one or more control sequences capable of directing the expression of the coding sequence in a *Bacillus* host cell under conditions compatible with the control sequences.

Each control sequence may be native or foreign to the polynucleotide encoding a thermostable serine protease. Such control sequences include, but are not limited to, a leader, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a thermostable serine protease.

The control sequence may be an appropriate promoter region, a nucleotide sequence that is recognized by a *Bacillus* host cell for expression of the polynucleotide encoding a thermostable serine protease. The promoter region contains transcription control sequences that mediate the expression of a thermostable serine protease. The promoter region may be any nucleotide sequence that shows transcriptional activity in the *Bacillus* host cell of choice and may be obtained from genes directing synthesis of extracellular or intracellular polypeptides having biological activity either homologous or heterologous to the *Bacillus* host cell.

The promoter region may comprise a single promoter or a combination of promoters. Where the promoter region comprises a combination of promoters, the promoters are preferably in tandem. A promoter of the promoter region can be any promoter that can initiate transcription of a polynucleotide encoding a polypeptide having biological activity in a *Bacillus* host cell of interest. The promoter may be native, foreign, or a combination thereof, to the nucleotide sequence encoding a polypeptide having biological activity. Such a promoter can be obtained from genes directing synthesis of extracellular or intracellular polypeptides having biological activity either homologous or heterologous to the *Bacillus* host cell.

Thus, in certain embodiments, the promoter region comprises a promoter obtained from a bacterial source. In other embodiments, the promoter region comprises a promoter obtained from a Gram positive or Gram negative bacterium. Gram positive bacteria include, but are not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but are not limited to, *E. coli, Pseudomonas, Salmonella, Campylobacter, Helicobacter, Flavobacterium, Fusobacterium, Ilyobacter, Neisseria,* and *Ureaplasma.*

The promoter region may comprise a promoter obtained from a *Bacillus* strain (*e.g., Bacillus agaradherens, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, or Bacillus thuringiensis;* or from a *Streptomyces* strain (*e.g., Streptomyces lividans* or *Streptomyces murinus*).

Examples of suitable promoters for directing transcription of a polynucleotide encoding a polypeptide having biological activity in the methods of the present disclosure are the promoters obtained from the *E. coli lac* operon, *Streptomyces coelicolor* agarase gene (dagA), *Bacillus lentus* or *Bacillus clausii* alkaline protease gene (aprH), *Bacillus licheniformis* alkaline protease gene (subtilisin Carlsberg gene), *Bacillus subtilis* levansucrase gene (sacB), *Bacillus subtilis* alpha-amylase gene (amyE), *Bacillus licheniformis* alpha-amylase gene (amyL), *Bacillus stearothermophilus* maltogenic amylase gene (amyM), *Bacillus amyloliquefaciens* alpha-amylase gene (amyQ), *Bacillus licheniformis* penicillinase gene (penP), *Bacillus subtilis* xylA and xylB genes, *Bacillus thuringiensis* subsp. tenebfionis CryIIIA gene (cryIIIA) or portions thereof, prokaryotic beta-lactamase gene (Villa-Kamaroff et al., 1978, Proceedings of the National Academy of Sciences USA 75:3727-3731), and *Bacillus megaterium* xylA gene (Rygus and Hillen, 1992, J. Bacteriol. 174: 3049-3055; Kim et al., 1996, Gene 181: 71-76). Other examples are the promoter of the spo1 bacterial phage promoter and the tac promoter (DeBoer et al., 1983, Proceedings of the National Academy of Sciences USA 80:21-25). Further promoters are described in "Useful proteins from recombinant bacteria" in Scientific American, 1980, 242:74-94; and in Sambrook, Fritsch, and Maniatis, 1989, Molecular Cloning, A Laboratory Manual, 2d edition, Cold Spring Harbor, N.Y.

The promoter region may comprise a promoter that is a "consensus" promoter having the sequence TTGACA for the "-35" region and TATAAT for the "-10" region. The consensus promoter may be obtained from any promoter that can function in a *Bacillus* host cell. The construction of a "consensus" promoter may be accomplished by site-directed mutagenesis using methods well known in the art to create a promoter that conforms more perfectly to the established consensus sequences for the "-10" and "-35" regions of the vegetative "sigma A-type" promoters for *Bacillus subtilis* (Voskuil et al., 1995, Molecular Microbiology 17: 271-279).

A control sequence may also be a suitable transcription terminator sequence, such as a sequence recognized by a *Bacillus* host cell to terminate transcription. The terminator sequence is operably linked to the 3' terminus of the nucleotide sequence encoding a thermostable serine protease. Any terminator that is functional in the *Bacillus* host cell may be used.

The control sequence may also be a suitable leader sequence, a non-translated region of a mRNA that is important for translation by a *Bacillus* host cell. The leader sequence is operably linked to the 5' terminus of the nucleotide sequence directing synthesis of the polypeptide having biological activity. Any leader sequence that is functional in a *Bacillus* host cell of choice may be used in the present invention.

The control sequence may also be a mRNA stabilizing sequence. The term "mRNA stabilizing sequence" is defined herein as a sequence located downstream of a promoter region and upstream of a coding sequence of a polynucleotide encoding a thermostable serine protease to which the promoter region is operably linked, such that all mRNAs synthesized from the promoter region may be processed to generate mRNA transcripts with a stabilizer sequence at the 5' end of the transcripts. For example, the presence of such a stabilizer sequence at the 5' end of the mRNA transcripts increases their half-life (Agaisse and Lereclus, 1994, supra, Hue et al., 1995, Journal of Bacteriology 177: 3465-3471). The mRNA processing/stabilizing sequence is complementary to the 3' extremity of bacterial 16S ribosomal RNA. In certain embodiments, the mRNA processing/stabilizing sequence generates essentially single-size transcripts with a stabilizing sequence at the 5' end of the transcripts. The mRNA processing/stabilizing sequence is preferably one, which is complementary to the 3' extremity of a bacterial 16S ribosomal RNA. *See,* U.S. Patent No. 6,255,076 and U.S. Patent No. 5,955,310.

The nucleic acid construct can then be introduced into a *Bacillus* host cell using methods known in the art or those methods described herein for introducing and expressing a thermostable serine protease.

A nucleic acid construct comprising a DNA of interest encoding a protein of interest can also be constructed similarly as described above.

For obtaining secretion of the protein of interest of the introduced DNA, the control sequence may also comprise a signal peptide coding region, which codes for an amino acid sequence linked to the amino terminus of a polypeptide that can direct the expressed polypeptide into the cell's secretory pathway. The signal peptide coding region may be native to the polypeptide or may be obtained from foreign sources. The 5' end of the coding sequence of the nucleotide sequence may inherently contain a signal peptide coding region naturally linked in translation reading frame with the segment of the coding region that encodes the secreted polypeptide. Alternatively, the 5' end of the coding sequence may contain a signal peptide coding region that is foreign to that portion of the coding sequence that encodes the secreted polypeptide. The foreign signal peptide coding region may be required where the coding sequence does not normally contain a signal peptide coding region. Alternatively, the foreign signal peptide coding region may simply replace the natural signal peptide coding region in order to obtain enhanced secretion of the polypeptide relative to the natural signal peptide coding region normally associated with the coding sequence. The signal peptide coding region may be obtained from an amylase or a protease gene from a *Bacillus* species. However, any signal peptide coding region capable of directing the expressed polypeptide into the secretory pathway of a *Bacillus* host cell of choice may be used in the present invention.

An effective signal peptide coding region for a *Bacillus* host cell, is the signal peptide coding region obtained from the maltogenic amylase gene from *Bacillus* NCIB 11837, the *Bacillus stearothermophilus* alpha-amylase gene, the *Bacillus licheniformis* subtilisin gene, the *Bacillus licheniformis* beta-lactamase gene, the *Bacillus stearothermophilus* neutral proteases genes (nprT, nprS, nprM), and the *Bacillus subtilis* prsA gene.

Thus, a polynucleotide construct comprising a nucleic acid encoding a thermostable serine protease construct comprising a nucleic acid encoding a polypeptide of interest (POI) can be constructed such that it is expressed by a host cell. Because of the known degeneracies in the genetic code, different polynucleotides encoding an identical amino acid sequence can be designed and made with routine skills in the art. For example, codon optimizations can be applied to optimize production in a particular host cell.

Nucleic acids encoding proteins of interest can be incorporated into a vector, wherein the vector can be transferred into a host cell using well-known transformation techniques, such as those disclosed herein.

The vector may be any vector that can be transformed into and replicated within a host cell. For example, a vector comprising a nucleic acid encoding a POI can be transformed and replicated in a bacterial host cell as a means of propagating and amplifying the vector. The vector also may be transformed into a *Bacillus* expression host of the disclosure, so that the protein encoding nucleic acid (*e.g*., an ORF) can be expressed as a functional protein.

A representative vector which can be modified with routine skill to comprise and express a nucleic acid encoding a POI is vector p2JM103BBI.

A polynucleotide encoding a thermostable serine protease or a POI can be operably linked to a suitable promoter, which allows transcription in the host cell. The promoter may be any nucleic acid sequence that shows transcriptional activity in the host cell of choice and may be derived from genes encoding proteins either homologous or heterologous to the host cell. Means of assessing promoter activity/strength are routine for the skilled artisan.

Examples of suitable promoters for directing the transcription of a polynucleotide sequence encoding comS1 polypeptide or a POI of the disclosure, especially in a bacterial host, include the promoter of the lac operon of *E. coli,* the *Streptomyces coelicolor* agarase gene *dagA* or *celA* promoters, the promoters of the *Bacillus licheniformis* alpha-amylase gene (*amyL*), the promoters of the *Bacillus stearothermophilus* maltogenic amylase gene (*amyM*), the promoters of the *Bacillus amyloliquefaciens* alpha-amylase (amyQ), the promoters of the *Bacillus subtilis xylA* and *xylB* genes, and the like.

A promoter for directing the transcription of a polynucleotide sequence encoding a POI can be a wild-type *aprE* promoter, a mutant *aprE* promoter or a consensus *aprE* promoter set forth in PCT International Publication No. WO2001/51643. In certain other embodiments, a promoter for directing the transcription of a polynucleotide sequence encoding a POI is a wild-type *spoVG* promoter, a mutant *spoVG* promoter, or a consensus *spoVG* promoter (Frisby and Zuber, 1991).

A promoter for directing the transcription of the polynucleotide sequence encoding a thermostable serine protease or a POI is a ribosomal promoter such as a ribosomal RNA promoter or a ribosomal protein promoter. The ribosomal RNA promoter can be a *rrn* promoter derived from *B. subtilis,* more particularly, the *rrn* promoter can be a *rrnB, rrnI* or *rrnE* ribosomal promoter from *B. subtilis.* In certain embodiments, the ribosomal RNA promoter is a P2 *rrnI* promoter from *B. subtilis* set forth in PCT International Publication No. WO2013/086219.

A suitable vector may further comprise a nucleic acid sequence enabling the vector to replicate in the host cell. Examples of such enabling sequences include the origins of replication of plasmids pUC19, pACYC177, pUB110, pE194, pAMB1, pIJ702, and the like.

A suitable vector may also comprise a selectable marker, *e.g.*, a gene the product of which complements a defect in the isolated host cell, such as the *dal* genes from *B. subtilis* or *B. licheniformis*; or a gene that confers antibiotic resistance such as, *e.g.*, ampicillin resistance, kanamycin resistance, chloramphenicol resistance, tetracycline resistance and the like.

A suitable expression vector typically includes components of a cloning vector, such as, for example, an element that permits autonomous replication of the vector in the selected host organism and one or more phenotypically detectable markers for selection purposes. Expression vectors typically also comprise control nucleotide sequences such as, for example, promoter, operator, ribosome binding site, translation initiation signal and optionally, a repressor gene, one or more activator genes sequences, or the like.

Additionally, a suitable expression vector may further comprise a sequence coding for an amino acid sequence capable of targeting the protein of interest to a host cell organelle such as a peroxisome, or to a particular host cell compartment. Such a targeting sequence may be, for example, the amino acid sequence "SKL". For expression under the direction of control sequences, the nucleic acid sequence of the protein of interest can be operably linked to the control sequences in a suitable manner such that the expression takes place.

Protocols, such as described herein, used to ligate the DNA construct encoding a protein of interest, promoters, terminators and/or other elements, and to insert them into suitable vectors containing the information necessary for replication, are well known to persons skilled in the art.

An isolated cell, either comprising a polynucleotide construct or an expression vector, is advantageously used as a host cell in the recombinant production of a POI. The cell may be transformed with the DNA construct encoding the POI, conveniently by integrating the construct (in one or more copies) into the host chromosome. Integration is generally deemed an advantage, as the DNA sequence thus introduced is more likely to be stably maintained in the cell. Integration of the DNA constructs into the host chromosome may be performed applying conventional methods, for example, by homologous or heterologous recombination. For example, PCT International Publication No. WO2002/14490 describes methods of *Bacillus* transformation, transformants thereof and libraries thereof. Alternatively, the cell may be transformed with an expression vector as described above in connection with the different types of host cells.

It is, in other embodiments, advantageous to delete genes from expression hosts, where the gene deficiency can be cured by an expression vector. Known methods may be used to obtain a bacterial host cell having one or more inactivated genes. Gene inactivation may be accomplished by complete or partial deletion, by insertional inactivation or by any other means that renders a gene nonfunctional for its intended purpose, such that the gene is prevented from expression of a functional protein.

Techniques for transformation of bacteria and culturing the bacteria are standard and well known in the art. They can be used to transform the improved hosts of the present invention for the production of recombinant proteins of interest. Introduction of a DNA construct or vector into a host cell includes techniques such as transformation, electroporation, nuclear microinjection, transduction, transfection (*e.g.*, lipofection mediated and DEAE-Dextrin mediated transfection), incubation with calcium phosphate DNA precipitate, high velocity bombardment with DNA-coated microprojectiles, gene gun or biolistic transformation and protoplast fusion, and the like. Transformation and expression methods for bacteria are also disclosed in Brigidi *et al.* (1990). A general transformation and expression protocol for protease deleted *Bacillus* strains is described in Ferrari *et al.* (U.S. Patent No. 5, 264,366).

Methods for transforming nucleic acids into filamentous fungi such as *Aspergillus spp., e.g., A. oryzae* or *A. niger, H. grisea, H. insolens,* and *T. reesei.* are well known in the art. A suitable procedure for transformation of *Aspergillus* host cells is described, for example, in EP238023. A suitable procedure for transformation of *Trichoderma* host cells is described, for example, in Steiger et al 2011, Appl. Environ. Microbiol. 77:114-121.

The choice of a production host can be any suitable microorganism such as bacteria, fungi and algae.

Typically, the choice will depend upon the gene encoding the thermostable serine protease and its source.

Introduction of a DNA construct or vector into a host cell includes techniques such as transformation; electroporation; nuclear microinjection; transduction; transfection, (e.g., lipofection mediated and DEAE-Dextrin mediated transfection); incubation with calcium phosphate DNA precipitate; high velocity bombardment with DNA-coated microprojectiles; and protoplast fusion. Basic texts disclosing the general methods that can be used include Sambrook et al., Molecular Cloning, A Laboratory Manual (2nd ed. 1989); Kriegler, Gene Transfer and Expression: A Laboratory Manual (1990); and Ausubel et al., eds., Current Protocols in Molecular Biology (1994)). The methods of transformation of the present invention may result in the stable integration of all or part of the transformation vector into the genome of a host cell, such as a filamentous fungal host cell. However, transformation resulting in the maintenance of a self-replicating extra-chromosomal transformation vector is also contemplated.

Many standard transfection methods can be used to produce bacterial and filamentous fungal (e.g. *Aspergillus* or *Trichoderma*) cell lines that express large quantities of the protease. Some of the published methods for the introduction of DNA constructs into cellulase-producing strains of *Trichoderma* include Lorito, Hayes, DiPietro and Harman, (1993) Curr. Genet. 24: 349-356; Goldman, VanMontagu and Herrera-Estrella, (1990) Curr. Genet. 17:169-174; and Penttila, Nevalainen, Ratto, Salminen and Knowles, (1987) Gene 6: 155-164, also see USP 6.022,725; USP 6,268,328 and Nevalainen et al., "The Molecular Biology of Trichoderma and its Application to the Expression of Both Homologous and Heterologous Genes" in Molecular Industrial Mycology, Eds, Leong and Berka, Marcel Dekker Inc., NY (1992) pp 129 - 148; for *Aspergillus* include Yelton, Hamer and Timberlake, (1984) Proc. Natl. Acad. Sci. USA 81: 1470-1474, for *Fusarium* include Bajar, Podila and Kolattukudy, (1991) Proc. Natl. Acad. Sci. USA 88: 8202-8212, for *Streptomyces* include Hopwood et al., 1985, Genetic Manipulation of Streptomyces: Laboratory Manual, The John Innes Foundation, Norwich, UK and Fernandez-Abalos et al., Microbiol 149:1623 - 1632 (2003) and for *Bacillus* include Brigidi, DeRossi, Bertarini, Riccardi and Matteuzzi, (1990) FEMS Microbiol. Lett. 55: 135-138).
However, any of the well-known procedures for introducing foreign nucleotide sequences into host cells may be used. These include the use of calcium phosphate transfection, polybrene, protoplast fusion, electroporation, biolistics, liposomes, microinjection, plasma vectors, viral vectors and any of the other well-known methods for introducing cloned genomic DNA, cDNA, synthetic DNA or other foreign genetic material into a host cell (see, e.g., Sambrook *et al., supra*). Also of use is the Agrobacterium-mediated transfection method described in U.S. Patent No. 6,255,115. It is only necessary that the particular genetic engineering procedure used be capable of successfully introducing at least one gene into the host cell capable of expressing the gene.

After the expression vector is introduced into the cells, the transfected or transformed cells are cultured under conditions favoring expression of genes under control of the promoter sequences.

The medium used to cultivate the cells may be any conventional medium suitable for growing the host cell and obtaining expression of an alpha-glucosidase polypeptide. Suitable media and media components are available from commercial suppliers or may be prepared according to published recipes (e.g., as described in catalogues of the American Type Culture Collection).

A thermostable serine polypeptide secreted from the host cells can be used, with minimal post-production processing, as a whole broth preparation.

Depending upon the host cell used post-transcriptional and/or post-translational modifications may be made. One non-limiting example of a post-transcriptional and/or post-translational modification is "clipping" or "truncation" of a polypeptide. For example, this may result in taking a thermostable serine protease from an inactive or substantially inactive state to an active state as in the case of a pro-peptide undergoing further post-translational processing to a mature peptide having the enzymatic activity. In another instance, this clipping may result in taking a mature thermostable serine protease polypeptide and further removing N or C-terminal amino acids to generate truncated forms of the thermostable serine protease that retain enzymatic activity.

Other examples of post-transcriptional or post-translational modifications include, but are not limited to, myristoylation, glycosylation, truncation, lipidation and tyrosine, serine or threonine phosphorylation. The skilled person will appreciate that the type of post-transcriptional or post-translational modifications that a protein may undergo may depend on the host organism in which the protein is expressed.

In some embodiments, a lipase and/or a phytase can be added to any one step of the fermentation process.

In some embodiments, the preparation of a spent whole fermentation broth of a recombinant microorganism can be achieved using any cultivation method known in the art resulting in the expression of a thermostable serine protease.

Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the alpha-glucosidase to be expressed or isolated. The term "spent whole fermentation broth" is defined herein as unfractionated contents of fermentation material that includes culture medium, extracellular proteins (*e.g*., enzymes), and cellular biomass. It is understood that the term "spent whole fermentation broth" also encompasses cellular biomass that has been lysed or permeabilized using methods well known in the art.

Host cells may be cultured under suitable conditions that allow expression of a thermostable serine protease. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or sophorose.

Any of the fermentation methods well known in the art can suitably be used to ferment the transformed or the derivative fungal strain as described above. In some embodiments, fungal cells are grown under batch or continuous fermentation conditions.

A classical batch fermentation is a closed system, where the composition of the medium is set at the beginning of the fermentation, and the composition is not altered during the fermentation. At the beginning of the fermentation, the medium is inoculated with the desired organism(s). In other words, the entire fermentation process takes place without addition of any components to the fermentation system throughout.

Alternatively, a batch fermentation qualifies as a "batch" with respect to the addition of the carbon source. Moreover, attempts are often made to control factors such as pH and oxygen concentration throughout the fermentation process. Typically, the metabolite and biomass compositions of the batch system change constantly up to the time the fermentation is stopped. Within batch cultures, cells progress through a static lag phase to a high growth log phase and finally to a stationary phase, where growth rate is diminished or halted. Left untreated, cells in the stationary phase would eventually die. In general, cells in log phase are responsible for the bulk of production of product. A suitable variation on the standard batch system is the "fed-batch fermentation" system. In this variation of a typical batch system, the substrate is added in increments as the fermentation progresses. Fed-batch systems are useful when it is known that catabolite repression would inhibit the metabolism of the cells, and/or where it is desirable to have limited amounts of substrates in the fermentation medium. Measurement of the actual substrate concentration in fed-batch systems is difficult and is therefore estimated on the basis of the changes of measurable factors, such as pH, dissolved oxygen and the partial pressure of waste gases, such as CO2. Batch and fed-batch fermentations are well known in the art.

Continuous fermentation is another known method of fermentation. It is an open system where a defined fermentation medium is added continuously to a bioreactor, and an equal amount of conditioned medium is removed simultaneously for processing. Continuous fermentation generally maintains the cultures at a constant density, where cells are maintained primarily in log phase growth. Continuous fermentation allows for the modulation of one or more factors that affect cell growth and/or product concentration. For example, a limiting nutrient, such as the carbon source or nitrogen source, can be maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

Separation and concentration techniques are known in the art and conventional methods can be used to prepare a concentrated solution or broth comprising a thermostable serine protease polypeptide of the invention.

After fermentation, a fermentation broth is obtained, the microbial cells and various suspended solids, including residual raw fermentation materials, are removed by conventional separation techniques in order to obtain a thermostable serine protease solution. Filtration, centrifugation, microfiltration, rotary vacuum drum filtration, ultrafiltration, centrifugation followed by ultra- filtration, extraction, or chromatography, or the like, are generally used.

It may at times be desirable to concentrate a solution or broth comprising an alpha- glucosidase polypeptide to optimize recovery. Use of un-concentrated solutions or broth would typically increase incubation time in order to collect the enriched or purified enzyme precipitate.

The enzyme-containing solution can be concentrated using conventional concentration techniques until the desired enzyme level is obtained. Concentration of the enzyme containing solution may be achieved by any of the techniques discussed herein. Examples of methods of enrichment and purification include but are not limited to rotary vacuum filtration and/or ultrafiltration.

The thermostable serine protease-containing solution or broth may be concentrated until such time the enzyme activity of the concentrated a thermostable serine protease polypeptide-containing solution or broth is at a desired level.

Concentration may be performed using, *e.g.,* a precipitation agent, such as a metal halide precipitation agent. Metal halide precipitation agents include but are not limited to alkali metal chlorides, alkali metal bromides and blends of two or more of these metal halides.

Exemplary metal halides include sodium chloride, potassium chloride, sodium bromide, potassium bromide and blends of two or more of these metal halides. The metal halide precipitation agent, sodium chloride, can also be used as a preservative. For production scale recovery, thermostable serine protease polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

Thermostable serine proteases may be isolated or purified in a variety of ways known to those skilled in the art depending on what other components are present in the sample. Standard purification methods include, but are not limited to, chromatography (e.g., ion exchange, affinity, hydrophobic, chromatofocusing, immunological and size exclusion), electrophoretic procedures (e.g., preparative isoelectric focusing), differential solubility (e.g., ammonium sulfate precipitation), extraction microfiltration, two phase separation and crystallization. For example, the protein of interest may be purified using a standard anti-protein of interest antibody column. Ultrafiltration and diafiltration techniques, in conjunction with protein concentration, are also useful. For general guidance in suitable purification techniques, see Scopes, Protein Purification (1982). The degree of purification necessary will vary depending on the use of the protein of interest. In some instances, no purification will be necessary.

Assays for detecting and measuring the enzymatic activity of an enzyme are well known. Various assays for detecting and measuring activity of proteases (such as the thermostable serine protease polypeptides described herein), are also known to those of ordinary skill in the art. In particular, assays are available for measuring protease activity that are based on the release of acid-soluble peptides from casein or hemoglobin, measured as absorbance at 280 nm or colorimetrically using the Folin method, and hydrolysis of the dye-labeled azocasein, measured as absorbance at 440-450 nm

Other exemplary assays involve the solubilization of chromogenic substrates (See e.g., Ward, "Proteinases," in Fogarty (ed.)., Microbial Enzymes and Biotechnology, Applied Science, London, [1983], pp. 251-317). A protease detection assay method using highly labeled fluorescein isothiocyanate (FITC) casein as the substrate, a modified version of the procedure described by Twining [Twining, S.S., (1984) "Fluorescein Isothiocyanate-Labeled Casein Assay for Proteolytic Enzymes" Anal. Biochem. 143:30-34] may also be used.
Other exemplary assays include, but are not limited to: cleavage of casein into trichloroacetic acid-soluble peptides containing tyrosine and tryptophan residues, followed by reaction with Folin-Ciocalteu reagent and colorimetric detection of products at 660 nm, cleavage of internally quenched FRET (Fluorescence Resonance Energy Transfer) peptide substrates followed by detection of product using a fluorometer. Fluorescence Resonance Energy Transfer (FRET) is the non-radiative transfer of energy from an excited fluorophore (or donor) to a suitable quencher (or acceptor) molecule. FRET is used in a variety of applications including the measurement of protease activity with substrates, in which the fluorophore is separated from the quencher by a short peptide sequence containing the enzyme cleavage site. Proteolysis of the peptide results in fluorescence as the fluorophore and quencher are separated. Numerous additional references known to those in the art provide suitable methods (See e.g., Wells et al., Nucleic Acids Res. 11:7911-7925 [1983]; Christianson et al., Anal. Biochem. 223:119 -129 [1994]; and Hsia et al., Anal Biochem. 242:221-227 [1999]).

In still another aspect, there is disclosed a feed, feedstuff, feed additive composition, premix, food or grain product comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 either alone or in combination with at least one direct fed microbial.

At least one DFM may comprise at least one viable microorganism such as a viable bacterial strain or a viable yeast or viable fungi. Preferably, the DFM comprises at least one viable bacteria.

It is possible that the DFM may be a spore forming bacterial strain and hence the term DFM may be comprised of or contain spores, e.g. bacterial spores. Thus, the term "viable microorganism" as used herein may include microbial spores, such as endospores or conidia. Alternatively, the DFM in the feed additive composition described herein may not comprise of or may not contain microbial spores, e.g. endospores or conidia.

The microorganism may be a naturally-occurring microorganism or it may be a transformed microorganism.

A DFM as described herein may comprise microorganims from one or more of the following genera: *Lactobacillus, Lactococcus, Streptococcus, Bacillus, Pediococcus, Enterococcus, Leuconostoc, Carnobacterium, Propionibacterium, Bifidobacterium, Clostridium* and *Megasphaera* and combinations thereof.

Preferably, the DFM comprises one or more bacterial strains selected from the following *Bacillus* spp: *Bacillus subtilis, Bacillus cereus, Bacillus licheniformis, Bacillus pumilis* and *Bacillus amyloliquefaciens.*

The genus "Bacillus", as used herein, includes all species within the genus "Bacillus," as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. gibsonii, B. pumilis* and *B. thuringiensis.* It is recognized that the genus Bacillus continues to undergo taxonomical reorganization. Thus, it is intended that the genus include species that have been reclassified, including but not limited to such organisms as *Bacillus stearothermophilus,* which is now named *"Geobacillus stearothermophilus",* or *Bacillus polymyxa,* which is now *"Paenibacillus polymyxa"* The production of resistant endospores under stressful environmental conditions is considered the defining feature of the genus Bacillus, although this characteristic also applies to the recently *named Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

In another aspect, the DFM may be further combined with the following *Lactococcus* spp: *Lactococcus cremoris* and *Lactococcus lactis* and combinations thereof. The DFM may be further combined with the following *Lactobacillus* spp: *Lactobacillus buchneri, Lactobacillus acidophilus, Lactobacillus casei, Lactobacillus kefiri, Lactobacillus bifidus, Lactobacillus brevis, Lactobacillus helveticus, Lactobacillus paracasei, Lactobacillus rhamnosus, Lactobacillus salivarius, Lactobacillus curvatus, Lactobacillus bulgaricus, Lactobacillus sakei, Lactobacillus reuteri, Lactobacillus fermentum, Lactobacillus farciminis, Lactobacillus lactis, Lactobacillus delbreuckii, Lactobacillus plantarum, Lactobacillus paraplantarum, Lactobacillus farciminis, Lactobacillus rhamnosus, Lactobacillus crispatus, Lactobacillus gasseri, Lactobacillus johnsonii* and *Lactobacillus jensenii,* and combinations of any thereof.

In still another aspect, the DFM may be further combined with the following *Bifidobacteria* spp: *Bifidobacterium lactis, Bifidobacterium bifidium, Bifidobacterium longum, Bifidobacterium animalis, Bifidobacterium breve, Bifidobacterium infantis, Bifidobacterium catenulatum, Bifidobacterium pseudocatenulatum, Bifidobacterium adolescentis,* and *Bifidobacterium angulatum,* and combinations of any thereof.

There can be mentioned bacteria of the following species: *Bacillus subtilis, Bacillus licheniformis, Bacillus amyloliquefaciens, Bacillus pumilis, Enterococcus , Enterococcus* spp, and *Pediococcus* spp, *Lactobacillus* spp, *Bifidobacterium* spp, *Lactobacillus acidophilus, Pediococsus acidilactici, Lactococcus lactis, Bifidobacterium bifidum, Bacillus subtilis, Propionibacterium thoenii, Lactobacillus farciminis, Lactobacillus rhamnosus, Megasphaera elsdenii, Clostridium butyricum, Bifidobacterium animalis* ssp. *animalis, Lactobacillus reuteri, Bacillus cereus, Lactobacillus salivarius* ssp. *Salivarius, Propionibacteria sp* and combinations thereof.

A direct-fed microbial described herein comprising one or more bacterial strains may be of the same type (genus, species and strain) or may comprise a mixture of genera, species and/or strains.

Alternatively, a DFM may be combined with one or more of the products or the microorganisms contained in those products disclosed in WO2012110778, and summarized as follows: *Bacillus subtilis* strain 2084 Accession No. NRRl B-50013, *Bacillus subtilis* strain LSSAO1 Accession No. NRRL B-50104, and *Bacillus subtilis* strain 15A-P4 ATCC Accession No. PTA-6507 (from Enviva Pro®. (formerly known as Avicorr®); *Bacillus subtilis* Strain C3102 (from Calsporin®); *Bacillus subtilis* Strain PB6 (from Clostat®); *Bacillus pumilis* (8G-*134); Enterococcus* NCIMB 10415 (SF68) (from Cylactin®); *Bacillus subtilis* Strain C3102 (from Gallipro® & GalliproMax®); *Bacillus licheniformis* (from Gallipro®Tect®); *Enterococcus* and *Pediococcus* (from Poultry star®); *Lactobacillus, Bifidobacterium* and/or *Enterococcus* from Protexin®); *Bacillus subtilis* strain QST 713 (from Proflora®); *Bacillus amyloliquefaciens* CECT-5940 (from Ecobiol® & Ecobiol® Plus); *Enterococcus faecium* SF68 (from Fortiflora®); *Bacillus subtilis* and *Bacillus licheniformis* (from BioPlus2B®); Lactic acid bacteria 7 *Enterococcus faecium* (from Lactiferm®); *Bacillus* strain (from CSI®); *Saccharomyces cerevisiae* (from Yea-Sacc®); *Enterococcus* (from Biomin IMB52®); *Pediococcus acidilactici, Enterococcus, Bifidobacterium animalis* ssp. *animalis, Lactobacillus reuteri, Lactobacillus salivarius* ssp. *salivarius* (from Biomin C5®); *Lactobacillus farciminis* (from Biacton®); *Enterococcus* (from Oralin E1707®); *Enterococcus* (2 strains), *Lactococcus lactis* DSM 1103(from Probios-pioneer PDFM®); *Lactobacillus rhamnosus and Lactobacillus farciminis* (from Sorbiflore®); *Bacillus subtilis* (from Animavit®); *Enterococcus* (from Bonvital®); *Saccharomyces cerevisiae* (from Levucell SB 20®); *Saccharomyces cerevisiae* (from Levucell SC 0 & SC10® ME); *Pediococcus acidilacti* (from Bactocell); *Saccharomyces cerevisiae* (from ActiSaf® (formerly BioSaf®)); *Saccharomyces cerevisiae* NCYC Sc47 (from Actisaf® SC47); *Clostridium butyricum* (from Miya-Gold®); *Enterococcus* (from Fecinor and Fecinor Plus®); *Saccharomyces cerevisiae* NCYC R-625 (from InteSwine®); *Saccharomyces cerevisia* (from BioSprint®); *Enterococcus* and *Lactobacillus rhamnosus* (from Provita®); *Bacillus subtilis and Aspergillus oryzae* (from PepSoyGen-C®); *Bacillus cereus* (from Toyocerin®); *Bacillus cereus* var. *toyoi* NCIMB 40112/CNCM I-1012 (from TOYOCERIN®), or other DFMs such as *Bacillus licheniformis* and *Bacillus subtilis* (from BioPlus® YC) and *Bacillus subtilis* (from GalliPro®).

The DFM may be combined with Enviva® PRO which is commercially available from Danisco A/S. Enviva Pro® is a combination of *Bacillus* strain 2084 Accession No. NRRl B-*50013, Bacillus* strain LSSAO1 Accession No. NRRL B-50104 and *Bacillus* strain 15A-P4 ATCC Accession No. PTA-6507 (as taught in US 7,754,469 B - incorporated herein by reference).

It is also possible to combine the DFM described herein with a yeast from the genera: *Saccharomyces spp.*

Preferably, the DFM described herein comprises microorganisms which are generally recognized as safe (GRAS) and, preferably are GRAS-approved.

A person of ordinary skill in the art will readily be aware of specific species and/or strains of microorganisms from within the genera described herein which are used in the food and/or agricultural industries and which are generally considered suitable for animal consumption.

In some embodiments, it is important that the DFM be heat tolerant, i.e. is thermotolerant. This is particularly the case when the feed is pelleted. Therefore, in another embodiment, the DFM may be a thermotolerant microorganism, such as a thermotolerant bacteria, including for example *Bacillus* spp.

In other aspects, it may be desirable that the DFM comprises a spore producing bacteria, such as *Bacilli,* e.g. *Bacillus* spp. Bacilli are able to form stable endospores when conditions for growth are unfavorable and are very resistant to heat, pH, moisture and disinfectants.

The DFM described herein may decrease or prevent intestinal establishment of pathogenic microorganism (such as *Clostridium perfringens* and/or *E. coli* and/or *Salmonella* spp and/or *Campylobacter* spp.). In other words, the DFM may be antipathogenic. The term "antipathogenic" as used herein means the DFM counters an effect (negative effect) of a pathogen.

As described above, the DFM may be any suitable DFM. For example, the following assay "DFM ASSAY" may be used to determine the suitability of a microorganism to be a DFM. The DFM assay as used herein is explained in more detail in US2009/0280090. For avoidance of doubt, the DFM selected as an inhibitory strain (or an antipathogenic DFM) in accordance with the "DFM ASSAY" taught herein is a suitable DFM for use in accordance with the present disclosure, i.e. in the feed additive composition according to the present disclosure.

Tubes were seeded each with a representative pathogen (e.g., bacteria) from a representative cluster.

Supernatant from a potential DFM, grown aerobically or anaerobically, is added to the seeded tubes (except for the control to which no supernatant is added) and incubated. After incubation, the optical density (OD) of the control and supernatant treated tubes was measured for each pathogen.

Colonies of (potential DFM) strains that produced a lowered OD compared with the control (which did not contain any supernatant) can then be classified as an inhibitory strain (or an antipathogenic DFM). Thus, The DFM assay as used herein is explained in more detail in US2009/0280090.

Preferably, a representative pathogen used in this DFM assay can be one (or more) of the following: Clostridium, such as *Clostridium perfringens* and/or *Clostridium difficile,* and/or *E. coli* and/or *Salmonella* spp and/or *Campylobacter* spp. In one preferred embodiment the assay is conducted with one or more of *Clostridium perfringens* and/or *Clostridium difficile* and/or *E. coli,* preferably *Clostridium perfringens* and/or *Clostridium difficile,* more preferably *Clostridium perfringens.*

Antipathogenic DFMs include one or more of the following bacteria and are described in WO2013029013: *Bacillus subtilis* strain 3BP5 Accession No. NRRL B-50510, *Bacillus subtilis* strain 918 ATCC Accession No. NRRL B-50508, and *Bacillus subtilis* strain 1013 ATCC Accession No. NRRL B-50509.

DFMs may be prepared as culture(s) and carrier(s) (where used) and can be added to a ribbon or paddle mixer and mixed for about 15 minutes, although the timing can be increased or decreased. The components are blended such that a uniform mixture of the cultures and carriers result. The final product is preferably a dry, flowable powder. The DFM(s) comprising one or more bacterial strains can then be added to animal feed or a feed premix, added to an animal's water, or administered in other ways known in the art (preferably simultaneously with the enzymes described herein.

Inclusion of the individual strains in the DFM mixture can be in proportions varying from 1% to 99% and, preferably, from 25% to 75%.

Suitable dosages of the DFM in animal feed may range from about $1x10^3$ CFU/g feed to about $1x10^{10}$ CFU/g feed, suitably between about $1x10^4$ CFU/g feed to about $1x10^8$ CFU/g feed, suitably between about $7.5x10^4$ CFU/g feed to about $1x10^7$ CFU/g feed.

In another aspect, the DFM may be dosed in feedstuff at more than about $1x10^3$ CFU/g feed, suitably more than about $1x10^4$ CFU/g feed, suitably more than about $5x10^4$ CFU/g feed, or suitably more than about $1x10^5$ CFU/g feed.

The DFM may be dosed in a feed additive composition from about $1x10^3$ CFU/g composition to about $1x10^{13}$ CFU/g composition, preferably $1x10^5$ CFU/g composition to about $1x10^{13}$ CFU/g composition, more preferably between about $1x10^6$ CFU/g composition to about $1x10^{12}$ CFU/g composition, and most preferably between about $3.75x10^7$ CFU/g composition to about $1x10^{11}$ CFU/g composition. In another aspect, the DFM may be dosed in a feed additive composition at more than about $1x10^5$ CFU/g composition, preferably more than about $1x10^6$ CFU/g composition, and most preferably more than about $3.75x10^7$ CFU/g composition. In one embodiment the DFM is dosed in the feed additive composition at more than about $2x10^5$ CFU/g composition, suitably more than about $2x10^6$ CFU/g composition, suitably more than about $3.75x10^7$ CFU/g composition.

A feed additive composition for use in animal feed may comprise a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10, used either alone or in combination with at least one direct fed microbial, and at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

In still another aspect, there is disclosed a granulated feed additive composition for use in animal feed comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10, used either alone or in combination with at least one direct fed microbial, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

Furthermore, the particles of the granulated feed additive composition can have a mean diameter of greater than 50 microns and less than 2000 microns.

The feed additive composition can be a liquid form and the liquid form can also be said suitable for spray-drying on a feed pellet.

Animal feeds may include plant material such as corn, wheat, sorghum, soybean, canola, sunflower or mixtures of any of these plant materials or plant protein sources for poultry, pigs, ruminants, aquaculture and pets. It is contemplated that animal performance parameters, such as growth, feed intake and feed efficiency, but also improved uniformity, reduced ammonia concentration in the animal house and consequently improved welfare and health status of the animals will be improved. More specifically, as used herein, "animal performance" may be determined by the feed efficiency and/or weight gain of the animal and/or by the feed conversion ratio and/or by the digestibility of a nutrient in a feed (e.g. amino acid digestibility) and/or digestible energy or metabolizable energy in a feed and/or by nitrogen retention and/or by the animal's ability to avoid the negative effects of necrotic enteritis and/or by the immune response of the subject.

Preferably "animal performance" is determined by feed efficiency and/or weight gain of the animal and/or by the feed conversion ratio.

By "improved animal performance" it is meant that there is increased feed efficiency, and/or increased weight gain and/or reduced feed conversion ratio and/or improved digestibility of nutrients or energy in a feed and/or by improved nitrogen retention and/or by improved ability to avoid the negative effects of necrotic enteritis and/or by an improved immune response in the subject resulting from the use of feed additive composition of the present invention in feed in comparison to feed which does not comprise said feed additive composition.

Preferably, by "improved animal performance" it is meant that there is increased feed efficiency and/or increased weight gain and/or reduced feed conversion ratio. As used herein, the term "feed efficiency" refers to the amount of weight gain in an animal that occurs when the animal is fed ad-libitum or a specified amount of food during a period of time.

By "increased feed efficiency" it is meant that the use of a feed additive composition according the present invention in feed results in an increased weight gain per unit of feed intake compared with an animal fed without said feed additive composition being present.

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the feed does not comprise said feed additive composition.

Nutrient digestibility as used herein means the fraction of a nutrient that disappears from the gastro-intestinal tract or a specified segment of the gastro-intestinal tract, e.g. the small intestine. Nutrient digestibility may be measured as the difference between what is administered to the subject and what comes out in the faeces of the subject, or between what is administered to the subject and what remains in the digesta on a specified segment of the gastro intestinal tract, e.g. the ileum.

Nutrient digestibility as used herein may be measured by the difference between the intake of a nutrient and the excreted nutrient by means of the total collection of excreta during a period of time; or with the use of an inert marker that is not absorbed by the animal, and allows the researcher calculating the amount of nutrient that disappeared in the entire gastro-intestinal tract or a segment of the gastro-intestinal tract. Such an inert marker may be titanium dioxide, chromic oxide or acid insoluble ash. Digestibility may be expressed as a percentage of the nutrient in the feed, or as mass units of digestible nutrient per mass units of nutrient in the feed.

Nutrient digestibility as used herein encompasses starch digestibility, fat digestibility, protein digestibility, and amino acid digestibility.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

In some embodiments, the compositions described herein can improve the digestibility or utilization of dietary hemicellulose or fibre in a subject. In some embodiments, the subject is a pig.

Nitrogen retention as used herein means as subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

The term survival as used herein means the number of subject remaining alive. The term "improved survival" may be another way of saying "reduced mortality".

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

An "increased weight gain" refers to an animal having increased body weight on being fed feed comprising a feed additive composition compared with an animal being fed a feed without said feed additive composition being present.

The term "animal" as used herein includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to, dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feed", "feedstuff" and "fodder" are used interchangeably and can comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grains with Solubles (DDGS) (particularly corn based Distillers Dried Grains with Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; and/or e) minerals and vitamins.

Thermostable serine proteases described herein or a feed additive composition may be used as, or in the preparation of, a feed. The terms "feed additive composition" and "enzyme composition" are used interchangeably herein.

The feed may be in the form of a solution or as a solid or as a semi-solid depending on the use and/or the mode of application and/or the mode of administration.

When used as, or in the preparation of, a feed, such as functional feed, the enzyme or feed additive composition described herein may be used in conjunction with one or more of: a nutritionally acceptable carrier, a nutritionally acceptable diluent, a nutritionally acceptable excipient, a nutritionally acceptable adjuvant, a nutritionally active ingredient. For example, there be mentioned at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

In a preferred embodiment the enzyme or feed additive composition of the present invention is admixed with a feed component to form a feedstuff. The term "feed component" as used herein means all or part of the feedstuff. Part of the feedstuff may mean one constituent of the feedstuff or more than one constituent of the feedstuff, e.g. 2 or 3 or 4 or more. In one embodiment the term "feed component" encompasses a premix or premix constituents. Preferably, the feed may be a fodder, or a premix thereof, a compound feed, or a premix thereof. A feed additive composition may be admixed with a compound feed, a compound feed component or to a premix of a compound feed or to a fodder, a fodder component, or a premix of a fodder.

Any feedstuff described herein may comprise one or more feed materials selected from the group comprising a) cereals, such as small grains (e.g., wheat, barley, rye, oats, triticale and combinations thereof) and/or large grains such as maize or sorghum; b) by products from cereals, such as corn gluten meal, wet-cake (particularly corn based wet- cake), Distillers Dried Grains (DDG) (particularly corn based Distillers Dried Grains (cDDG)), Distillers Dried Grains with Solubles (DDGS) (particularly corn based Distillers Dried Grains with Solubles (cDDGS)), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

The term "fodder" as used herein means any food which is provided to an animal (rather than the animal having to forage for it themselves). Fodder encompasses plants that have been cut. Furthermore, fodder includes silage, compressed and pelleted feeds, oils and mixed rations, and also sprouted grains and legumes.

Fodder may be obtained from one or more of the plants selected from: corn (maize), alfalfa (Lucerne), barley, birdsfoot trefoil, brassicas, Chau moellier, kale, rapeseed (canola), rutabaga (swede), turnip, clover, alsike clover, red clover, subterranean clover, white clover, fescue, brome, millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, wheat, canola meal, rapeseed meal, lupin, soybeans, sorghum, oats, and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

In one embodiment the feedstuff comprises or consists of corn, DDGS (such as cDDGS), wheat, wheat bran or any combination thereof.

In one embodiment the feed component may be corn, DDGS (e.g. cDDGS), wheat, wheat bran or a combination thereof. In one embodiment the feedstuff comprises or consists of corn, DDGS (such as cDDGS) or a combination thereof.

A feedstuff described herein may contain at least 30%, at least 40%, at least 50% or at least 60% by weight corn and soybean meal or corn and full fat soy, or wheat meal or sunflower meal.

For example, a feedstuff may contain between about 5 to about 40% corn DDGS. For poultry, the feedstuff on average may contain between about 7 to 15% corn DDGS. For swine (pigs), the feedstuff may contain on average 5 to 40% corn DDGS. It may also contain corn as a single grain, in which case the feedstuff may comprise between about 35% to about 80% corn.

In feedstuffs comprising mixed grains, e.g. comprising corn and wheat for example, the feedstuff may comprise at least 10% corn.

In addition, or in the alternative, a feedstuff also may comprise at least one high fibre feed material and/or at least one by-product of the at least one high fibre feed material to provide a high fibre feedstuff. Examples of high fibre feed materials include: wheat, barley, rye, oats, by products from cereals, such as corn gluten meal, corn gluten feed, wet-cake, Distillers Dried Grains (DDG), Distillers Dried Grains with Solubles (DDGS), wheat bran, wheat middlings, wheat shorts, rice bran, rice hulls, oat hulls, palm kernel, and citrus pulp. Some protein sources may also be regarded as high fibre: protein obtained from sources such as sunflower, lupin, fava beans and cotton. In one aspect, the feedstuff as described herein comprises at least one high fibre material and/or at least one by-product of the at least one high fibre feed material selected from the group consisting of Distillers Dried Grains with Solubles (DDGS), particularly cDDGS, wet-cake, Distillers Dried Grains (DDG), particularly cDDG, wheat bran, and wheat for example. In one embodiment the feedstuff of the present invention comprises at least one high fibre material and/or at least one by-product of the at least one high fibre feed material selected from the group consisting of Distillers Dried Grains with Solubles (DDGS), particularly cDDGS, wheat bran, and wheat for example.

The feed may be one or more of the following: a compound feed and premix, including pellets, nuts or (cattle) cake; a crop or crop residue: corn, soybeans, sorghum, oats, barley copra, straw, chaff, sugar beet waste; fish meal; meat and bone meal; molasses; oil cake and press cake; oligosaccharides; conserved forage plants: silage; seaweed; seeds and grains, either whole or prepared by crushing, milling etc.; sprouted grains and legumes; yeast extract.

The term "feed" as used herein encompasses in some embodiments pet food. A pet food is plant or animal material intended for consumption by pets, such as dog food or cat food. Pet food, such as dog and cat food, may be either in a dry form, such as kibble for dogs, or wet canned form. Cat food may contain the amino acid taurine.

Animal feed can also include a fish food. A fish food normally contains macro nutrients, trace elements and vitamins necessary to keep captive fish in good health. Fish food may be in the form of a flake, pellet or tablet. Pelleted forms, some of which sink rapidly, are often used for larger fish or bottom feeding species. Some fish foods also contain additives, such as beta carotene or sex hormones, to artificially enhance the color of ornamental fish.

In still another aspect, animal feed encompasses bird food. Bird food includes food that is used both in birdfeeders and to feed pet birds. Typically, bird food comprises of a variety of seeds, but may also encompass suet (beef or mutton fat).

As used herein the term "contacted" refers to the indirect or direct application of a thermostable serine protease enzyme (or composition comprising the thermostable serine protease) to a product (e.g. the feed). Examples of application methods which may be used, include, but are not limited to, treating the product in a material comprising the feed additive composition, direct application by mixing the feed additive composition with the product, spraying the feed additive composition onto the product surface or dipping the product into a preparation of the feed additive composition. In one embodiment the feed additive composition of the present invention is preferably admixed with the product (e.g. feedstuff). Alternatively, the feed additive composition may be included in the emulsion or raw ingredients of a feedstuff. For some applications, it is important that the composition is made available on or to the surface of a product to be affected/treated. This allows the composition to impart a performance benefit.

In some aspects, the thermostable serine proteases described are used for the pretreatment of food or feed. For example, the feed having 10-300% moisture is mixed and incubated with the proteases at 5-80°C, preferably at 25-50°C, more preferably between 30-45 °C for 1 min to 72 hours under aerobic conditions or 1 day to 2 months under anaerobic conditions. The pre-treated material can be fed directly to the animals (so called liquid feeding). The pre-treated material can also be steam pelleted at elevated temperatures of 60-120°C. The proteases can be impregnated to feed or food material by a vacuum coater.

Thermostable serine proteases (or composition comprising the thermostable serine proteases) may be applied to intersperse, coat and/or impregnate a product (e.g. feedstuff or raw ingredients of a feedstuff) with a controlled amount of said enzyme.

Preferably, the feed additive composition will be thermally stable to heat treatment up to about 70 °C; up to about 85°C; or up to about 95°C. The heat treatment may be performed for up to about 1 minute; up to about 5 minutes; up to about 10 minutes; up to about 30 minutes; up to about 60 minutes. The term thermally stable means that at least about 75% of the enzyme components and/or DFM that were present/active in the additive before heating to the specified temperature are still present/active after it cools to room temperature. Preferably, at least about 80% of the protease component and/or DFM comprising one or more bacterial strains that were present and active in the additive before heating to the specified temperature are still present and active after it cools to room temperature. In a particularly preferred embodiment the feed additive composition is homogenized to produce a powder.

Alternatively, the feed additive composition is formulated to granules as described in WO2007/044968 (referred to as TPT granules) incorporated herein by reference.

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the at least one protease and/or DFM comprising one or more bacterial strains. Preferably, the salt used for the salt coating has a water activity greater than 0.25 or constant humidity greater than 60% at 20°C. Preferably, the salt coating comprises a $Na_2SO_4$.

The method of preparing a feed additive composition may also comprise the further step of pelleting the powder. The powder may be mixed with other components known in the art. The powder, or mixture comprising the powder, may be forced through a die and the resulting strands are cut into suitable pellets of variable length.

A method of preparing thermostable serine proteases (or composition comprising the thermostable serine proteases) may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes 2 minutes., 5 minutes, 10 minutes, 15 minutes, 30 minutes and 1 hour. It will be understood that the thermostable serine proteases (or composition comprising the thermostable serine proteases) described herein are suitable for addition to any appropriate feed material.

It will be understood by the skilled person that different animals require different feedstuffs, and even the same animal may require different feedstuffs, depending upon the purpose for which the animal is reared.

Optionally, the feedstuff may also contain additional minerals such as, for example, calcium and/or additional vitamins. In some embodiments, the feedstuff is a corn soybean meal mix.

Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting, in particular by suitable techniques that may include at least the use of steam.

The feedstuff may be a feedstuff for a monogastric animal, such as poultry (for example, broiler, layer, broiler breeders, turkey, duck, geese, water fowl), and swine (all age categories), a ruminant such as cattle (e.g. cows or bulls (including calves)), horses, sheep, a pet (for example dogs, cats) or fish (for example agastric fish, gastric fish, freshwater fish such as salmon, cod, trout and carp, e.g. koi carp, marine fish such as sea bass, and crustaceans such as shrimps, mussels and scallops). Preferably the feedstuff is for poultry.

The feed additive composition and/or the feedstuff comprising same may be used in any suitable form. The feed additive composition may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, the feed additive compositions may be mixed with feed or administered in the drinking water.

A feed additive composition, comprising admixing a protease as taught herein with a feed acceptable carrier, diluent or excipient, and (optionally) packaging.

The feedstuff and/or feed additive composition may be combined with at least one mineral and/or at least one vitamin. The compositions thus derived may be referred to herein as a premix.

In some embodiments, thermostable serine protease can be present in the feedstuff in the range of 1 ppb (parts per billion) to 10 % (w/w) based on pure enzyme protein. In some embodiments, the protease is present in the feedstuff is in the range of 1-100 ppm (parts per million). A preferred dose can be 1-20 g of thermostable serine protease per ton of feed product or feed composition or a final dose of 1 - 20 ppm thermostable serine protease in final product.

Preferably, the thermostable serine protease is present in the feedstuff should be at least about 200PU/kg or at least about 300 PU/kg feed or at least about 400 PU/kg feed or at least about 500 PU/kg feed or at least about 600 PU/kg feed, at least about 700 PU/kg feed, at least about 800 PU/kg feed, at least about 900 PU/kg feed or at least about 1000 PU/kg feed, or at least about 1500PU/kg feed, or at least about 2000PU/kg feed or at least about 2500 PU/kg feed, or at least about 3000 PU/kg feed, or at least about 3500 PU/kg feed, or at least about 4000 PU/kg feed, or at least about 4500 PU/kg feed, or at least about 5000 PU/kg feed.

In another aspect, thermostable serine protease can be present in the feedstuff at less than about 60,000PU/kg feed, or at less than about 70,000PU/kg feed, or at less than about 80,000PU/kg feed, or at less than about 90,000PU/kg feed, or at less than about 100,000PU/kg feed, or at less than about 200,000PU/kg feed, or at less than about 60000PU/kg feed, or at less than about 70000 PU/kg feed.

Ranges can include, but are not limited to, any combination of the lower and upper ranges discussed above.

It will be understood that one protease unit (PU) is the amount of enzyme that liberates 2.3 micrograms of phenolic compound (expressed as tyrosine equivalents) from a casein substrate per minute at pH 10.0 at 50°C. This may be referred to as the assay for determining 1 PU.

Formulations comprising any of thermostable serine protease and compositions described herein may be made in any suitable way to ensure that the formulation comprises active enzymes. Such formulations may be as a liquid, a dry powder or a granule. Preferably, the feed additive composition is in a liquid form suitable for spray-drying on a feed pellet.

Dry powder or granules may be prepared by means known to those skilled in the art, such as, high shear granulation, drum granulation, extrusion, spheronization, fluidized bed agglomeration, fluidized bed spray

Thermostable serine proteases and compositions described herein may be coated, for example encapsulated. In one embodiment, the coating protects the enzymes from heat and may be considered a thermo-protectant.

Feed additive composition described herein can be formulated to a dry powder or granules as described in WO2007/044968 (referred to as TPT granules) or WO1997/016076 or WO1992/012645 (each of which is incorporated herein by reference).

In one embodiment the feed additive composition may be formulated to a granule for feed compositions comprising: a core; an active agent; and at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

With regard to the granule at least one coating may comprise a moisture hydrating material that constitutes at least 55% w/w of the granule; and/or at least one coating may comprise two coatings. The two coatings may be a moisture hydrating coating and a moisture barrier coating. In some embodiments, the moisture hydrating coating may be between 25% and 60% w/w of the granule and the moisture barrier coating may be between 2% and 15% w/w of the granule. The moisture hydrating coating may be selected from inorganic salts, sucrose, starch, and maltodextrin and the moisture barrier coating may be selected from polymers, gums, whey and starch.

The granule may be produced using a feed pelleting process and the feed pretreatment process may be conducted between 70°C and 95°C for up to several minutes, such as between 85°C and 95°C.

The feed additive composition may be formulated to a granule for animal feed comprising: a core; an active agent, the active agent of the granule retaining at least 80% activity after storage and after a steam-heated pelleting process where the granule is an ingredient; a moisture barrier coating; and a moisture hydrating coating that is at least 25% w/w of the granule, the granule having a water activity of less than 0.5 prior to the steam-heated pelleting process.

The granule may have a moisture barrier coating selected from polymers and gums and the moisture hydrating material may be an inorganic salt. The moisture hydrating coating may be between 25% and 45% w/w of the granule and the moisture barrier coating may be between 2% and 10% w/w of the granule.

The granule may be produced using a steam-heated pelleting process which may be conducted between 85°C and 95°C for up to several minutes.

Alternatively, the composition is in a liquid formulation suitable for consumption preferably such liquid consumption contains one or more of the following: a buffer, salt, sorbitol and/or glycerol.

Also, the feed additive composition may be formulated by applying, e.g. spraying, the enzyme(s) onto a carrier substrate, such as ground wheat for example.

In one embodiment the feed additive composition may be formulated as a premix. By way of example only the premix may comprise one or more feed components, such as one or more minerals and/or one or more vitamins.

In one embodiment a direct fed microbial ("DFM") and/or thermostable serine proteases are formulated with at least one physiologically acceptable carrier selected from at least one of maltodextrin, limestone (calcium carbonate), cyclodextrin, wheat or a wheat component, sucrose, starch, $Na_2SO_4$, Talc, PVA, sorbitol, benzoate, sorbate, glycerol, sucrose, propylene glycol, 1,3-propane diol, glucose, parabens, sodium chloride, citrate, acetate, phosphate, calcium, metabisulfite, formate and mixtures thereof.

In another embodiment, there is disclosed a method for hydrolyzing starch-containing material comprising:

(a) contacting the starch-containing material with a liquid to form a mash; and

(b) hydrolyzing starch in the mash to form a liquefact by contacting the mash with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.

Furthermore, the enzyme cocktail may comprise at least one enzyme selected from the group consisting of alpha-amylase, amyloglucosidase, phytase, pullulanase, beta-glucanase, cellulase and xylanase.

Any of these enzymes can be used in an amount ranging from 0.5 to 500 micrograms/g feed or feedstock.

Alpha-amylases (alpha-1,4-glucan-4-glucanohydrolase, EC 3.2.1.1.) hydrolyze internal alpha-1,4-glucosidic linkages in starch, largely at random to produce smaller molecular weight dextrans. These polypeptides are used, *inter alia,* in starch processing and in alcohol production. Any alpha-amylases can be used, e.g., those described in U.S. Patent Nos. 8,927,250 and 7,354,752.

Amyloglucosidase catalyzes the hydrolysis of terminally 1,4-linked alpha-D-glucose residues successively from the non-reducing ends of maltooligo- and polysaccharides with release of beta-D-glucose. Any amyloglucosidase can be used.

Phytase refers to a protein or polypeptide which is capable of catalyzing the hydrolysis of phytate to (1) myo-inositol and/or (2) mono-, di-, tri-, tetra-, and/or penta-phosphatess thereof and (3) inorganic phosphate. For example, enzymes having catalytic activity as defined in Enzyme Commission EC number 3.1.3.8 or EC number 3.1.3.26. Any phytase can be used such as described in U.S. Patent Nos. 8,144,046, 8,673,609, and 8,053,221.

Pullulanase (EC 3.2.1.41) is a specific kind of glucanase, an amylolytic exoenzyme that degrades pullan (a polysaccharide polymer consisting of maltotriose units, also known as alpha-1,4-; alpha-1,6-glucan. Thus, it is an example of a debranching enzyme. Pullulanase is also known as pullulan-6-glucanohydrolase. Pullulanases are generally secreted by a *Bacillus* species. For example, *Bacillus deramificans* (US Patent No. 5,817,498; 1998), *Bacillus acidopullulyticus* (European Patent No. 0 063 909) and *Bacillus naganoensis* (US Patent No. 5,055,403). Enzymes having pullulanase activity used commercially are produced, for example, from *Bacillus* species (trade name OPITMAX® 1-100 from DuPont-Genencor and Promozyme® D2 fro Novozymes). Other examples of debranching enzymes include, but are not limited to, iso-amylase from *Sulfolobus solfataricus, Pseudomonas* sp. and thermostable pullulanase from *Fervidobacterium nodosum* (e.f., WO2010/76113). The iso-amylase from *Pseudomonas* sp. is available as purified enzyme from Megazyme International. Any pullulanase can be used.

Glucanases are enzymes that break down a glucan, a polysaccharide made several glucose sub-units. As they perform hydrolysis of the glucosidic bond, they are hydrolases.

Beta-glucanase enzymes (EC 3.2.1.4) digests fiber. It helps in the breakdown of plant walls (cellulose).

Cellulases are any of several enzymes produced by fungi, bacteria and protozoans that catalyze cellulolysis, the decomposition of cellulose and of some related polysaccharides. The name is also used for any naturally-occurring mixture or complex of various such enzymes, that act serially or synergistically to decompose cellulosic material. Any cellulases can be used.

Xylanase (EC 3.2.1.8) is the name given to a class of enzymes which degrade the linear polysaccharide beta-1,4-xylan into xylose, those breaking down hemicellulose, one of the major components of plant cell walls. Any xylanases can be used.

Starch or amylum is a polymeric carbohydrate consisting of a large number of glucose units joined by glycosidic bonds. Starch can be hydrolyzed into simpler carbohydrates by acids, various enzymes or a combination of the two.

In industry, starch is converted into sugars, for example by malting, and fermented to produce ethanol in the manufacture of beer, whiskey and biofuel.

Any suitable starch-containing material may be used. The starting material is generally selected based on the desired fermentation product. Examples of starch-containing materials, include but are not limited to, whole grains, corn, wheat, barley, rye, milo, sago, cassava, tapioca, sorghum, rice, peas, beans, or mixtures thereof or starches derived therefrom, or cereals. The starting material can be a dry solid, such as but not limiting to a dry solid of a feed or feedstock as described herein. Contemplated are also waxy and non-waxy types or corn and barley. Starch-containing materials used for ethanol production is corn or wheat. Starch is initially collected from plant grains using either a wet milling, a dry milling or a dry grind process.

A substrate comprising plant material is reduced or milled by methods known in the art. Plant material can be obtained from: wheat, corn, rye, sorghum (milo), rice, millet, barley, triticale, cassava (tapioca), potato, sweet potato, sugar beets, sugarcane, and legumes such as soybean and peas. Preferred plant material includes corn, barley, wheat, rice, milo and combinations thereof. Plant material can include hybrid varieties and genetically modified varieties (*e.g.* transgenic corn, barley or soybeans comprising heterologous genes).

Any part of the plant containing starch can be used to produce the liquefact, including but not limited to, plant parts such as leaves, stems, hulls, husks, tubers, cobs, grains and the like. Preferred whole grains include corn, wheat, rye, barley, sorghum and combinations thereof. In other embodiments, starch can be obtained from fractionated cereal grains including fiber, endosperm and/or germ components. Methods for fractionating plant material, such as corn and wheat, are known in the art. In some embodiments, plant material obtained from different sources can be mixed together (*e.g.* corn and milo or corn and barley). Methods of milling are well known in the art and reference is made TO THE ALCOHOL TEXTBOOK: A REFERENCE FOR THE BEVERAGE, FUEL AND INDUSTRIAL ALCOHOL INDUSTRIES 3rd ED. K.A. Jacques et al., Eds, (1999) Nottingham University Press. See, Chapters 2 and 4.

In some embodiments, the plant material, whether reduced by milling or other means, will be combined with a solution resulting in a slurry comprising starch substrate. In some embodiments, the slurry can include a side stream from starch processing such as backset. In some embodiments, the slurry will comprise 15 - 55% ds (e.g., 20 - 50%, 25 - 45%, 25 - 40%, and 20 - 35%). In some embodiments the slurry can comprise 10% to 60% of backset. The slurry comprising the reduced plant material can be subject to a liquefaction process wherein an alpha amylase can be added during the liquefaction step. This results in a liquefact. To produce the liquefact, a single or split dose of an alpha amylase can be added to the slurry. One skilled in the art can readily determine the effective dosage of alpha amylase to be used in the liquefaction processes.

The amount of alpha amylase used for liquefaction is an amount effective to cause liquefaction of a majority of the starch. In other embodiments, the amount is effective to enable liquefaction of greater than 40% of the starch, including 50%, 60%, 70%, 80%, 90%, and 100%. In some embodiments, the range will be 0.05 to 50 AAU/g ds (alpha-amylase units per gram of dry solids (e.g., feed or feedstock, such as corn), also 0.1 to 20 AAU/gDS and also 1.0 to 10 AAU/gDS. In further embodiments, the alpha amylase dosage will be in the range of 0.01 to 10.0 kg/metric ton (MT)ds; also 0.05 to 5.0 kg/MT ds; and also 0.1 to 4.0 kg/MT ds.

An alpha amylase can be added at a temperature of 0 to 30°C below the starch gelatinization temperature of the granular starch of the reduced plant material. This temperature can be 0 to 25°C, 0 to 20°C, 0 to 15°C and 0 to 10°C below the starch gelatinization temperature. This specific value will vary and depends on the type of grain comprising the slurry. For example, the starch gelatinization temperature of corn is generally higher than the starch gelatinization temperature of rye or wheat. In some embodiments, the temperature will be between 45 to 80°C, also between 50 to 75°C, also between 50 to 72°C and in some embodiments the temperature will be below 68°C; below 65°C, below 62°C, below 60°C and also below 55°C. In other embodiments the temperature will be above 40°C, above 45°C, above 50°C, and above 55°C. In some preferred embodiments, the temperature of the incubation will be between 58 to 72°C and also between 60 to 68°C.

In some embodiments, the slurry will be maintained at a pH range of about 3.0 to less than 6.5, also at a pH range of 4.0 to less than 6.2, also at a pH range of about 4.5 to less than 6.0 and preferably at a pH range of about 5.0 to 6.0 (e.g. about 5.4 to 5.8), and the milled grain in the slurry will be contacted with the enzyme composition for a period of time of 2 minutes to 8 hours (*e.g.,* 5 mins to 3 hrs; 15 mins to 2.5 hrs and 30 min to 2 hrs). In a further step the incubated substrate will be liquefied by exposing the incubated substrate to an increase in temperature such as 0 to 55°C above the starch gelatinization temperature. (e.g. to 65°C to 120°C, 70°C to 110°C, 70°C to 90°C) for a period of time of 2 minutes to 8 hours (*e.g.,* 2 minutes to 6 hrs, 5 minutes to 4 hours and preferably 1hr to 2 hrs) at a pH of about 4.0 to 6.5. In some embodiments, the temperature can be raised to a temperature to between about 85-90°C and a single dose of alpha amylase can be used. If the temperature is raised above 90-105°C, a second dose of alpha amylase can be added after the temperature returns to normal. In a further embodiment, the temperature can be raised to between about 105 and 140°C and a split dose of alpha amylase can be used with one part being added before raising the temperature and the other part added after the temperature has been brought down to at least below 105°C, including below 104, 103, 102, 101, 100, 99, 98, 97, 96, 95, 94, 93, 92, and 91°C, but preferably below 90°C. In some embodiments, the resulting liquefact is cooled before saccharification.

The liquefact obtained above can be contacted with a glucoamylase, an acid stable alpha amylase, and an acid fungal protease (such as FERMGEN™, DuPont or AP1 from CTE Global) in a single dose or a split dose as long as a desired ratio of enzymes is maintained. Thus, a split dose means that the total dose in desired ratio is added in more than one portion, including two portions or three portions. In one embodiment, one portion of the total dose is added at the beginning and a second portion is added at a specified time in the process. In one embodiment, at least a portion of the dose is added at the beginning of the saccharification (or SSF) to begin the saccharification process. In one embodiment, each enzyme in the enzyme composition can be added to the liquefact separately, but simultaneously or close enough in time such that the activity ratio is maintained. Alternatively, the enzyme blend composition comprising a glucoamylase, an acid stable alpha amylase, and an acid fungal protease can be added during one or both of the saccharification and fermentation. The ratio of the glucoamylase, an acid stable alpha amylase, and an acid fungal protease is preferably about 1:1.5:0.1 to about 1:8:1, and more preferably about 1:2:0.2 to 1:5: 0.6, as measured by GAU:SSU:SAPU.

The saccharification or SSF process typically comprises the addition of urea or ammonia used as a nitrogen source for the host organism (such as but not limiting to Yeast). In commercial ethanol plants, the addition of the nitrogen source (such as urea) during saccharification or SSF typically ranges between 200-1000 ppm, such as at least 200, 300, 400, 500, 600, 700, 800, 900 up to 1000 ppm urea. Another rich nitrogen source in an ethanol plant are the corn proteins present in the corn kernel. This nitrogen source is made available for the fermenting organism when it is hydrolysed into smaller fragments like small peptides and amino acids.

As described herein, it has been found surprisingly and unexpectedly that when a thermostable serine protease such as ME3 is present during the liquefying step of a method for producing fermentation products from starch containing materials, the need for adding a nitrogen source during the saccharification or SSF process is reduced by at least 30%, 40%, 50, 60%, 65%, 70%, 75%, 80%, 85%, 90%, 95%, 96%, 97%, 98%, or 99% or completely eliminated (100% reduction). This indicates that a thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 (such as but not limiting to ME-3 described herein) can hydrolyze corn proteins to such extend that it enables the fermenting organism to use corn proteins as a nitrogen source. Consequently, less urea or no urea at all can be added to the SSF. The strong reduction or elimination of a nitrogen source enables the plant to run at a lower cost.

Furthermore, this indicates that the presence of a protease other than a thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 (such as but not limiting to ME-3 described herein) in the fermentation process may not be needed.

The saccharification process can last for 12 to 120 hours. However, it is common to perform a saccharification for 30 minutes to 2 hours and then complete the saccharification during fermentation. Sometimes this is referred to as simultaneous saccharification and fermentation (SSF). Saccharification is commonly carried out at temperatures of 30 to 65°C and typically at pH of 3.0 to 5.0, including 4.0 to 5.0. The saccharification can result in the production of fermentable sugars.

In some embodiments the fermentable sugars are subjected to fermentation with fermenting microorganisms. The contacting step and the fermenting step can be performed simultaneously in the same reaction vessel or sequentially. In general, fermentation processes are described in The Alcohol Textbook 3rd ED, A Reference for the Beverage, Fuel and Industrial Alcohol Industries, Eds Jacques et al., (1999) Nottingham University Press, UK.

In some embodiments, the method further comprises using the fermentable sugars (dextrin e.g. glucose) as a fermentation feedstock in microbial fermentations under suitable fermentation conditions to obtain end-products, such as alcohol (e.g., ethanol), organic acids (e.g., succinic acid, lactic acid), sugar alcohols (e.g., glycerol), ascorbic acid intermediates (e.g., gluconate, DKG, KLG) amino acids (e.g., lysine), proteins (e.g., antibodies and fragment thereof).

The fermentable sugars can be fermented with a yeast at temperatures in the range of 15 to 40°C, 20 to 38°C, and also 25 to 35°C; at a pH range of pH 3.0 to 6.5; also pH 3.0 to 6.0; pH 3.0 to 5.5, pH 3.5 to 5.0 and also pH 3.5 to 4.5 for a period of time of 5 hrs to 120 hours, preferably 12 to 120 and more preferably from 24 to 90 hours to produce an alcohol product, preferably ethanol.

Yeast cells are generally supplied in amounts of $10^4$ to $10^{12}$, and preferably from $10^7$ to $10^{10}$ viable yeast count per ml of fermentation broth. The fermentation will include in addition to a fermenting microorganism (e.g. yeast) nutrients, optionally acid and additional enzymes. In some embodiments, in addition to the raw materials described above, fermentation media will contain supplements including but not limited to vitamins (e.g. biotin, folic acid, nicotinic acid, riboflavin), cofactors, and macro and micro-nutrients and salts (e.g. $(NH4)_2SO_4$; $K_2HPO_4$; NaCl; $MgSO_4$; $H_3BO_3$; $ZnCl_2$; and $CaCl_2$).

In some preferred embodiments, the milled plant material includes barley, milo, corn and combinations thereof, and the contacting and fermenting steps are conducted simultaneously at a pH range of 3.5 to 5.5, a temperature range of 30 - 45°C, and for a period of time of 48 to 90 hrs, wherein at least 50% of the starch is solubilized.

One end product of a fermentation process can be an alcohol product, e.g. ethanol. Other end products can be the fermentation co-products such as distillers dried grains (DDG) and distiller's dried grain plus solubles (DDGS), which can be used as an animal feed.

Use of an appropriate fermenting microorganisms as known in the art, the fermentation end products can include without limitation glycerol, 1,3-propanediol, gluconate, 2-keto-D-gluconate, 2,5-diketo-D-gluconate, 2-keto-L-gulonic acid, succinic acid, lactic acid, amino acids and derivatives thereof.

Examples of fermenting organisms are ethanologenic microorganisms or ethanol producing microorganisms such as ethanologenic bacteria which express alcohol dehydrogenase and pyruvate dehydrogenase and which can be obtained from *Zymomonas moblis* (See e.g. USP 5,000,000; USP 5,028,539, USP 5,424,202; USP 5,514,583 and USP 5,554,520). In additional embodiments, the ethanologenic microorganisms express xylose reductase and xylitol dehydrogenase, enzymes that convert xylose to xylulose. In further embodiments, xylose isomerase is used to convert xylose to xylulose. In particularly preferred embodiments, a microorganism capable of fermenting both pentoses and hexoses to ethanol are utilized. For example, in some embodiments the microorganism can be a natural or non-genetically engineered microorganism or in other embodiments the microorganism can be a recombinant microorganism.

The fermenting microorganisms include, but not limited to, bacterial strains from *Bacillus, Lactobacillus, E. coli, Erwinia, Pantoea* (e.g., P. citrea), *Pseudomonas* and *Klebsiella* (e.g. *K. oxytoca*). (See e.g. USP 5,028,539, USP 5,424,202 and WO 95/13362). Bacillus is a preferred fermenting microorganism. The fermenting microorganism used in the fermenting step will depend on the end product to be produced.

The ethanol-producing microorganism can be a fungal microorganism, such as Trichoderma, a yeast and specifically Saccharomyces such as strains of *S. cerevisiae* (USP 4,316,956). A variety of *S. cerevisiae* are commercially available and these include but are not limited to FALI (Fleischmann's Yeast), SUPERSTART (Alltech), FERMIOL (DSM Specialties), (Ethanol Red, Fermentis, France), (SYNERXIA® Thrive, Dupont), RED STAR (Lesaffre) and Angel alcohol yeast (Angel Yeast Company, China).

For example, when lactic acid is the desired end product, a *Lactobacillus* sp. (*L. casei*) can be used; when glycerol or 1,3-propanediol are the desired end-products, *E. coli* can be used; and when 2-keto-D-gluconate, 2,5-diketo-D-gluconate, and 2-keto-L-gulonic acid are the desired end products, *Pantoea citrea* can be used as the fermenting microorganism. The above enumerated list are only examples and one skilled in the art will be aware of a number of fermenting microorganisms that can be appropriately used to obtain a desired end product.

The end product produced can be separated and/or purified from the fermentation media. Methods for separation and purification are known, for example by subjecting the media to extraction, distillation and column chromatography. In some embodiments, the end product is identified directly by submitting the media to high-pressure liquid chromatography (HPLC) analysis.

The mash can be separated by, for example, centrifugation into the liquid phase and solids phase and end products such as alcohol and solids recovered. The alcohol can be recovered by means such as distillation and molecular sieve dehydration or ultrafiltration.

In some embodiments, use of an enzyme blend or composition according to the invention in a method of ethanol production will result in a yield of ethanol that is greater than 8%, 10%, 12%, 14%, 16%, 17%, 18%, 19%, 20%, 21%, and 22% (v/v).

Optionally following fermentation, alcohol (e.g. ethanol) can be extracted by for example distillation. Ethanol can be used for fuel, portable or industrial ethanol.

In another embodiment, there is disclosed a method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10;
(b) saccharifying the product of step (a);
(c) fermenting with a suitable organism; and
(d) optionally, recovering the product produced in step (c).

In still another aspect, this method can be conducted wherein steps (b) and (c) are performed sequentially or simultaneously.

Surprisingly, it has been discovered that when a thermostable serine protease such as ME-3 is present during the liquefying step of fermenting starch containing materials, the need for adding a nitrogen source during the saccharification or SSF process is reduced by at least at least 30%, 40%, 50, 60%, 65%, 70%, 75%, 80%, 85%, 90%, 95%, 96%, 97%, 98%, or 99% or completely eliminated (100% reduction). When steps (b) and (c) are performed simultaneously, addition of a nitrogen source (such as but not limiting to) urea or ammonia is either eliminated or reduced by at least 30%, 40%, 50, 60%, 65%, 70%, 75%, 80%, 85%, 90%, 95%, 96%, 97%, 98%, or 99% by using at least 1, 2, 3, 4, 5, 6, 7, 8, 9, 10, 11, 12, 13, 14, 15,16, 17, 18, 19, or 20 g thermostable serine protease/metric ton (MT) starch-containing material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 is used. When steps (b) and (c) are performed simultaneously, addition of a nitrogen source (such as but not limiting to) urea or ammonia is either eliminated or reduced by at least 30%, 40%, 50%, 65%, 70%, 75%, 80%, 85%, 90%, 95%, 96%, 97%, 98%, or 99% by using at least 3, 4, 5, or 6 g thermostable serine protease/MT dry solid material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs:3, 8, or 10 is used. It is believed that when step (b) and (c) are performed sequentially a similar reduction or elimination of a nitrogen source in step (b) can be observed.

When steps (b) and (c) are performed simultaneously, addition of a nitrogen source, such as but not limiting to, urea or ammonia is either eliminated or reduced by at least 30%, 40%, 50%, 65%, 70%, 75%, 80%, 85%, 90%, 95%, 96%, 97%, 98%, or 99% by using at least 1, 2, 3, 4, 5, 6, 7, 8, 9, 10, 11, 12, 13, 14, 15,16, 17, 18, 19, or 20 g thermostable serine protease/MT starch-containing material, wherein said thermostable serine protease has at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 is used and, furthermore, when the fermentation product is ethanol then no acid proteolytic enzyme is needed in step (c).

As described herein, the use of a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 in a method for producing fermentation products from starch-containing material can lead to additional benefits such as an increased level of crude protein, an increased level of acid hydrolyzed fat, a decreased level of glycerol (lower glycerol formation), a faster rate of ethanol production and an increased ethanol yield. An increased level of hydrolyzed fat is indicative of increased recovery of oil. Glycerol is a low value by-product of the fermentation that uses some of the available carbon. Reducing glycerol indicates more effective use of carbon and a reduced glycerol level indicates less stress on the yeast. Furthermore, any reduction in the production of glycerol enables carbon to flow to higher value products such as ethanol and can lead to reduced viscosity.

In one embodiment, there is disclosed a method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10;
(b) saccharifying the product of step (a);
(c) fermenting with a suitable organism; and
(d) optionally, recovering the product produced in step (c),

wherein acid hydrolyzed fat from materials obtained at the end of the fermentation is increased, when compared to materials obtained at the end of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 .

In one embodiment, there is disclosed a method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10;
(b) saccharifying the product of step (a);
(c) fermenting with a suitable organism; and
(d) optionally, recovering the product produced in step (c),

wherein the fermentation product (such as but not limiting to ethanol) is produced at a faster rate, when compared to materials obtained at the end of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 .

In one embodiment, there is disclosed a method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10;
(b) saccharifying the product of step (a);
(c) fermenting with a suitable organism; and
(d) optionally, recovering the product produced in step (c),

wherein the fermentation product (such as but not limiting to ethanol) yield is higher, when compared to the fermentation product yield of a control fermentation not comprising said a thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.

In one embodiment, there is disclosed a method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10;
(b) saccharifying the product of step (a);
(c) fermenting with a suitable organism; and
(d) optionally, recovering the product produced in step (c),

wherein glycerol levels from materials obtained at the end of the fermentation are decreased, when compared to materials obtained at the end of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 .

Fermentation products contemplated hereunder can include alcohols (e.g., ethanol, methanol, butanol; polyols such as glycerol, sorbitol and inositol); organic acids, such as, citric acid, acetic acid, itaconic acid, lactic acid, succinic acid, gluconic acid and the like; antibiotics, such as, pencillin and tetracycline; enzymes, vitamins, and hormones.

In another embodiment, the fermentation product can be ethanol, e.g., fuel ethanol; drinking ethanol such as potable neutral spirits; or industrial ethanol or products used in the consumable alcohol industry such as beer and wine, dairy industry (e.g., fermented dairy products), leather industry and tobacco industry. Beer types may comprise ales, stouts, porters, lagers, bitters, malt liquors, happoushu, high alcohol beer, low alcohol beer, low calorie beer or light beer. When ethanol is the fermentation product, it may be used as a fuel, which is typically blended with gasoline.

Starch to glucose processing generally consists of two steps and these steps include liquefaction of starch and saccharification of the liquefied starch. Further steps can include (a) purification and isomerization when the desired end product is a purified dextrose or fructose or (b) fermentation and distillation when the desired end product is, for example an alcohol (*e.g.,* ethanol).

An object of the starch liquefaction process is to convert a slurry of starch polymer granules into a solution of shorter chain length dextrins of low viscosity. This is an important step for convenient handling of industrial equipment used in starch conversion processes. Commonly, the starch is liquefied by use of high temperature and enzymatic bioconversion. For example, a common enzymatic liquefaction process involves adding a thermostable bacterial alpha amylase (*e.g*. SPEZYME® PRIME and SPEZYME® FRED, SPEZYME® ETHYL, SPEZYME® RSL, SPEZYME® CL, SPEZYME® HT-WB from Danisco U.S., Inc, Genencor Division or TERMAMYL SC, TERMAMYL SUPRA or TERMANYL 120L from Novozymes) to a slurry comprising a substrate including starch and adjusting the pH to between 5.5 to 6.5 and the temperature to greater than 90°C. SPEZYME® RSL , SPEZYME® CL and SPEZYME® HT-WB can be added at a relevant commercial dose ranging from at least 0.05 to 5 kG/metric ton (MT) starch containing material, such as at least about 0.05, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0 kG/metric ton (MT) or any dose in between. The starch is liquified and then subjected to saccharifying enzymes. Typically, saccharification (in the case of carbohydrate processing) takes place in the presence of glucoamylase enzymes such as glucoamylase from *Aspergillus niger* (*e.g.,* OPTIDEX L-400, DISTILLASE® SSF, DISTILLASE® CS, Spirizyme Fuel, Spirizyme Achieve) at a pH more acidic than the pH of the liquefaction step.

Following fermentation or SSF, the fermentation product may be separated from the fermentation medium. The mash (slurry) may be distilled to extract the desired fermentation product, e.g., ethanol. Alternatively, the desired fermentation produce may be extracted from the medium by micro or membrane filtration techniques. The fermentation product may also be recovered by stripping or other method well known in the art.

Production of fermentable sugars from lignocellulosic biomass requires that the biomass be treated to release sugars such as glucose, xylose, and arabinose from the polysaccharides of the biomass. Lignocellulosic biomass may be treated by any method known by one skilled in the art to produce fermentable sugars in a hydrolysate. The biomass may be pretreated using physical, thermal, or chemical treatments, or a combination thereof, and saccharified enzymatically. Thermochemical pretreatment methods include steam explosion or methods of swelling the biomass to release sugars (see for example WO2010113129; WO2010113130, both of which are incorporated by reference). Chemical saccharification may also be used. Physical treatments may be used for particle size reduction prior to further chemical treatment. Chemical treatments include base treatment such as with strong base (ammonia or NaOH), 5 or acid treatment (US8545633, WO2012103220, incorporated by reference).

For example, the biomass can be pretreated with ammonia (US 20080008783, US 7932063, US7781191, US 7998713, US7915017, all of which are incorporated by reference). These treatments release polymeric sugars from the biomass. The pretreatment can be a low ammonia pretreatment where biomass is contacted with an aqueous solution comprising ammonia to form a biomass aqueous ammonia mixture where the ammonia concentration is sufficient to maintain alkaline pH of the biomass-aqueous ammonia mixture but is less than about 12 weight percent relative to dry weight of biomass. In embodiments, the dry weight of biomass is at least about 15 weight percent solids relative to the weight of the biomass-aqueous ammonia mixture, as disclosed in the U.S. Patent No. 7,932,063, which is herein incorporated by reference.

Saccharification, which converts polymeric sugars to monomeric sugars, may be either by enzymatic or chemical treatments. The pretreated biomass is 20 contacted with a saccharification enzyme consortium under suitable conditions to produce a lignocellulosic biomass hydrolysate which comprises fermentable sugars. Prior to saccharification, the pretreated biomass can be brought to the desired moisture content and treated to alter the pH, composition or temperaturesuch that the enzymes of the saccharification enzyme consortium will be active.

The pH can be altered through the addition of acids in solid or liquid form. Alternatively, carbon dioxide (CO2), which can be recovered from fermentation, can be utilized to lower the pH. For example, CO2 can be collected from a fermenter and fed into the pretreatment product headspace in the flash tank or bubbled through the pretreated biomass if adequate liquid is present while 30 monitoring the pH, until the desired pH is achieved. The temperature is brought to a temperature that is compatible with saccharification enzyme activity, as noted below. Typically suitable conditions can include temperature from about 40 °C to about 50 °C and pH between from about 4.8 to about 5.8.

Enzymatic saccharification of cellulosic or lignocellulosic biomass typically makes use of an enzyme composition or blend to break down cellulose and/or hemicellulose and to produce a hydrolysate containing sugars 5 such as, for example, glucose, xylose, and arabinose. Saccharification enzymes are reviewed in Lynd, L. R., *et al.* (Microbiol. Mol. Biol. Rev., 66:506-577, 2002).

A saccharification enzyme blend can be used that includes one or more glycosidases. Glycosidases hydrolyze the ether linkages of di-, oligo-, and polysaccharides and are found in the enzyme classification EC 3.2.1.x (Enzyme Nomenclature 1992, Academic Press, San Diego, CA with Supplement 1 (1993), Supplement 2 (1994), Supplement 3 (1995, Supplement 4 (1997) and Supplement 5 [in Eur. J. Biochem., 223:1-5, 1994; Eur. J. Biochem., 232:1-6, 1995; Eur. J. Biochem., 237:1-5, 1996; Eur. J. Biochem., 250:1-6, 1997; and Eur. 15 J. Biochem., 264:610-650 1999, respectively]) of the general group "hydrolases" (EC 3.). Glycosidases useful in saccharification can be categorized by the biomass components they hydrolyze. Glycosidases useful in saccharification can include cellulose-hydrolyzing glycosidases (for example, cellulases, endoglucanases, exoglucanases, cellobiohydrolases, b-glucosidases), hemicellulose-hydrolyzing glycosidases (for example, xylanases, endoxylanases, exoxylanases, b-xylosidases, arabino-xylanases, mannases, galactases, pectinases, glucuronidases), and starch-hydrolyzing glycosidases (for example, amylases, a-amylases, b-amylases, glucoamylases, a-glucosidases, isoamylases). In addition, it can be useful to add other activities to the saccharification enzyme consortium such as peptidases (EC 3.4.x.y), lipases (EC3.1.1.x and 3.1.4.x), ligninases (EC 1.11.1.x), or feruloyl esterases (EC 3.1.1.73) to promote the release of polysaccharides from other components of the biomass. It is known in the art that microorganisms that produce polysaccharide hydrolyzing enzymes often exhibit an activity, such as a capacity to degrade cellulose, which is catalyzed by several enzymes or a group of enzymes having different substrate specificities. Thus, a "cellulase" from a microorganism can comprise a group of enzymes, one or more or all of which can contribute to the cellulose-degrading activity.

Commercial or non-commercial enzyme preparations, such as cellulase, can comprise numerous enzymes depending on the purification scheme utilized to obtain the enzyme. Many glycosyl hydrolase enzymes and compositions thereof that are useful for saccharification are disclosed in WO 2011/038019 or WO 2012/125937, incorporated herein by reference. Additional enzymes for saccharification include, for example, glycosyl hydrolases that hydrolyze the glycosidic bond between two or more carbohydrates, or between a carbohydrate and a noncarbohydrate moiety.

Suitable enzymes may include enzymes from Glycoside hydrolase families, such as the families GH3, GH5, GH 6, GH 7, GH10, GH11, GH39, GH43, GH51, and GH 61. GHs are a group of enzymes that hydrolyze the glycosidic bond between two or more carbohydrates, or between a carbohydrate and a non-carbohydrate moiety. Families of GHs have been classified based on sequence similarity and are available in the Carbohydrate-Active enzyme (CAZy) database (Cantarel et al. (2009) Nucleic Acids Res. 37 (Database issue):D233-238).

These enzymes are able to act on a number of substrates and are effective in the saccharification process. Glycoside hydrolase family 3 ("GH3") enzymes have a number of known activities: b-glucosidase (EC:3.2.1.21); b-xylosidase (EC:3.2.1.37); N- acetyl b-glucosaminidase (EC:3.2.1.52); glucan b-1,3-glucosidase (EC:3.2.1.58); cellodextrinase (EC:3.2.1.74); exo-1,3-1,4-glucanase (EC:3.2.1); and b-galactosidase (EC 3.2.1.23). Glycoside hydrolase family 39 ("GH39") enzymes have a-L-iduronidase (EC:3.2.1.76) or b-xylosidase (EC:3.2.1.37) activity.

Glycoside hydrolase family 43 ("GH43") enzymes have the following activities: L-25 a-arabinofuranosidase (EC 3.2.1.55); b-xylosidase (EC 3.2.1.37); endoarabinanase (EC 3.2.1.99); and galactan 1,3-b-galactosidase (EC 3.2.1.145). Glycoside hydrolase family 51 ("GH51") enzymes have L-a-arabinofuranosidase (EC 3.2.1.55) or endoglucanase (EC 3.2.1.4) activity. Glycoside hydrolase family 10 ("GH10") are described, for example, in Schmidt et 30 al., 1999, Biochemistry 38:2403-2412 and Lo Leggio et al., 2001, FEBS Lett 509: 303-308) and the Glycoside hydrolase family 11 ("GH11") are more fully described in Hakouvainen et al., 1996, Biochemistry 35:9617-24. Suitable enzymes may include glycoside hydrolase family 61 ("GH61") enzymes which have been reclassified into Auxiliary Activity Family 9 (AA9; cazypedia.org) and may be referred to as GH61 enzymes herein. As described in U.S. Patent Application Publication Number US2014/0106408 suitable enzymes may include EG4 (such as from *Trichoderma reesei* and variants thereof as described, for example, in WO201517256A1, WO201517255A1, WO201517254A1, incorporated by reference), Fv3A, Fv51A and/or Fv43D (such as from *Fusarium verticilloides* as described, for example in US2014/016408, incorporated by reference). Suitable enzymes may include, for example, enzymes disclosed in PCT Application Publication Nos. WO03/027306, WO200352118_A2, WO200352054_A2, WO200352057 _A2, WO200352055_A2, WO200352056 _A2, WO200416760_A2, WO9210581_A1, WO200448592_A2, WO200443980_A2, WO200528636_A2, WO200501065 _A2, WO2005/001036, WO2005/093050, WO200593073_A1, WO200674005 _A2, WO2009/149202, WO2011/038019, WO2010/141779, WO2011/063308, WO2012/125951, WO2012/125925, WO2012125937, WO/2011/153276, WO2014/093275, WO2014/070837, WO2014/070841, WO2014/070844, WO2014/093281, WO2014/093282, WO2014/093287, WO2014/093294, WO2015/084596, orWO2016/069541, incorporated herein by reference.

Saccharification enzymes can be obtained commercially. Such enzymes include, for example, SPEZYME® CP cellulase, Multifect® xylanase, Accelerase® 1500, Accellerase® DUET, Accellerase® TrioTM, ® CS (from DuPont), DISTILLASE® SSF, and Novozyme-188 (Novozymes). DISTILLASE® SSF can be dosed at a commercial relevant dose of 0.06 to 0.08% weight enzyme / weight as is corn. The actual dose required will depend upon the conditions of the fermentation: time, initial pH and the level of solids. DISTILLASE® CS enzyme can be added at a commercial relevant dose of 0.055 to 0.075 % w/w (starch, dry solid basis). This corresponds to a level of 0.375 to 0.50 kg DISTILLASE® CS / MT grain 'as is'. Additional enzymes can be added such as but not limiting to trehalase.

In addition, saccharification enzymes can be provided as crude preparations of a cell extract or a whole cell broth. The enzymes can be produced using recombinant microorganisms that have been engineered to express one or more saccharifying enzymes. For example, an H3A protein preparation that may be used for saccharification of pretreated lignocellulosic biomass is a crude preparation of enzymes produced by a genetically engineered strain of *Trichoderma reesei,* which includes a combination of cellulases and hemicellulases and is described in WO 2011/038019, which is incorporated herein by reference.

It will be appreciated that in embodiments whereby the pretreated lignocellulosic biomass material is contacted with an acetamidase enzyme, the enzyme may be introduced with or separate from the saccharification enzyme consortium.

Chemical saccharification treatments can be used and are known to one skilled in the art, such as treatment with mineral acids including HCl and H2SO4 (US5580389, WO2011002660, incorporated by reference). 10 Sugars such as glucose, xylose and arabinose are released by saccharification of lignocellulosic biomass and these monomeric sugars provide a carbohydrate source for a biocatalyst used in a fermentation process. The sugars are present in a biomass hydrolysate that is used as fermentation medium. The fermentation medium can be composed solely of hydrolysate, or can include components additional to the hydrolysate such as sorbitol or mannitol at a final concentration of about 5 mM as described in US 7,629,156, which is incorporated herein by reference. The biomass hydrolysate may make up at least about 50% of the fermentation medium. In embodiments, about 10% of the final volume of fermentation broth is seed inoculum containing the fermentation microorganism.

The fermentation medium comprising lignocellulosic biomass hydrolysate contacted with a fermentation microorganism is fermented in a fermentation vessel, which is any vessel that holds the fermentation medium and at least one biocatalyst, and has valves, vents, and/or ports used in managing the fermentation process. Any microorganism that produces a target product utilizing glucose and preferably also xylose, either naturally or through genetic engineering, may be used for fermentation of the fermentable sugars in the lignocellulosic biomass hydrolysate. Target products that may be produced by fermentation include, for example, acids, alcohols, alkanes, alkenes, aromatics, aldehydes, ketones, biopolymers, proteins, peptides, amino acids, vitamins, antibiotics, and pharmaceuticals. Alcohols include, but are not limited to methanol, ethanol, propanol, isopropanol, butanol, ethylene glycol, propanediol, butanediol, glycerol, erythritol, xylitol, mannitol, and sorbitol. Acids may include acetic acid, formic acid, lactic acid, propionic acid, 3-hydroxypropionic acid, butyric acid, gluconic acid, itaconic acid, citric acid, succinic acid, 3- hydroxyproprionic acid, fumaric acid, maleic acid, and levulinic 5 acid. Amino acids may include glutamic acid, aspartic acid, methionine, lysine, glycine, arginine, threonine, phenylalanine and tyrosine. Additional target products include methane, ethylene, acetone and industrial enzymes.

The fermentation of sugars in lignocellulosic biomass hydrolysate to target products can be carried out by one or more appropriate microorganisms, that are able to grow in medium containing biomass hydrolysate, in single or multistep fermentations. Suitable microorganisms may be selected from bacteria, filamentous fungi and yeast. The suitable microorganisms can be wild type microorganisms or recombinant microorganisms, and can include, for example, 15 organisms belonging to the genera of *Escherichia, Zymomonas, Saccharomyces, Candida, Pichia, Streptomyces, Bacillus, Lactobacillus,* and *Clostridiuma.* Typical examples of suitable microorganisms include recombinant *Escherichia coli, Zymomonas mobilis, Bacillus stearothermophilus, Saccharomyces cerevisiae, Clostridia thermocellum, Thermoanaerobacterium saccharolyticum,* and *Pichia stipitis.* To grow well and have high product production in a lignocellulosic biomass hydrolysate fermentation broth, a microorganism can be selected or engineered to have higher tolerance to inhibitors present in biomass hydrolysate such as acetate. For example, the biocatalyst may produce ethanol as a target product, such as production of ethanol by *Zymomonas mobilis,* or production of ethanol by *Saccharomyces cerevisiae.* Suitable engineered *Zymomonas mobilis* and *Saccharomyces cerevisiae* strains are known in the art, for example as described in US 8,247,208 and US 8,669,076, both incorporated herein by reference.

Fermentation is carried out with conditions appropriate for the particular microorganism used. Adjustments can be made for conditions such as pH, temperature, oxygen content, and mixing. Conditions for fermentation of yeast and bacterial biocatalysts are well known in the art. In addition, saccharification and fermentation may occur at the same time in the same vessel, called simultaneous saccharification and fermentation (SSF). In addition, partial saccharification may occur prior to a periodof concurrent saccharification and fermentation in a process called HSF (hybrid saccharification and fermentation).

For large scale fermentations, typically a smaller culture (seed culture) of the fermentation microorganism is first grown. The seed culture is added to the fermentation medium as an inoculum typically in the range from about 2% to about 20% of the final volume. Typically fermentation by the fermentation microorganism produces a fermentation broth containing the target product made by the organism. For example, in an ethanol process the fermentation broth may be a beer containing from about 6% to about 10% ethanol. In addition to target product, the fermentation broth contains water, solutes, solids from the hydrolysate medium and from biocatalyst metabolism of sugars in the hydrolysate medium, as well as the biocatalyst itself. The target product may be isolated from the fermentation broth producing a depleted broth, which can be called whole stillage. For example, when ethanol is the product, the broth is distilled, typically using a beer column, to generate an ethanol product stream and a whole stillage. Distillation can be using any conditions known to one skilled in the art including at atmospheric or reduced pressure. The distilled ethanol is further passed through a rectification column and molecular sieve to recover an ethanol product.

The target product may alternatively be removed in a later step such as from a solid or liquid fraction after separation of fermentation broth.

It is possible to add an alpha-amylase during liquefaction along with the thermostable serine protease described herein. Optionally, a carbohydrate-source generating enzyme such as a glucoamylases, and/or optionally a pullulanase. Any alpha-amylase may be used.

Alpha-amylases and pullulanases are described above.

Biomass for second generation bioethanol production needs pretreatment before subjecting to enzyme hydrolysis to produce fermentable sugar. Biomass is usually pretreated at temperatures higher 100°C either under acidic or alkaline conditions (DUNSON et al., TREATMENT OF BIOMASS TO OBTAIN FERMENTABLE SUGARS. WO2006110901 (A2) - 2006-10-19).

Thermostable and thermoactive proteases like the thermostable serine proteases described herein may be used in such processes to hydrolyze any protein present in the biomass, to provide the ethanologen with amino acids and peptide nutrients, and to reduce the viscosity of the biomass slurry. Biomass pretreated under alkaline conditions, such as with diluted ammonium (WO2006110901), is especially suitable for hydrolysis with said thermostable serine proteases due to their high pH.

Thus, in still another aspect, there is disclosed a method for hydrolyzing proteins in lignocellulosic biomass comprising

(a) contacting the biomass with a liquid; and
(b) hydrolyzing the proteins in the biomass by contacting the biomass with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.

In another embodiment, there is disclosed a method for reducing viscosity of a liquefied starch-containing material which comprises contacting a slurry of starch-containing material with a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10.

In another embodiment, there is disclosed a method for extracting oil from an oilseed crop comprising:

(a) contacting an oil-containing oilseed fraction with an aqueous extractant to form an extract oilseed fraction; and
(b) separating the extracted oilseed fraction into an aqueous phase, an oil-in-water emulsion, and an insoluble phase;
(c) contacting the oil-in-water emulsion with at least a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10, either alone or in combination with a phospholipase, under conditions permitting enzyme activity for a time sufficient to destabilize the emulsion; and
(d) separating the destabilizing emulsion of step (c) into an aqueous phase, an oil phase, and an insoluble phase to obtain oil from the oilseed.

Furthermore, the oilseed fraction can be from soybean, corn seed, rape seed, palm kernel, sunflower seed, safflower seed, coconut, peanut, cotton seed, sesame seed, flax seed, poppy seed, almond, hazelnut, walnut, evening primrose seed, grape seed, hemp seed, black currant seed, red raspberry seed, carrot seed, cumin seed, blueberry seed, cranberry seed, parsley seed, onion seed, pumpkin seed, apricot kernel, mustard seed, linseed, castor seed or jatropha.

The oilseed fraction can comprise a protein fraction that is useful as a food, food ingredient, a food additive or food supplement.

Clearly, plant-derived oil can be prepared by using the process described herein.

In addition, a feed, feedstuff, feed additive composition, premix, food or grain product can comprise the oil described herein.

Plant-derived lipids, particularly oils, are a major source of lipids for food processing, and for industrial feedstock. More recently they are of interest and use as alternatives to petrochemicals for fuels. Plant lipids can be derived from one or more parts of a plant, shrub, or tree. In various plants, the root, stem, bark, leaves, flowers, seeds, fruits, or other parts may serve as a source of oil. Such lipids can be extracted mechanically, e.g. through the application of external pressure, or chemically, e.g. through organic or aqueous solvent extraction processes, or combination processes.

Oils obtained from oilseed plants are known for their uses in food, and food products, as well as for soaps, detergents, lotions, lubricants, insecticides, paints, coatings, inks, and other industrial or consumer products. Although the vast majority of oilseeds oils are extracted with an organic solvent extraction process, some are now extracted through aqueous extraction.

Aqueous processes for extracting oils from oilseeds have been developed in recent years. See Freitas et al., Fett/Lipid 99: 333-337, 1997; and Caetano et al., La Rivista Italiana Delle Sostanze Grasse 79: 165-169, 2002. Both Freitas *et al.* and Caetano *et al.* provide a combination of extrusion and protease treatment to extract oils in aqueous processes. Aqueous process steps are inherently safer than organic solvent extraction steps and accordingly, the initial start-up investment in capital equipment is substantially less for aqueous extraction processes.

As used herein an "emulsion" comprises an at least transiently stable system comprising a physical mixture of at least two materials, not completely miscible with, or soluble in, each other. Preferred emulsions, as used herein, do not readily separate when they are allowed to stand undisturbed, and can remain mixed for considerable lengths of time. They preferably will remain stable for extended periods of time such as greater than about 1, 2, 4, 8 12, or 24 hours, or even longer.

While emulsion systems can comprise solids, liquids, and gases, preferably, the emulsions for use herein comprise at least a lipid phase and an aqueous phase, both of which are in the liquid state. One phase in an emulsion is continuous, while the other phase is dispersed in and thus, discontinuous with, the other. For example, an "oil-in-water" emulsion has a discontinuous phase of oil dispersed in a continuous aqueous phase, whereas a "water-in-oil" emulsion has a discontinuous aqueous phase dispersed in a continuous lipid or oil phase. In a practical sense, the discontinuous phase consists of small droplets dispersed in and contained within the other phase. The discontinuous phase is thus also sometimes called the "internal" phase, and by analogy, the continuous phase is sometimes called the "external" phase. Under certain circumstances an emulsion can invert, i.e. the continuous and discontinuous phases may change roles, for example, an oil-in-water emulsion becomes a water-in-oil emulsion or vice versa.

The terms "oil-in-water" and "water-in-oil" as used herein are merely descriptive to help understand which of the phases is continuous and which is dispersed in a given emulsion system; these terms are not intended to limit the emulsion literally to oil and water. The "water" or aqueous phase may contain one or more solutes, such as salts, and any number of other soluble, or partially soluble compounds. Similarly, the "oil" or lipidic phase may contain a wide variety of lipids or lipid-soluble compounds.

As used herein "oil" denotes any of a group of fats (or lipids) that remain liquid at room temperature. "Plant oil" as used herein denotes any such oils that are obtained from any tissue of a plant. Plant oils are also referred to herein alternatively as "plant-derived oils". In certain embodiments plant oils are edible, while in other embodiments they are not necessarily edible. Some oils may be appropriately and safely used for external application to an animal such as a human, while other oils may be completely inedible, and also not safe for external use on an animal. Such oils may nonetheless be valuable for use industrial process, as lubricants, cleaning or polishing products, or simply as feedstock for making other compositions or products requiring a lipid as a raw material. For example, some lipids are useful as fuels or fuel supplements. There is presently significant interest in biological or renewable sources of fuels, such as combustible fuels, for example lipids for use in biodiesel.

"Oilseed" as used herein refers to any oil-containing seed, nut, kernel, or the like produced by a plant. All such plants, as well as their seeds, nuts, or kernels are contemplated for use herein. For example, the National Sustainable Agriculture Information Service lists the following as sources of oil for food, specialty, or industrial uses: almonds, apricot kernels, avocado, beech nut, billberry, black currant, borage, brazil nut, calendula, caraway seed, cashew nut, castor seed, citrus seed, clove, cocoa, coffee, copra (dried coconut), coriander, corn seed, cotton seed, elderberry, evening primrose, grape seed, groundnut, hazelnut, hemp seed, jojoba, linseed, macadamia nut, mace, melon seed, mustard seed, neem seed, niger seed, nutmeg, palm kernel, passion fruit, pecan, pistachio, poppy seed, pumpkin seed, rape seed, raspberry seed, red pepper, rose hip, rubber seed, safflower seed, sea buckthorn, sesame seed, soybean, spurge, stinging nettle, sunflower seed, tropho plant, tomato seed, or walnut. Also useful herein are various oilseed and related plants whose oil content is of interest for use as fuel, such as "eco-fuel", biodiesel or the like. Such plants include but are not limited to jatropha (e.g. *Jatropha curcas, J. mahafalensis,* and cultivars thereof); *Elaeis guineensis (e.g. Oil palm), Aleurites fordii* (tung oil tree or wood oil tree), *Ricinus communis* (castor bean tree), *Copaifera langsdorfii* (diesel tree), and *Pongammia pinnata* (Honge oil tree, or Pongam tree, and cultivars thereof).

An "oil-containing fraction" or "lipid-containing fraction" as used herein refers to the oilseed or some portion or part thereof, however obtained. In preferred embodiments, the oil-containing fraction will comprise all, or at least a majority of, the oil (or fat or lipid content) of the oilseed. In other embodiments, a prior processing step may remove at least some, or even a majority of the oil from the oilseed prior to obtaining the fraction. Thus, for example, in certain embodiments using soybean as source of oilseed, the "fraction" comprises soy flour or soy flakes. Preferably, the flour or flakes obtained are "full-fat" as that term is understood in the art. However, that does not preclude the use of the processes provided herein with, for example, partially defatted fractions, such as soy flour or soy flakes. Economic factors may provide commercial motivation to use fractions containing at least a majority of the fat, however there are no known technical barriers to using the disclosed processes fractions comprising less than a majority of the fat of the whole oilseed.

For certain embodiments, the oil-containing fraction is from a major source of food or industrial oil. Presently, soybean, corn seed, cotton seed, and rape seed, as well as sunflower seed, safflower, flax seed, and peanut are preferred as sources of food oil.

As used herein, an "aqueous solvent" comprises at least water. Aqueous solvents typically comprise other components such as salts, buffering compounds, small molecules, and more. Any number and concentration of additional components may be present provided the aqueous solvent is substantially a homogeneous solution or suspension in water. This aqueous solvent, with the additional components as a substantially homogenous solution or suspension, is sometimes referred to herein as an "aqueous extractant" for convenience and to distinguish it from the solvent *per se,* e.g. water. The two terms are synonymous as defined herein. Preferably all components present are in true solution in the aqueous extractant or aqueous solvent. An aqueous solvent is distinguished from an organic solvent in that rather than water as the solvent, the term "organic solvent" refers to most other solvents that are organic compounds and contain carbon atoms. Organic solvents are more volatile and potentially explosive than aqueous solvents. Typically, organic solvent extraction of oils refers to any process used to remove an oil from oilseeds through direct contact with an organic solvent such as n-hexane or other hexanes.

As noted above, methods or processes are provided for destabilizing an emulsion comprising an oil phase and an aqueous phase. The emulsion is derived from, or produced in, an aqueous solvent extraction of a lipid from an oilseed. The emulsion is contacted with at least a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10, either alone or in combination with a phospholipase, under conditions allowing enzyme activity, for a time sufficient to destabilize the emulsion.

In another aspect, the thermostable serine protease may be used in combinations with at least a phospholipase. While such enzyme activities from any source such as animal, plant, or microbial, are contemplated for use herein, the enzyme activity preferably comprises a phospholipase activity from a mammalian pancreas, *Streptomyces violaceoruber, Aspergillus oryzae,* or *Aspergillus niger.* In one embodiment, the phospholipase activity is from porcine pancreas.

The water phase is a continuous phase and the oil phase can be a discontinuous phase, i.e. the emulsion is an oil-in-water emulsion.

Processes for separating oil from aqueous phases are known in the art. Frequently, they involve gravity or more preferably forces in excess of gravity, for example forces applied through physical means such as centrifugation. In one embodiment, the emulsions are centrifuged in a batch-wise process. In various embodiments, the conditions for separating may include adjusting the emulsion after enzyme treatment to a preferred temperature, for example by heating. In another embodiment, continuous centrifugation is preferred for separating the oil from the aqueous phase. Continuous processes may be preferred to larger-scale operations and are well-suited to handling material by the vessel-full, for example, from silos, tanks, vats, or the like, or even in connected series of such vessels.

This process may improve the yield of oil from the emulsion. The skilled artisan will appreciate how to monitor yield on a variety of bases. Generally, the yield comparisons are made by comparing, for example, the yield of oil (for example on a % of theoretical maximum yield based on the oil content of the untreated emulsion) using the processes disclosed herein to the yield of an aqueous extraction that does not use the step of contacting the emulsion with the enzyme activity. Other bases of yield may be used, for example, an improvement of oil recovery using the processes disclosed herein versus a "control" process.

The emulsion can be contacted with at least one enzyme activity comprising at least a thermostable serine protease activity as described herein either alone or in combination with a phospholipase. While such enzyme activities from any source such as animal, plant, or microbial, are contemplated for use herein, the enzyme activity preferably comprises a phospholipase activity from a mammalian pancreas, *Streptomyces violaceoruber, Aspergillus oryzae,* or *Aspergillus niger.* In one embodiment, the phospholipase activity is from porcine pancreas.

Various phospholipase activities have proven useful for destabilizing the emulsion in accordance with the processes disclosed herein. Phospholipases for purposes herein include, but are not limited to, phospholipases A (including A1 and A2), B (also sometimes referred to as lysophospholipase), C, and D. Phospholipases are a class of enzymes that hydrolyze phospholipids, such as phosphatidylcholine or phosphatidylethanolamine. Within the phospholipase class of enzymes are five major subclasses, A1, A2, B, C, and D phospholipases.

A1 phospholipases (E.C. 3.1.1.32) preferentially hydrolyze the sn1 ester bonds of phospholipids, such as phosphatidylcholine or phosphatidylethanolamine, to yield 1-lysophospholipids plus carboxylic acids. Typically, A1 phospholipases require calcium as a cofactor. A1 phospholipases generally exhibit broader specificity than A2 phospholipases.

A2 phospholipases (E.C. 3.1.1.4) preferentially hydrolyze the sn2 ester bonds of phospholipids, such as phosphatidylcholine or phosphatidylethanolamine, to yield 2-lysophospholipids plus carboxylic acids. In addition to phospholipids, A2 phospholipases show some specificity for hydrolysis of choline derivatives and phosphatides. Typically, A2 phospholipases require calcium as a cofactor.

B phospholipases (E.C. 3.1.1.5) are also known as lysophospholipases. They preferentially hydrolyze the sn1 ester bonds of 2-lysophospholipids to yield glycerophosphatides plus carboxylic acids. B phospholipases will also hydrolyze the sn2 ester bonds of 1-lysophospholipids.

C phospholipases (E.C. 3.1.4.3) preferentially hydrolyze the phosphate bonds of phospholipids, such as phosphatidylcholine or phosphatidylethanolamine, to yield the corresponding diacylglycerols and choline phosphates. In addition to hydrolysis of phospholipids, C phospholipases will also act on lysophospholipids.

D phospholipases (E.C. 3.1.4.4) preferentially hydrolyze the phosphate bond of phospholipids such as phosphatidylcholine or phosphatidylethanolamine to yield the corresponding phosphatidic acids and choline. In addition to hydrolysis of phospholipids, D phospholipases will also act on lysophospholipids.

Phospholipases can be used individually or in combination or mixtures of one or more activities of the same or different E.C. classifications, and from the same or different sources. Crude or partially purified enzyme preparations containing one or more phospholipase activities are suitable for use in some embodiments herein. Commercial sources of phospholipases are also suitable for use herein. For example, Genencor- A Danisco Division (Rochester, NY) offers LysoMax® and G-ZYME® G999 phospholipases, from bacterial and fungal sources, respectively. Phospholipase C is available commercially, for example, from Sigma (St. Louis, MO).

C phospholipases (E.C. 3.1.4.3) preferentially hydrolyze the phosphate bonds of phospholipids, such as phosphatidylcholine or phosphatidylethanolamine, to yield the corresponding diacylglycerols and choline phosphates. In addition to hydrolysis of phospholipids, C phospholipases will also act on lysophospholipids.

D phospholipases (E.C. 3.1.4.4) preferentially hydrolyze the phosphate bond of phospholipids such as phosphatidylcholine or phosphatidylethanolamine to yield the corresponding phosphatidic acids and choline. In addition to hydrolysis of phospholipids, D phospholipases will also act on lysophospholipids.

The oil-containing oilseed fraction comprises cells and the process further comprises the step of disrupting the cells prior to contacting the oilseed fraction with an aqueous extractant. Presently preferred methods of disrupting cells include application of a mechanical force. Extrusion is a convenient method of disrupting cells in oil-containing seeds. Such use of extruders is known in the art, for example single-screw or twin-screw extruders are useful therefore. The skilled artisan can readily determine parameters for disrupting cells in oilseeds by measuring the oil recovery or yield, while varying the extrusion parameters such as speed, transit time, pressure, temperature, pH, and the like. Other methods of disrupting cells include chemical and or enzymatic treatment, in addition to additional mechanical methods such as pressing, rolling, abrading, sonication, and others.

The oil-in-water emulsion can be contacted with enzyme activity comprising at least a thermostable serine protease either alone or in combination with phospholipase.

This process can include a centrifugation step for either or both of the separating steps. The skilled artisan will appreciate the use of centrifugation for separating phases of differing densities, such as the oil and aqueous phases present in the emulsion of the process provided.

It is possible to adjust the pH is to between about 3.5 to about 5 prior to the separating step. Such adjustment may help to destabilize the emulsion in a variety of ways. Without limiting the process to any one theory of operation, a pH adjustment may help to precipitate one or more proteins, said proteins perhaps involved in stabilizing the emulsion. Alternatively, they may minimize charge difference on groups that slow or prevent the discontinuous phase from coalescing or aggregating. The pH adjustments may also work by allowing the enzymes to function better in the destabilization process - e.g. allowing the enzyme and substrate to more quickly associate, enhancing actual catalysis, or by helping the product to more quickly diffuse or leave the active site, thus driving the conversion of substrate to product.

The pH of the oil-in-water emulsion may be adjusted to between about 3.5 and about 5. There are a wide variety of methods for affecting pH adjustment - including but not limited to the addition of HCl, or other acids, including organic acids, or salts thereof. For food uses, such acids are preferably food ingredients, or generally recognized as safe for use in foods by the appropriate regulatory bodies.

In some cases, at least one phospholipase activity or one protease activity in the aqueous extractant separates with the oil-in-water emulsion at the first separating step. The surviving or co-separating enzyme (phospholipase or protease) is sometimes referred to herein as carryover activity. The carryover activity is present with the oil-in-water emulsion and destabilizes that emulsion when provided with suitable conditions for activity, for an adequate time. Such a process of providing sufficient enzyme initially, then employing suitable process conditions to provide carryover activity in the later step can facilitate oil recovery by allowing the one-step addition to suffice for the entire process. The skilled artisan can readily determine whether sufficient enzyme activity has carried over into the oil-in-water emulsion.

The oilseed fraction can be obtained from soybean, corn seed, rape seed, palm kernel, sunflower seed, safflower seed, coconut, peanut, cotton seed, sesame seed, flax seed, poppy seed, almond, hazelnut, walnut, evening primrose seed, grape seed, hemp seed, black currant seed, red raspberry seed, carrot seed, cumin seed, blueberry seed, cranberry seed, parsley seed, onion seed, pumpkin seed, apricot kernel, mustard seed, linseed, or castor seed. As discussed above, various species considered to be useful or potentially useful for biodiesel and similar fuel-related uses including, for example, jatropha, oil palm, tung oil or wood oil tree, castor bean tree, diesel tree, and honge oil tree and any cultivars of any of the foregoing are also contemplated for use herein.

In another aspect, the oilseed fraction comprises a protein fraction that is useful as a food, a food ingredient, a food additive, or a food supplement. It is sometimes or even often the case that the protein fraction of the oilseed is more valuable than the oil fraction. One anticipated benefit of aqueous extraction processes is that they leave the extracted residue in a native or more functional state, e.g. the proteins are not denatured or retain greater functionality through contact with the aqueous solvent; this is valuable to the food processor. Whether for use as food or feed, or other use, the processes provided herein allow recovery of a protein fraction that has not been exposed to potentially dangerous or harmful organic solvents. As such the proteins are less likely to be denatured and may even have better consumer acceptance among certain groups of consumers.

Protein compositions prepared by the methods described herein are also provided, as are food products, consumer products, and industrial feedstock comprising a protein prepared by the methods or processes disclosed herein.

The oilseed fraction may comprise soy flakes or soy flour. It is preferred that the oilseed fraction is "full-fat" flakes or "full-fat" flour. The skilled artisan will understand that "full-fat" is a term of the art in working with oilseed fractions such as from soy and corn. Full-fat is in one sense the opposite of defatted - wherein essentially all of the lipids are removed from, for example soy flakes, soy flour, or corn germ. The skilled artisan will appreciate that the process provided herein are compatible with not only full-fat fractions, but also with partially defatted fractions of oilseed - whether flakes, meal, germ, flour, or the like.

It may be possible to improve the yield of oil from the oilseed as compared to that of an aqueous solvent extraction that does not use the step of contacting the emulsion with the enzyme activity. In a more preferred embodiment, the oil recovery approaches that obtained with an organic solvent extraction.

In yet another aspect plant-derived oils are provided, said oils prepared by any of the processes described herein. In one embodiment, the oil is substantially free of proteins, phospholipids, or aqueous impurities. Also provided are food products comprising the oil.

In another embodiment, there is described a method for preparing a wort comprising:

a) contacting starch-containing material comprising a mix of milled grain with water and a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 to form a mash;

b) slowly heating the mash of step (a) to convert the starch to sugars; and

c) separating the heated mash of step (b) into residual grain and liquid wort,

wherein the thermostable protease is present in an amount from 1-30 g/ton milled grain, such as at least 1, 2, 3, 4, 4, 5, 6, 7, 8, 9, 10, 11, 12, 13, 14, 15, 16, 17, 18, 19, 20, 21, 22, 23, 24, 25, 26, 27, 28, 29 or 30 g/ton milled grain.

Additionally, alpha- amylase may be added in step (a) along with the thermostable serine protease.

Wort production typically entails contacting a mix of milled grain with water, and, in the process described herein, a thermostable serine protease having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs:3, 8, or 10 is added in a mash tun to form a mash. The thermostable protease is present in an amount from 1-30 g/ton milled grain, such as at least 1, 2, 3, 4, 4, 5, 6, 7, 8, 9, 10, 11, 12, 13, 14, 15, 16, 17, 18, 19, 20, 21, 22, 23, 24, 25, 26, 27, 28, 29 or 30 g/ton milled grain.

In another aspect, an alpha-amylase may be added along with the thermostable serine protease.

During the mash, enzymes present convert the starches (long chain carbohydrates) in the grain into smaller molecules or simple sugars (mono-, di- and tri-saccharides.) This conversion process is called saccharification. The result of the mashing process is sugar-rich liquid or "wort", which is then strained through the bottom of the mash turn in a process known as lautering, i.e., separating. Prior to separating, the mash temperature may be raised to about 75-78°C (known as "mashout") to free up more starch and reduce mash viscosity. Additional water may be sprinkled on the grains to extract additional sugars (a process known as sparging).

Proteases can be added during the saccharification process to release the nitrogen source from proteins present in the wort. Standard Brewing quality control analyses include the test for free amino nitrogen (FAN). This allows an estimation of the concentration of individual amino acids and small peptides which may be utilized by the yeast during beer fermentation for cell growth and proliferation. The inclusion of non-malt raw materials as adjuncts in the brew process increase the requirements for higher FAN content in the wort to ensure proper fermentation process.

As described herein, a thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10 (such as but not limiting to ME-3 described herein) facilitated a dose-dependent production of free alpha-amino nitrogen (FAN) in the wort.

In another embodiment, there is described a method for hydrolyzing at least one food or animal by-product comprising:

(a) contacting the food or animal by-product with a liquid; and
(b) hydrolyzing food or animal by-product to form a liquefact by contacting with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10.

The food by-product can be a keratin rich material selected from the group consisting of feather, hair and wool.

Proteins can be hydrolyzed into polypeptides and oligopeptides of suitable length and free amino acids either by chemical hydrolysis or enzymatic hydrolysis. Protein hydrolysate preparations can be used in human nutrition, e.g., as ingredients in energy drinks, weight-control and sports nutrition products, as a source of nutrition for the elderly and underweight patients, and in the flavor industry. The protein can be derived from plants or animals.

In many facilities, by-products are created along-side the food being processed. By-products can be costly to dispose of and present a potential revenue stream.

Examples of human food by-products that can be used for animal food include grain products (hulls, bran, germ, gluten meal, grits and meal); wheat middlings generated while processing wheat for flour; peels, rinds, pomace, pulp, culls, or other similar material generated from processing fruits or vegetables for human consumption; human food such as potato chips, cookes, bread, pastry products, and pasta that is not adulterated and is safe for use as animal food, but is not acceptable as human food for quality reasons such as wrong size, shape, color or texture.

Animal by-products are carcasses and parts of carcasses from slaughterhouses, animal shelters, zoos and veterinarians, and products of animal origin not intended for human consumption, including catering waster (all waste food from restaurants, catering facilities, central kitchens, slaughterhouses and household kitchens). Examples of animal products by-products include, but are not limited to, blood meal (from slaughterhouse operations), poultry by-product meal, collagen and gelatin (from the boiled skin and other parts of slaughtered livestock), feathers (from poultry processing), feather meal (from poultry processing), lanolin (from the cleaning of wool), leather, manure, meat and bone meal (from rendering of animal bones and offal) and the like.

The thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs:3, 8, or 10 described herein can be used to remove proteins from animals and their subsequent degradation or disposal, such as, e.g., feathers, skin, hair, and hide. In some instances, immersion of the animal carcass in a solution comprising a thermostable serine protease described herein can act to protect the skin from damage in comparison to the traditional immersion in scalding water or the defeathering process. In one embodiment, feathers can be sprayed with a thermostable serine protease described herein under conditions suitable for digesting or initiating degradation of the plumage. In some embodiments, the variant can be used in combination with an oxidizing agent.

In some embodiments, the removal of the oil or fat associated with raw feathers can be assisted by using the thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 described herein. In some embodiments, one or more ME-3 (thermostable serine protease) homologs and/or variants described herein is used in compositions for cleaning the feathers as well as to sanitize and partially dehydrate the fibers. In yet other embodiments, the thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 described herein finds use in recovering protein from plumage.

Non-limiting examples of compositions and methods disclosed herein include:

1. A method for producing a thermostable serine protease comprising:

(a) transforming a production host with a recombinant construct encoding a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10; and

(b) culturing the production host of step (a) at a temperature above 37.5°C to increase expression level of the thermostable serine protease.

2. The method of embodiment 1 wherein the thermostable serine protease is optionally recovered from the production host.

3. A thermostable serine protease-containing culture supernatant obtained by the method of embodiments 1 or 2.

4. A feed, feedstuff, feed additive composition, premix, food or grain product comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 wherein said thermostable serine protease can be used alone or in combination with at least one direct fed microbial.

5. Animal feed comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO: 3, 8, or 10 wherein the thermostable serine protease is present in an amount from 1, 2, 3, 4, 5, 6, 7, 8, 9, 10, 11, 12, 13, 14, 15,16, 17, 18, 19, or 20 g /ton feed and further wherein said thermostable serine protease can be used alone or in combination with at least one direct fed microbial.

6. The feed additive composition of embodiment 4 for use in animal feed further comprising 10 and at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

7. A granulated feed additive composition for use in animal feed comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10, used either alone or in combination with at least one direct fed microbial, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

8. A granulated feed additive composition of embodiment 7, wherein the mean diameter of the particles is greater than 50 microns and less than 2000 microns

9. The feed additive composition of embodiment 7 wherein said composition is in a liquid form.

10. The feed additive composition of embodiment 9 wherein said composition is in a liquid form suitable for spray-drying on a feed pellet.

11. A method for hydrolyzing starch-containing material comprising:

(a) contacting starch-containing material with a liquid to form a mash; and

(b) hydrolyzing starch in the mash to form a liquefact by contacting the mash with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10.

12. The method of embodiment 11 wherein the enzyme cocktail further comprises at least one enzyme selected from the group consisting of alpha-amylase, amyloglucosidase, phytase, pullulanase, beta-glucanase, cellulase and xylanase.
13. A method for producing fermentation products from starch-containing material comprising:

(a) liquefying the starch-containing material with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99%sequence identity to SEQ ID NOs: 3, 8, or 10;
(b) saccharifying the product of step (a);
(c) fermenting with a suitable organism; and
(d) optionally, recovering the product produced in step (c).

13b. The method of embodiment 13 wherein steps (b) and (c) are performed sequentially.
14. The method of embodiment 13 wherein steps (b) and (c) are performed simultaneously.
15. The method of embodiment 13 or embodiment 14 wherein addition of a nitrogen source (such as but not limiting to urea or ammonia) is eliminated or reduced by at least 30%, 40%, 50%, 65%, 70%, 75%, 80%, 85%, 90%, 95%, 96%, 97%, 98%, or 99% by using at least 1, 2, 3, 4, 5, 6, 7, 8, 9, 10, 11, 12, 13, 14, 15,16, 17, 18, 19, or 20 g thermostable serine protease/MT starch-containing material having at least 80% sequence identity to SEQ ID NOs: 3, 8, or 10.
15b. The method of embodiment 13 or embodiment 14 wherein addition of a nitrogen source is eliminated or reduced by at least 30%, 40%, 50%, 65%, 70%, 75%, 80%, 85%, 90%, 95%, 96%, 97%, 98%, or 99% by using at least 3, 4, 5, or 6 g thermostable serine protease/MT dry solid having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10.
15c The method of embodiment 1b5 wherein the nitrogen source is selected from the group consisting of urea and ammonia.
15d. The method of embodiment 13 or embodiment 14, wherein levels of acid hydrolyzed fat from materials obtained at the end of the fermentation are increased, when compared to materials obtained at the end of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.
15e. The method of embodiment 13 or embodiment 14, wherein the fermentation product (such as but not limiting to ethanol) is produced at a faster rate, when compared to a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.
15f. The method of embodiment 13 or embodiment 14, wherein the fermentation product (such as but not limiting to ethanol) yield is higher, when compared to the fermentation product yield of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.
15h. The method of embodiment 13 or embodiment 14, wherein glycerol levels from materials obtained at the end of the fermentation are decreased, when compared to materials obtained at the end of a control fermentation not comprising said thermostable serine protease that has at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NOs: 3, 8, or 10.
16. The method of embodiment 15 wherein when the fermentation product is ethanol then no acid proteolytic enzyme is needed in step (c) when using at least 1, 2, 3, 4, 5, 6, 7, 8, 9, 10, 11, 12, 13, 14, 15,16, 17, 18, 19, or 20 g thermostable serine protease/MT starch-containing material having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10.
17. A method for reducing viscosity of a liquefied starch-containing material which comprises contacting a slurry of starch-containing material with a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99%sequence identity to SEQ ID NOs: 3, 8, or 10.
18. A method for extracting oil from an oilseed crop comprising:

(a) contacting an oil-containing oilseed fraction with an aqueous extractant to form an extract oilseed fraction; and
(b) separating the extracted oilseed fraction into an aqueous phase, an oil-in-water emulsion, and an insoluble phase;
(c) contacting the oil-in-water emulsion with at least a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10, either alone or in combination with at least a phospholipase, under conditions permitting enzyme activity for a time sufficient to destabilize the emulsion; and
(d) separating the destabilizing emulsion of step (c) into an aqueous phase, an oil phase, and an insoluble phase to obtain oil from the oilseed.

19. The method of embodiment 19 wherein the oilseed fraction is from soybean, corn seed, rape seed, palm kernel, sunflower seed, safflower seed, coconut, peanut, cotton seed, sesame seed, flax seed, poppy seed, almond, hazelnut, walnut, evening primrose seed, grape seed, hemp seed, black currant seed, red raspberry seed, carrot seed, cumin seed, blueberry seed, cranberry seed, parsley seed, onion seed, pumpkin seed, apricot kernel, mustard seed, linseed, castor seed or jatropha.
20. The process of embodiment 18 wherein the oilseed fraction comprises a protein fraction that is useful as a food, food ingredient, a food additive or food supplement.
21. A plant-derived oil prepared by the process of embodiment 18.
22. A feed, feedstuff, feed additive composition, premix, food or grain product comprising the oil of embodiment 21.
23. A method for preparing a wort comprising:

a) contacting starch-containing material comprising a mix of milled grain with water and a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10 to form a mash;
b) slowly heating the mash of step (a) to convert the starch to sugars; and
c) separating the heated mash of step (b) into residual grain and liquid wort

wherein the thermostable protease is present in an amount from 1-30 g/ton milled grain.
24. The method of embodiment 23 wherein at least one alpha-amylase is added in step (a) along with the thermostable serine protease.
25. A method for hydrolyzing at least one food or animal by-product comprising:

(a) contacting the food or animal by-product with a liquid; and
(b) hydrolyzing food or animal by-product in the mash to form a liquefact by contacting with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ **ID** NOs: 3, 8, or 10.

26. The method of embodiment 25 wherein the food by-product is keratin rich material selected from the group consisting of feather, hair and wool.
27. A method for hydrolyzing proteins in lignocellulosic biomass comprising

(a) contacting the biomass with a liquid; and
(b) hydrolyzing the proteins in the biomass by contacting the biomass with an enzyme cocktail comprising a thermostable serine protease having at least 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NOs: 3, 8, or 10.

EXAMPLES

Unless defined otherwise herein, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, N.Y. (1991) provide one of skill with a general dictionary of many of the terms used with this disclosure.
The disclosure is further defined in the following Examples. It should be understood that the Examples, while indicating certain embodiments, is given by way of illustration only. From the above discussion and the Examples, one skilled in the art can ascertain essential characteristics of this disclosure, and without departing from the spirit and scope thereof, can make various changes and modifications to adapt to various uses and conditions.

**EXAMPLE 1**

***Thermobifida cellulosilytica* DSM 44535 serine protease ME-3**

*Thermobifida cellulosilytica* DSM 44535, a bacterial strain obtained from the Leibniz Institute DSMZ-German Collection of Microorganisms and Cell Cultures (Braunschweig, Germany), was selected as a potential source for enzymes useful in various industrial applications. The entire genome of this strain was sequenced using ILLUMINA® sequencing by synthesis technology. Genome sequencing and assembly of the sequence data was performed by BaseClear (Leiden, The Netherlands). Contigs were annotated by BioXpr (Namur, Belgium).

One of the genes identified this way in *Thermobifida cellulosilytica* DSM 44535, *Tce01961n* (SEQ ID NO: 1), encodes a protease, designated ME-3 that showed homology to serine proteases of other bacteria. The nucleotide sequence of *Tce01961n,* encoding the protease ME-3, is set forth as SEQ ID NO:1. The amino acid sequence of the ME-3 preproenzyme encoded by *Tce01961n* is set forth as SEQ ID NO:2. At the N-terminus, the ME-3 preproenzyme has a signal peptide with a predicted length of 27 residues (amino acids 1-27 in SEQ ID NO:2) as determined using SignalP (Emanuelsson et al. (2007) Nature Protocols, 2: 953-971). The presence of a signal peptide sequence indicates that this serine protease is a secreted enzyme. Like other serine proteases, the enzyme has a pro-sequence, with a predicted length of 162 residues ( amino acids 28-189 in SEQ ID NO:2). The pro-sequence prediction was based on knowledge of the pro-mature junction in homologous proteases such as Cellulomonadin (Shaw et al., (2007) Acta Crystallogr Sect F Struct Biol Cryst Commun 63: 266-269) and *Thermobifida fusca* Protease A (Kelch BA & Agard DA (2007) J Mol Biol. 370:784-795).

The amino acid sequence of the fully processed mature enzyme, ME-3 (186 amino acids), is depicted in SEQ ID NO:3.

**EXAMPLE 2**

**Heterologous Expression of ME-3 protease**

The ME-3 protease was produced in *Bacillus subtilis* using an expression cassette consisting of the *B. subtilis aprE* promoter, a modified *B. subtilis aprE* signal peptide sequence, a codon-optimized sequence encoding the pro-mature sequence of ME-3 protease, and a BPN' terminator.

The sequence of the modified *aprE* signal peptide sequence fused to the codon-optimized DNA sequence encoding the pro-mature sequence of ME-3 protease is depicted in SEQ ID NO:4. The ME-3 expression cassette was cloned into the pHYT replicating shuttle vector and transformed into a suitable *B. subtilis* strain.

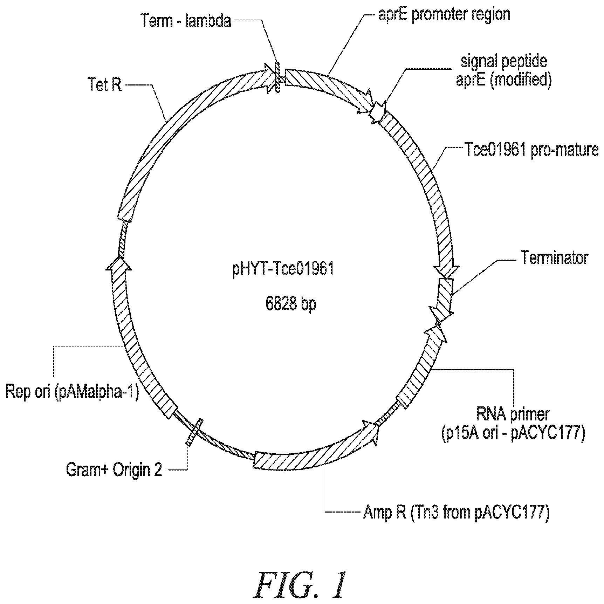

The pHYT vector was derived from pHY300PLK (Takara Bio Inc.) by adding a terminator after the tetracycline resistance gene using the *Bst*EII and *Eco*RI sites (terminator sequence is set forth as SEQ ID NO:5). The *Hind*III site in pHY300PLK was also removed using a linker cloned into the *Bam*HI and *Hind*III sites (linker sequence is set forth as SEQ ID NO:6). A map of the pHYT vector for expression of the ME-3 protease (pHYT-Tce01961) is shown in Figure 1.

To produce ME-3 protease, a 96-well tray containing 150 μL tryptone soy broth (TBS) + 15 ppm tetracycline in each well was inoculated with a *B. subtilis* transformant containing pHYT-Tce01961. The 96-well tray was incubated at overnight at 37°C, 200rpm in a shaking incubator. 30 μL of this overnight (pre)culture was added to a well in a 24-well tray containing 3 mL of enriched semi-defined media (based on MOPs buffer, with urea as major nitrogen source, glucose as the main carbon source, and supplemented with 1% soytone) supplemented with 5 mM $CaCl_2$ and 25 ppm tetracycline. The 24-well tray was incubated 32°C, 200 rpm for 48 hours.

The culture was harvested by centrifugation at 4500 rpm for 20 minutes. The resulting supernatant was filtered through a 0.22 μm filter to produce a cell-free supernatant. The supernatant contained ME-3 protease (confirmed by SDS-PAGE) and this fraction was further used for assays, and in some instances was further purified as described below. ME-3 protease was purified by incubating the cell-free supernatant for 1 hour at 70°C in a water batch.

The incubated supernatant was centrifuged at 5100g for 10 minutes and subsequently filtered through a 0.22um filter. 0.02% azide was added and the sample was buffer exchanged using Viva spin 20ml ultrafiltration tubes of 10.000 MWCO (Sartorius). Centrifugation was performed to 5-10% of the original volume and 25mM HEPES buffer pH 7.0, 1mM $CaCl_2$, 0.02% azide was added to a final volume of 20mL. This step was repeated 3 times and finally the sample was recovered from the ultrafiltration tube and 25mM HEPES buffer pH 7.0, 1mM $CaCl_2$, 0.02% azide was added to obtain the desired final volume.

The effect of temperature on the expression of ME-3 protease was evaluated. *B. subtilis* strain containing ME-3 expression cassette was cultured in 2 mL LB broth at 37°C for 8 hrs under antibiotic selection. A 100 μL aliquot of this pre-culture was added to a 125 mL baffled flask containing 10 mL of enriched semi-defined media (based on MOPs buffer, with urea as major nitrogen source, glucose as the main carbon source, and supplemented with 1% soytone) supplemented with antibiotics. The flasks were incubated for 2 days at 37°C or 42°C with constant rotational mixing at 250 rpm. The culture supernatant was recovered by centrifugation (20,000 g for 1 min) and used for quantification of ME-3 protease using the Suc-AAPF-pNA substrate as described in Example 3 below.

The concentration of ME-3 protease was calculated using the standard curve generated using purified enzyme. The average yield of ME-3 protease (3 replicates) from cultures grown at 37°C and 42°C is reported in Table 1 below. As shown, an increase of approximately 50% in the yield of ME-3 protease was obtained when cells were grown at 42°C compared to 37°C.

| Table 1. Relative expression levels of ME-3 serine protease in *Bacillus subtilis* cultured at 37 or 42° C. | | |
|---|---|---|
| | 37°C | 42°C |
| ME-3 yield (ppm) | 540±74 | 807±86 |

**EXAMPLE 3**

**In Vitro ME-3 Protease Activity**

**Effect of ME-3 on Corn Soy Feed in the Presence of Pepsin and Pancreatin**

1. Enzyme treatment of cornsoy feed

Cornsoy feed flour (composition shown on Table 2) was sifted to a particle size less than 212μm and suspended in water to 10% (w/w) slurry and pH adjusted to pH 3. 138μL of this slurry was added to each well in 96 MTP well-plate. A 20 μL sample of ME-3 protease prepared in 50mM acetate pH 3 was added, then 10μLporcine pepsin (Sigma, P7000, prepared at 800 U/mL in water) was added. The plate was incubated at 40°C for 45 min in iEMS microplate shaker incubator (iEMS) at 1150 rpm. Then, 34μL porcine pancreatin (Sigma P7545, prepared as 0.463 mg/mL in 1M Na-bicarbonate) was added and the plate was incubated at 40°C for 60 minutes in iEMS at 1150 rpm. Afterwards, the plate was centrifuged at 5°C, 4000 rpm for 15 min, 20μL supernatant was transferred to new plates (corning plate #3641 non-binding) containing 180μL water in each well to a 10x dilution.

2. Degree of protein hydrolysis measurement

The degree of hydrolysis (DH) of protein is based on the reaction of primary amino groups with ortho-phthaldialdehyde (OPA assay) as described by P.M. Nielsen, D. Petersen and C. Dambmann. Improved Method for Determining Food Protein Degree of Hydrolysis. Journal of Food Science. 66 (2001) 642-646. This OPA assay was used to determine extent of hydrolysis.

3. Catalytic activity measurement using AAPF substrate in solution

To measure catalytic activity of the protease, the substrate Suc-AAPF-pNA (Sigma, S-7388) was used. The activity was measured as follows: 5μL supernatant containing enzyme, 50μL 0.2M Mes-NaOH (pH6.0), 50μL water or 55μL water (blank), 5μL Suc-AAPF-pNA (20 mg/mL) were added to a half bottom area of 96 well microplate (Costar 3695, Corning Inc. NY, USA). The extent of hydrolysis was followed at 410 nm for up to 20 min.

4. Catalytic activity measurements on feed extracts

For the assay of ME-3 recovery in feed pelleting process, prior to the assay, feed samples were ground using a Perten laboratory mill with a particle size <0.75μm selection. 5 gram of the ground feed was dissolved in 50 mL of 0.1 M Tris pH 10 solution containing 1% (w/v) SDS, and centrifuged. The supernatant was filtered and used for detection of protease activity using the AAPF-pNA substrate and method described above. The composition of cornsoy feed as presented in the Table 2 was derived from the following reference: Interactions of phytate and myo-inositol phosphate esters ($IP_{1-5)}$ including $IP_5$ isomers with dietary protein and iron and inhibition of pepsin. S. Yu, A. Cowieson, C. Gilbert, P. Plumstead and S. Dalsgaard. J. Anim. Sci. 90 (2012) 1824-1832, Supplementary Information.

| **Table 2. Composition of cornsoy feed** | |
|---|---|
| **Ingredient** | **Amount, %** |
| Corn | 60.01 |
| Soybean meal | 31.52 |
| Soy oil | 4.00 |
| Salt | 0.40 |
| DL-Methionine | 0.20 |
| Limestone | 1.16 |

(continued)

| Table 2. Composition of cornsoy feed | |
|---|---|
| Ingredient | Amount, % |
| Di calcium Phosphate | 1.46 |
| Vitamin and mineral mixture | 1.25 |

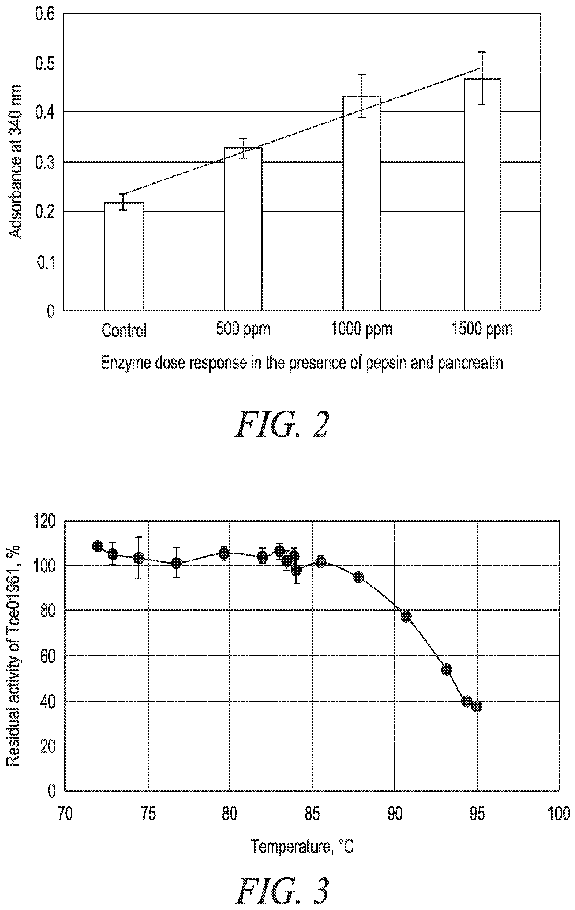

The results of cornsoy feed treatment with ME-3 protease at different concentrations (500, 1000, 1500 ppm) in the presence of pepsin and pancreatin enzymes are presented below on Figure 2.

**EXAMPLE 4**

**Thermostability of ME-3 protease at various pH**

Aliquots of ME-3 protease were incubated for 60 seconds in 0.1M Tris pH10 containing 5mM $CaCl_2$ and 0.005% Triton X-100, in a 96-well MTP plate at temperatures ranging from 72°C to 95°C. The plate was cooled down to 4°C, and the catalytic activity was measured at 37°C using the AAPF-pNA substrate using method described on Example 3. The results are presented in Figure 3. As can be seen on Figure 3, ME-3 retains about 80% activity at 90°C at pH 10. In other experiments, no effect of EDTA was detected (data are not presented). The thermal stability of ME-3 was also measured at pH 4.5 and pH 5.5 using 0.1M acetate It was found that after incubation at 85.5°C for 10min, the ME-3 enzyme retains about 85% activity at pH 4.5 and 5.5, while during incubation at 100°C for 5 min, the residual activity at pH 5.5 is about 65%.

**EXAMPLE 5**

**pH Profile of ME-3 Protease**

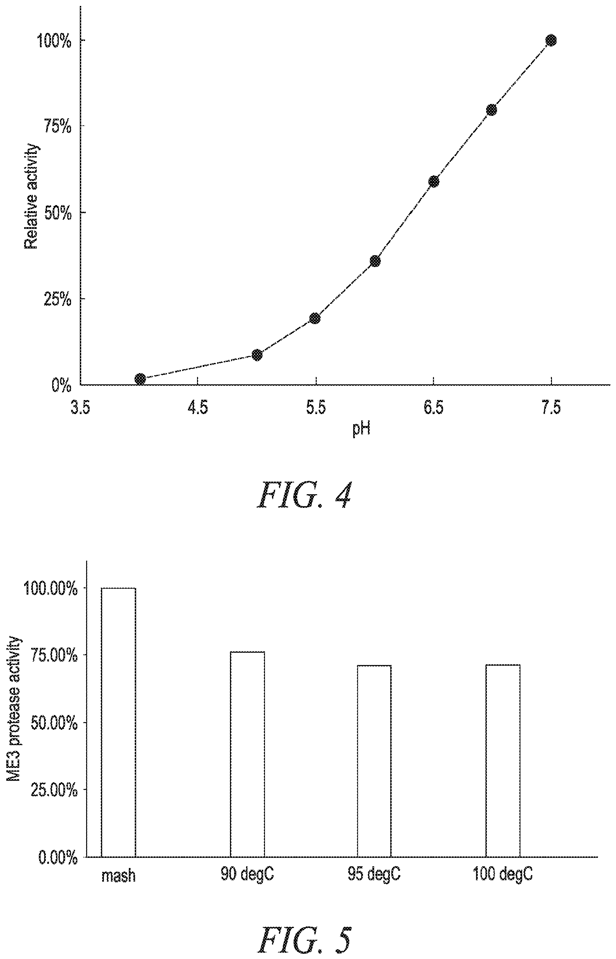

The effect of pH on the activity of ME-3 protease was evaluated between pH 3.75 and 7.5. Two stock buffers were used to generate that pH range: 0.1M citrate buffer and 0.2M phosphate buffer, both containing 0.05% (w/v) Tween-80. The catalytic activity of the enzyme was measured at 40°C using the AAPF-pNA substrate assay described in Example 3, and the results are presented in Figure 4. The stability of ME-3 protease at low pH was also studied. It was found that after 30 min incubation at pH 2.5 the enzyme retained 90% of the activity relative to that measured at pH 5.0 (data not shown).

**EXAMPLE 6**

**Pelleting stability of directly sprayed ME-3 protease on feed**

The stability of the ME-3 protease during pelleting of animal feed was evaluated by measuring the percent enzyme activity recovery after the procedure. Pelleting of feed was performed at different temperatures at the Danish Technological Institute (Kolding, Denmark) according to standard operational procedures. ME-3 enzyme without coating was diluted into 600 mL of water, and then sprayed directly onto 120 Kg of feed. This feed sample with sprayed-on ME-3 was pelleted at either 90, 95 or 100°C for 60 seconds. For determination of protease stability after this procedure, the pellets were ground using a Perten laboratory mill and extracted in 50 mL of 0.1 M Tris pH 10 solution containing 1% (w/v) SDS at 22°C for 20min. The extract was centrifuged and filtered through filter paper. The filtrate then used to determine the catalytic activity of ME-3 using the AAPF-pNA substrate assay described in Example 3. As shown on Figure 5, the ME-3 enzyme without any coating retains more than 70% activity after pelleting at 90°C to 100°C for 60 seconds.

**EXAMPLE 7**

**Evaluation of ME-3 Protease in Starch Liquefaction and Simultaneous Saccharification and Fermentation**

The effect of ME-3 protease was tested in lab scale corn liquefaction and simultaneous saccharification and fermentation (SSF) protocols as described below. Corn kernels (Arie Blok Animal Nutrition, NL-3440 AA Woerden, Artnr: 3777) were milled using a Retsch ZM200 grinding machine with settings: 3mm screen, 10k rpm. The milled corn flour was used to generate 2 kG slurry at 32.0% dry solids by adding a 1:1 mixture of tap water/demineralized water to the flour. The pH was adjusted to 5.5 with $H_2SO_4$ and afterwards SPEZYME® RSL (DuPont commercial product containing a bacterial alpha amylase) was dosed at a relevant commercial dose. Subsequently, the ME-3 protease was added to a final concentration of 5ug/gDS and the 2kG mixture was incubated at 85°C for two hours under constantly stirring using an overhead mixer. A control sample was generated by adding no protease to the liquefaction. Following the incubation, the treated corn sample (Liquefact) was collected and used in subsequent simultaneous saccharification and fermentation (SSF) experiments, and aliquots were also analyzed by HPLC size exclusion to determine the extent of corn liquefaction. Viscosity analysis during corn liquefaction. Corn kernels (Arie Blok Animal Nutrition, NL-3440 AA Woerden, Artnr: 3777) were milled using a Retsch ZM200 grinding machine, settings: 3mm screen, 10k rpm. The milled corn flour was used to generate a 25 gram 32% dry solids corn slurry by adding a 1:1 mixture of tap water/demineralized water to the flour. The pH was adjusted to 5.5 with sulfuric acid and afterwards SPEZYME® CL (DuPont commercial product containing a bacterial alpha amylase) was added at a commercial relevant dose. To this slurry the ME-3 protease was added to a final concentration of 10 microgram/gram dry solid (ug/gDS). As a control sample there was no protease added. The samples were incubated in a rapid viscosity analyzer (Perten Instruments, RVA4500, settings: 160rpm) for 1hour at 85°C and afterwards the temperature was lowered to 32°C. After 15 minutes at 32°C the average final viscosity levels of the last minute of incubation were taken and the result is shown in Table 3.

**Table 3. Final Viscosity levels of a liquefaction with and without ME-3 protease.**

| | Final viscosity (cP) |
|---|---|
| SPEZYME CL alone (no protease control) | 2102 |
| SPEZYME CL + ME-3 | 1641 |

Simultaneous saccharification and fermentation (SSF)

The pH of the liquefact samples (with and without protease added to liquefaction reaction) were adjusted to 4.8 and DISTILLASE® SSF (DuPont commercial product containing a fungal alpha amylase, a fungal glucoamylase, and an acid fungal protease) was added at a commercial relevant dose. Afterwards, 0.1% w/w active dry yeast (Ethanol Red, Fermentis, France) was added. A 50gram sample of this mixture was aliquoted into SSF vessels. Zero or 0.178 SAPU/g DS of FERMGEN™ 2.5x (DuPont commercial product containing an acid fungal protease) and various amounts of urea (0, 200 and 600 ppm) were added. The experiments were performed in duplicate. The fermentation incubations were performed using two different methods described below.

SSF Method 1. The fermentation vessels were incubated at 32°C in a water bath with magnetic stirring at 300rpm was applied. Samples were collected at four different time points (5h, 22h, 29h and 46hours). These samples were analyzed for ethanol concentration using HPLC separation and quantitation as described below.

The HPLC (Agilent Technologies 1200 series) run conditions were as follows: Phenomenex Rezex™ RFQ-Fast Acid H+ column held at 80°C, run at 1.0mL/min isocratic flow of 0.01N $H_2SO_4$ solvent, a 10 μL injection volume, and 5.3 min elution runtime. Refractive index detection was used for quantification of ethanol, and the results are shown in Table 4.

**Table 4. SSF ethanol yields (% v/v) obtained with various urea quantities, with and without ME-3 protease during corn liquefaction step.**

| | | | | Time (hours) | | | |
|---|---|---|---|---|---|---|---|
| **Liquefaction enzyme** | **ME-3 (ug/gDS)** | **FERMGEN™ 2.5x, (SAPU/g DS)** | **Urea (ppm)** | **5** | **22** | **29** | **46** |
| SPEZYME RSL | 0 | 0.178 | 600 | 1.4 | 12.4 | 15.5 | 17.8 |
| SPEZYME RSL | 0 | 0.178 | 200 | 1.4 | 9.8 | 12.9 | 16.9 |
| SPEZYME RSL | 0 | 0.178 | 0 | 1.2 | 8.2 | 11.1 | 15 |
| SPEZYME RSL | 5 | 0.178 | 0 | 1.2 | 12 | 15.8 | 17.8 |
| SPEZYME RSL | 5 | 0.178 | 200 | 1.4 | 12.9 | 16.8 | 17.7 |

Table 4 shows that if urea dose is reduced from 600 to 200ppm (equivalent to 67% urea dose reduction) the rate of ethanol formation by liquefying enzymes such as SPEZYME RSL is slower. When ME-3 protease was added to the liquefaction mix including SPEZYME RSL, the fermentation rate is comparable to the one obtained when 600 ppm urea was included. In addition, addition of ME-3 to liquefaction mix including SPEZYME RSL yielded that same ethanol yield when either zero or only 200 ppm urea were used instead. Therefore, ME-3 addition during liquefaction allows for a significant reduction, or elimination in urea requirement.

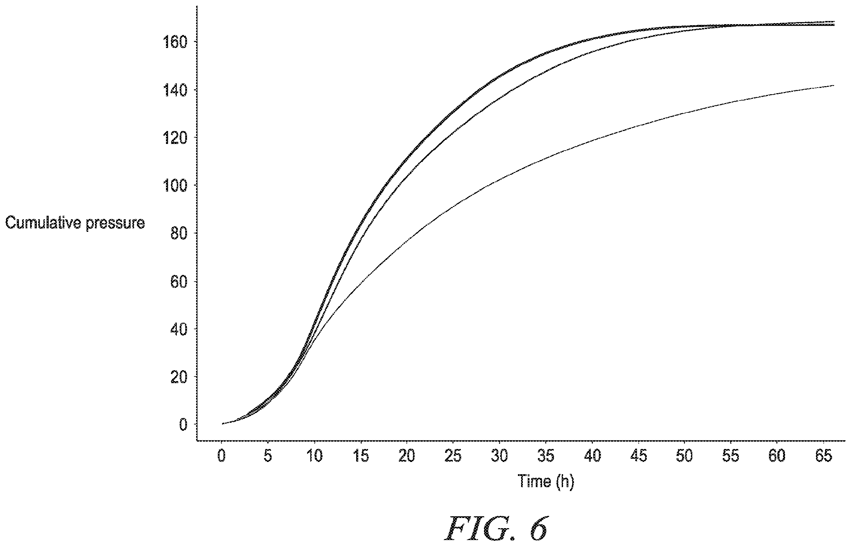

SSF Method 2. The fermentation vessels were incubated at 32°C in a water bath and magnetic stirring at 300rpm was applied. Cumulative pressure was recorded over time using the ANKOM RF Gas Production System (ANKOM Technology, Macedon, New York, USA) which provides an easy to use method for monitoring and measuring gas production. Gas ($CO_2$) production is a measure for ethanol production. The rates of gas production are shown in Figure 6. Figure 6 shows the cumulative pressure plots from simultaneous saccharification and fermentation performed on Ankom system. In medium-grey line is the control condition where 600ppm urea and 0.178 SAPU/gDS FERMGEN™ 2.5x was added to the fermentation, and no ME-3 was used in the liquefaction. The light grey line shows the ethanol yields using 240ppm urea and no FERMGEN™ 2.5x added to the fermentation and no ME-3 was used in the liquefaction. The black line shows the ethanol yields when ME-3 protease was added to the liquefaction and the fermentation was performed with 240 ppm urea and no FERMGEN™ 2.5x addition. As can be observed, even with a 60% lower urea dose and no FERMGEN™ protease added to the fermentation, when ME-3 protease was added to the liquefaction, ethanol yield was improved as compared to the control conditions.

Detection of corn flour protein hydrolysis by HPLC analysis of corn of liquefaction samples. To assess corn protein hydrolysis by the ME-3 protease, HPLC-size exclusion was used to quantify corn protein solubilization. The end-of-liquefaction samples were spun down at 13,000 rpm for 5 minutes and 200 uL of supernatant was filtrated through a 0.22um filter. A 5uL aliquot of this filtrate, was injected on an HPLC (Agilent Technologies 1200 series), on a Waters BEH125Å, 1.7um/300mm column; and run at 0.4mL/min. Running buffer: 25mM $NaPO_4$ pH6.8, 0.1M NaCl. Total area between retention time 7min to 10min was integrated, Detection: OD220nm.

Results of the HPLC detection of peptides in liquefaction samples are shown in Table 5, as the integrated area under the curve with retention time between 7-10minutes for samples treated with and without ME-3 protease. The protein hydrolysis results shown on Table 5 indicate that when ME-3 is added in liquefaction an increase in the levels of soluble peptides were observed.

**Table 5. HPLC detection of peptides in liquefaction samples.**

| Sample | Relative area under the curve |
|---|---|
| SPEZYME RSL | 13,908 |
| SPEZYME RSL + ME-3 | 35.990 |

**EXAMPLE 8**

**Use of ME-3 Protease for Free Amino Nitrogen Production in High Temperature Mashing**

Standard Brewing quality control analyses include the test for free amino nitrogen (FAN). This allows an estimation of the concentration of individual amino acids and small peptides which may be utilized by the yeast during beer fermentation for cell growth and proliferation. The inclusion of non-malt raw materials as adjuncts in the brew process increase the requirements for higher FAN content in the wort to ensure proper fermentation process. The protease ME-3 was tested in mashing operation with 50% Pilsner malt (Pilsner malt; Fuglsang Denmark, Batch 13.01.2016) and 50% corn grist (Benntag Nordic; Nordgetreide GmbH Lübec, Germany, Batch: 02.05.2016.), using a water to grist ratio of 4.0:1. Pilsner malt was milled with a Buhler Miag malt mill (0.5 mm setting).

Maize grits (1.5g), Malt (milled pilsner malt, 1.5g) and tap water (12.0g) was mixed in Wheaton cups (Wheaton glass containers with cap) preincubated with 12.0g tap water pH adjusted to pH 5.4 with 2.5M sulphuric acid and heated to 70°C. ME-3 protease was added based on ppm active protein (in total 0.5mL) and water as no enzyme control. A commercially relevant dose of alpha-amylase (Amylex® 5T, DuPont commercial product) was applied to all samples to facilitate liquefaction. Wheaton cups were placed in a dry bath (Thermo Scientific Stem station) with magnetic stirring and the following mashing program was applied; kept at 70°C for 30 minutes; heated to 95°C for 12.5 minutes by increasing temperature with 2°C/minute; kept at 95°C for 17.5 minutes; cooled to 70°C for 15 minutes and held at 70°C for 30 minutes and finally heated to 79°C for 15 minutes and mashed off. A10 mL sample was transferred to Falcon tubes and boiled at 100°C for 20 minutes to ensure complete enzyme inactivation. Spend grains was separated from the wort by centrifugation in a Multifuge X3R centrifuge (Heraeus) at 4500 rpm for 30 minutes at 10°C.

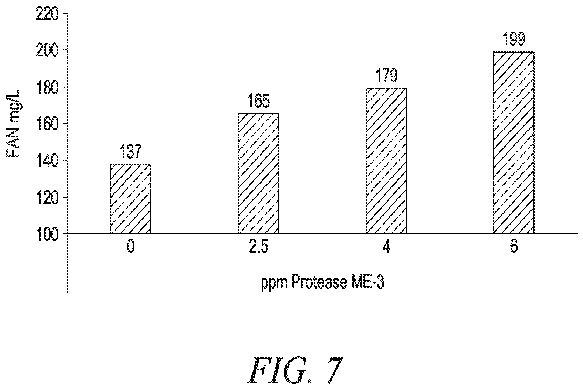

Supernatant was collected for free Alpha Amino Nitrogen analysis (FAN). The FAN content (mg/litre) was measured in the wort with the EBC 8.10 international method for the determination of the free amino nitrogen content of wort by colorimetry (applying a Genesys 10S UV-Vis Spectrophotometer) using ninhydrin. As shown in Figure 7, the ME-3 protease facilitated a dose-dependent production of free alpha-amino nitrogen (FAN) in the wort.

**EXAMPLE 9**

**Oil extraction from oil seed with the aid of ME-3 protease**

Flaked, de-hulled and ground peanut seeds (*Arachis hypogaea*) were suspended in 2mL 0.1M Tris-HCl containing 5mM $CaCl_2$ (pH8.0) and incubated at 60°C with shaking at 1180 ppm for 3 h to mimic the oil extraction process. At the end of the extraction, the oil water supernatant was collected by centrifugation at 4000 rpm for 15min using a benchtop centrifuge. The collected supernatant was treated with ME-3 protease to hydrolyze the proteins partitioned in the oil water emulsion. Briefly, a 120 μL aliquot of supernatant was mixed with increasing amounts of ME-3 (3.7μg protein/μL stock solution) protease: 0, 1, 3, 5 and 10μL and was incubated at 60°C and1180 rpm shaking for 1h (n=2). At the end of the incubation period, the reaction mixture was cooled to 0°C and 20μL of each sample were taken to measure the turbidity at 600nm. The results are shown on Table 6, and they show that addition of ME-3 protease reduced the turbidity of the sample.

**Table 6. Effect of ME-3 on the hydrolysis of proteins in peanut oil extraction.**

| | **ME-3 dose (μL)** | | | | |
|---|---|---|---|---|---|
| | **0** | **1** | **3** | **5** | **10** |
| .Absorbance at 600nm | 0.377 | 0.288 | 0.244 | 0.248 | 0.286 |
| Percent (%) reduction in turbidity | 0 | 23.6 | 35.3 | 34.2 | 24.1 |

This decreased in turbidity of the reaction mixture is attributed to the hydrolysis of proteins in the oil protein emulsion. It is well known that plant oil can be extracted with organic solvents like hexane but the risk for fire and explosion is high. Plant oil extraction with aqueous solution at reduced temperature can avoid these risks but the formation of water oil emulsion is a challenge since it is difficult to separate the free oil from the water phase. Use of proteases to hydrolyze the plant material or plant seed cell wall to release more trapped oil, and also the protein parts in the water oil emulsion increases the oil yield (as described in patent application US2010227042).

The use of a thermostable protease like ME-3 is more compatible for the high temperature oil extraction process from plant and algae than those proteases disclosed in patent US2010227042 which are less thermostable. Lipases including phospholipases may also be used in the process, but the advantage of using a thermostable protease is that it hydrolyzes plant proteins including those that may cause allergenicity and proteinaceous inhibitors that negatively affect digestion, thus producing a more desirable product.

**EXAMPLE 10**

**Keratin hydrolysis with ME-3 protease**

The evaluation of keratin hydrolysis by ME-3 protease was performed as described below. ME-3 protease (3.7mg protein/mL) dosed at 0, 5μL, 10μL and 20μL was added to a 2mL suspension of 1% (w/w) wool keratin (K0044, Tokyo Chemical Industry Co. Ltd) suspended in 0.1M Tris-HCl containing 5mM $CaCl_2$ (pH8.0) and 0.1mL cysteine (0.1M). The reactions were performed at 60°C with shaking at 1180 rpm for 3 h (n=3). At the end of the incubation period, the reaction mixtures were centrifuged at 10,000 rpm for 20 min. The supernatant was removed and the precipitate was dried and weighed to determine the amount of hydrolyzed wool. The results are shown on Table 7, and indicate that increasing amounts of ME-3 protease effectively hydrolyze wool keratin.

**Table 7. Use of ME-3 for the hydrolysis of wool keratin.**

| | **ME-3 Enzyme dose (μL)** | | | |
|---|---|---|---|---|
| | 0 | 5 | 10 | 20 |
| Wool keratin residual weight (mg) | 20.07 | 11.97 | 10.03 | 8.77 |
| Percentage (%) of hydrolysis | 0 | 40.3 | 50.0 | 55.5 |

Keratin-rich materials including poultry feather, mammal hair and wool are highly crosslinked proteins that are notoriously difficult to hydrolyze with enzymes. Certain proteases can hydrolyze some of these keratin materials to some extent, such as feather (Pedersen et al., Comparison of four feed proteases for improvement of nutritive value of poultry feather meal. J. Anim. Sci. 2012, Vol. 90 No. Supplement_4, p. 350-352). However, none of the previously evaluated protease are efficient, and none can hydrolyze wool keratin due to their instability at the elevated temperatures.

The efficiency observed for the ME-3 protease to hydrolyze keratin wool material may be due to its tolerance to high temperature, since keratin proteins unfold more readily at higher temperature. In addition, the current process for making feather meal for animal feed and pet food usually involves the treatment of feathers (high in keratin content) with high temperature to kill possible pathogens and to denature feather proteins. ME-3 protease could either be used alone or in combination with the high temperature treatment of feathers (as described in patent number US2015197783).

**EXAMPLE 11**

**Use of ME-3 protease to hydrolyze proteins in biomass**

Biomass for second generation bioethanol production or other purposes requires a pretreatment before being subjected to enzyme hydrolysis to produce fermentable sugars. Biomass is usually pretreated at elevated temperatures, typically higher than 100°C, either under acidic or alkaline conditions (as described in patent application WO2006110901, WO2014202716).

A thermostable protease such as ME-3 may be used in such a process to hydrolyze the proteins present in the biomass in order to provide the fermenting organisms such as ethanologen with additional amino acids and peptide nutrients, and also to reduce the viscosity of the biomass slurry to increase its fluidity. Alkaline pretreated biomass, such as dilute ammonia pretreatment (as described in patent WO2006110901) would be especially suitable for ME-3 treatment to generate these nutrients due to the higher pH optimum of ME-3. To evaluate the effect of ME-3 under these conditions, corn stover (63% solids) pretreated with diluted ammonia and suspended in deionized water at 10% (w/v) was used. The pH of this slurry was 8.25. The slurry was distributed into 12 tubes each containing 4mL slurry. ME-3 protease (3.7mg/ml) was dosed at 0, 1, 5, 10, 15, and 25 μL (n=2). The samples were mixed and reaction was performed at 60°C for 4h. The samples were then centrifuged and the supernatant collected was filtered through 0.45 μm filter and diluted 10 times with water. The samples were used to measure free amino groups from proteins and amino acids, by reacting 20uL of the diluted sample with OPA reagent as described in Example 3. The extent of protein hydrolysis from biomass treatment by ME-3 are shown on Table 8. From Table 8, it can be seen that free amino groups as indicated by OPA value increased with the addition of ME-3.

**Table 8. Use of ME-3 to hydrolyze proteins in biomass.**

| ME-3 dose, μL | 0 | 1 | 5 | 10 | 15 | 25 |
|---|---|---|---|---|---|---|
| OPA 340nm (average) | 0.431 | 0.449 | 0.491 | 0.483 | 0.485 | 0.541 |
| Stdev | 0.017 | 0.016 | 0.016 | 0.019 | 0.035 | 0.059 |
| CV | 4.0 | 3.5 | 3.2 | 4.0 | 7.2 | 11.0 |
| Percentage (%) | 100 | 104 | 114 | 112 | 112 | 125 |

**EXAMPLE 12**

**Homologs of ME-3 Protease**

A BlastP search with ME-3 protease (SEQ ID NO:3) against the nr and pat databases at NCBI revealed homologs of ME-3 protease in two other Thermobifida species: *Thermobifida fusca* and *Thermobifida halotolerans.*

A homolog present in *Thermobifida fusca*, Tfpa, was first described by Kelch,B.A. and Agard,D.A. (2007) J. Mol. Biol. 370: 784-795. The accession numbers of Tfpa at NCBI (PDB ID) is 2PFE. Accession numbers WP_011290931, WP_016188200 and SCV75185 are identical proteins (mature chains). The NCBI protein accession number of the ME-3 protease homolog in *Thermobifida halotolerans* (Thpa) is WP_068687914. The amino acid sequence of *Thermobifida fusca* Tfpa preproenzyme is set forth as SEQ ID NO:7. At the N-terminus, the Tfpa preproenzyme has a signal peptide with a predicted length of 31 residues (amino acids 1-31 in SEQ ID NO:7). The pro-sequence of Tfpa has a length of 151 residues (amino acids 32-182 in SEQ ID NO:7) , and the amino acid sequence of the fully processed mature enzyme, Tfpa (186 amino acids), is depicted in SEQ ID NO:8.

The amino acid sequence of *Thermobifida halotolerans* WP_068687914 peptidase S1, Thpa, is set forth as SEQ ID NO:9. Based on alignments with ME-3 protease and Tfpa preproenzyme, the sequence of WP_068687914 seems to lack part of the N-terminal signal peptide. The predicted, partial signal peptide corresponds to 15 residues(amino acids 1-15 in SEQ ID NO:9). The predicted pro-sequence of *Thermobifida halotolerans* WP_068687914 has a length of 152 residues (amino acids 16-167 in SEQ ID NO:9). The amino acid sequence of the predicted fully proc essed mature enzyme, *Thermobifida halotolerans* WP_068687914, Thpa (186 amino acids), is depicted in SEQ ID NO: 10.

Additional homologs of ME-3 protease were found by BLAST P search of NCBI database that is outside the genus *Thermobifida* include the following S1 proteases: *Nocardiopsis potens* protease (WP_017594871), *Marinactinospora thermotolerans* (WP_078763344.1), *Amycolatopsis marina* (WP_091675221.1), *Actinoalloteichus hymeniacidonis* (WP_069846166.1), *Nocardiopsis trehalosi* (WP_067965505.1), *Saccharothrix* sp. NRRL B-16314 (WP_033441214.1). The percent sequence identity across all the mature enzyme sequences was calculated and are shown on Table 9.

The amino acid sequence of *Nocardiopsis potens* WP_017594871 is set forth as SEQ ID NO:11. The predicted signal peptide is 29 amino acids long. The predicted pro-sequence of *Nocardiopsis potens WP_017594871* protease has a length of 161 amino acids, and the amino acid sequence of the predicted fully processed mature enzyme, *Nocardiopsis potens* WP_017594871 serine protease (185 amino acids), is depicted in SEQ ID NO:12.

**Table 9. Percent sequence identity among the various proteases homologous to ME-3.** Organism of origin and accession number are listed in the row and column headings.

| | ME-3 | T fusca WP_011290931 | T halotolerans WP_068687914 | M. thermotolerans WP_078763344 | A. marina WP_091675221 | N. potens WP_017594871 | A. hymeniacidonis WP_069846166 | N. trehalosi WP_067965505 | Saccharothrix sp. WP_033441214 |
|---|---|---|---|---|---|---|---|---|---|
| ME-3 | 100 | 87.6 | 83.2 | 69.8 | 69.4 | 67 | 66.1 | 66.7 | 66.3 |
| T fusca WP_011290931 | 87.6 | 100 | 82.7 | 70.4 | 68.9 | 69.1 | 65.1 | 67.2 | 64.1 |
| T halotolerans WP_068687914 | 83.2 | 82.7 | 100 | 64.9 | 67.2 | 63.3 | 64 | 63.3 | 63.6 |
| M. thermotolerans_WP_078763344 | 69.8 | 70.4 | 64.9 | 100 | 65.9 | 72.2 | 67.6 | 72.2 | 65.4 |
| A. marina_WP_091675221 | 69.4 | 68.9 | 67.2 | 65.9 | 100 | 68.1 | 69.4 | 65.4 | 86.6 |
| N. potens_WP_017594871 | 67 | 69.1 | 63.3 | 72.2 | 68.1 | 100 | 71.9 | 76.2 | 63.9 |
| A. hymeniacidonis_WP_069846166 | 66.1 | 65.1 | 64 | 67.6 | 69.4 | 71.9 | 100 | 66.1 | 66.8 |
| N. trehalosi_WP_067965505 | 66.7 | 67.2 | 63.3 | 72.2 | 65.4 | 76.2 | 66.1 | 100 | 65.9 |
| Saccharothrix sp._WP_033441214 | 66.3 | 64.1 | 63.6 | 65.4 | 86.6 | 63.9 | 66.8 | 65.9 | 100 |

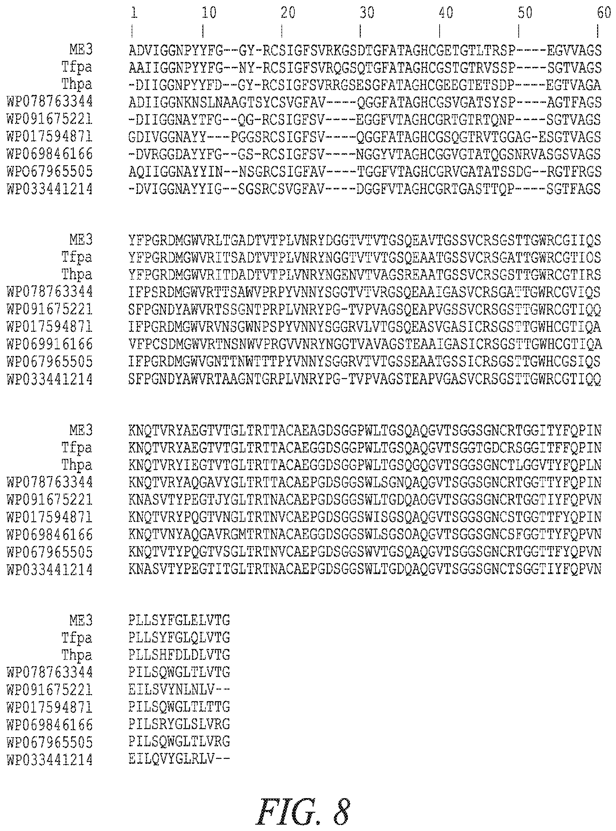

A multiple sequence alignment of the mature amino acid sequences for ME-3 (SEQ ID NO:3), Tfpa (SEQ ID NO:8), Thpa (SEQ ID NO: 10), N. potens_WP_017594871 (SEQ ID NO:12), *Marinactinospora thermotolerans* WP_078763344 (amino acids197-381); *Amycolatopsis marina* WP_091675221 (amino acids 193-435);; *Actinoalloteichus hymeniacidonis* WP_069846166 (amino acids 201-385); *Nocardiopsis trehalosi* WP_067965505 (amino acids 191-375); and *Saccharothrix* sp. NRRL B-16314 WP_033441214 (amino acids 191-437) is shown in Figure 8. The sequences were aligned with default parameters using the MUSCLE program from Geneious software (Biomatters Ltd.) (Robert C. Edgar. MUSCLE: multiple sequence alignment with high accuracy and high throughput Nucl. Acids Res. (2004) 32 (5): 1792-1797).

## EXAMPLE 13

### Effect of ME-3 protease on *in vitro* corn dry matter digestibility and gas production in ruminal fermentation

The effects of the ME-3 protease was evaluated on dry matter digestibility (DMD), gas production, starch digestibility and pH in an *in-vitro* batch fermentation system using dent corn as substrate (4 mm ground). The enzyme efficacy was evaluated at three doses (0.25, 0.5, and 0.75 mg/g of feed substrate) in three separate runs with four replicates per enzyme-dose in each run. The effectiveness of the ME-3 was determined on the rumen fermentation pattern after incubation of buffered rumen contents with substrate and enzyme in 160 mL serum vials for 7 h 39°C.

The experiments were based on the protocol established and published earlier (Adesogan, A.T., Krueger, N.A., and Kim, S.C. 2005. Anim. Feed Sci. Technol. 123: 211-223). The dent corn used in this *in vitro* batch fermentation system had 88.8% dry matter (DM), 9.3% crude protein (CP), 3.2% acid detergent fiber (ADF), 8.3% neutral detergent fiber treated with amylase (aNDF), 76.9% non-fibrous carbohydrates (NFC), 88.0% total digestible nutrients (TDN). The ground dent corn (4 mm) was weighed in 6 replications per run into the F57 filter bags (ANKOM Technology, Macedon, NY).

Prior to placement in the bottles, the enzyme sample was diluted in 0.1 M citrate-phosphate buffer (pH 6.0) and added to 0.5 g of the ground dent corn in F57 bags (Krueger, N. A., and A. T. Adesogan. 2008. Anim. Feed Sci. Tech. 145: 84-94; Goering, H. K. and P. J. Van Soest. 1970. Agric. Handbook No. 379. ARS USDA, Washington, DC, pp. 20). The F57 filter bags were sealed with an Uline Tabletop Poly Bag Sealer (Impulse® type AIE-200) then immediately placed in the fermentation vessels (160 mL serum bottles). Blank bottles consisting of only a filter bag, as well as controls containing only ground dent corn with no enzyme were also included. Ruminal fluid was representatively aspirated from three lactating ruminally-cannulated Holstein dairy cows 2 to 3 h after consuming total mixed ration (TMR). The TMR ingredient composition fed to the fistulated dairy cows based on dry matter consisted of 38.2% corn silage, 27.3% ground shelled corn, 14.5% soybean meal with 44% crude protein, 9.1% Citrus pulp, 4.5% Feedlot premix, 4.0% alfalfa hay mid bloom, 1.8% energy booster (MS Specialty Nutrition, Dundee, IL), 0.5% Novasil (BASF, Germany), 100% in total.

Rumen fluid collected was filtered through four layers of cheesecloth prior to mixing with pre-warmed artificial saliva (39°C). The composition of artificial saliva included a micromineral solution of $CaCl_2 \cdot 2H_2O$, $MnCl_2 \cdot 4H_2O$, $CoCl_2 \cdot 6H_2O$, $FeCl_2 \cdot 6H_2O$; a macromineral solution of $Na_2HPO_4 \cdot 12H_2O$, $KH_2PO_4$, $MgSO_4 \cdot 7H_2O$; a buffer solution of $(NH_4)_2HCO_3$ and $NaHCO_3$; a trypticase peptone solution (tryptone, Sigma-Aldrich, St Louise, MO, USA); oxidation-reduction indicator resazurin and a reducing solution containing cysteine HCl, 1 M NaOH, $Na_2S \cdot 9H_2O$ and distilled water. The volume ratio between the rumen fluid and the artificial saliva is 1:2. Buffered rumen fluid (52 mL) was added to 160 mL serum bottles containing the filter bags and the bottles were closed with rubber stoppers and sealed with aluminum seals. The bottles were incubated for 7h at 39°C in a forced-air incubator. At the end of incubation, the filter bags containing the residues were oven dried at 60°C for 48 h and weighed in order to quantify DMD. The gas pressure within the bottles was measured with a pressure transducer and posteriorly converted to gas volume at 7 h incubation. The following formula was used for the conversion of gas pressure to gas volume:

$$\text{Gas volume (mL)} = (\text{Gas pressure (psi)} * 4.8843) + 3.1296$$

The equation was formulated based on the experiment conditions using 160 mL serum bottles and is used for all the experiments for Table 10 and 11

Statistical analysis: For the experiments described in this Example, the data collected was analyzed using the GLIMMIX procedure of SAS (version 9.1; SAS Institute, Cary, NC). Experiments were designed as completely randomized block design where each run was considered a block. Dose was used as fixed effect in the model when enzyme was tested at different doses. Response variable included in-vitro DMD and gas production. Run was considered a random factor. Fixed effects of the model included sampling time for the fermentation parameters measured at different hours post-incubation. The UNIVARIATE procedure of SAS was used to test the residuals for outliers and normality before the final analyses were performed. Treatment effects were declared significant at $P < 0.05$ while any trends were defined at $0.05 \leq P \leq 0.10$.

The results shown in Table 10 below indicate that under the *in vitro* rumen fermentation conditions, the protease ME-3 was able to promote DMD and gas production in a dose response manner.

**Table 10. Effects of exogenous enzyme (ME-3) supplemented at different doses on in-vitro dry matter digestibility, gas production, pH levels, and starch digestibility using dent corn grain as substrate.**

| Variables | ME-3 as mg/g of feed substrate | | | | Statistics | |
|---|---|---|---|---|---|---|
| ME-3 dose | 0 | 0.25 | 0.50 | 0.75 | SE | P-value |
| DMD, % | $13.9^{c}$ | $16.4^{b}$ | $17.8^{ab}$ | $18.7^{b}$ | 0.60 | <0.01 |
| Gas production, mL | $8.77^{b}$ | $11.9^{a}$ | $12.7^{a}$ | 12.8a | 0.44 | <0.01 |
| pH | $6.67^{a}$ | $6.44^{b}$ | $6.38^{b}$ | $6.42^{b}$ | 0.06 | <0.01 |
| Starch digestibility, % | 21.4 | 20.8 | 23.5 | 23.7 | 2.26 | 0.74 |

$^{a\text{-}c}$ Means within a row with different superscripts differ ($P < 0.05$).

**EXAMPLE 14**

**Further evaluation of ME-3 Protease as a Liquefaction Protease**

Liquefaction-SSF studies were performed in which the thermostable serine protease, ME-3 was further tested in lab scale corn liquefaction and simultaneous saccharification and fermentation (SSF). Milled corn flour from a commercial ethanol plant was used to generate a slurry at 33% dry solids by adding a 6:4 w/w mixture of tap water:backset from a commercial ethanol plant to the flour. The pH was adjusted to 5.1 with $H_2SO_4$ and afterwards SPEZYME® HT-WB (DuPont commercial product containing a bacterial alpha amylase) was dosed at a relevant commercial dose. The mixture was incubated at 85°C for 20 minutes. Afterwards the slurry was boiled for 10 minutes at 100°C and subsequently cooled down for 10 minutes at 85°C. Another dose of SPEZYME® HT-WB was added, and in addition the ME-3 protease was added to a final concentration of 3, 4, 5 or 6 ug/gDS. The liquefaction was extended for another 2 hours under constantly stirring using an overhead mixer. A control sample was generated by adding no protease to the liquefaction. Following the incubation, the treated corn sample (Liquefact) was collected and used in a subsequent SSF experiment.

The pH of the liquefact samples (with and without ME-3 protease added to liquefaction reaction) was adjusted to 4.8 and a fungal alpha amylase, a fungal glucoamylase, and a fungal trehalase were added at a relevant dose to each SSF vessel containing 98 grams of liquefact. Afterwards, 2mL of a propagated yeast culture (SYNERXIA® Thrive, Dupont) was added and subsequently urea and FERMGEN™ 2.5x (DuPont commercial product containing an acid fungal protease) were added at different concentrations as indicated in Table 11. The experiments were performed in quadruplicate.

The fermentation vessels were incubated at 32°C in a forced air incubator at 150rpm. Samples were collected at five different time points (16h, 24h, 40h, 48h and 54hours). These samples were analyzed for ethanol concentration, glycerol concentration, acid hydrolyzed fat and crude protein analysis as described below.

For determination of ethanol and glycerol concentration, fermentation samples were centrifuged for 3 minutes 12,000g. 50ul of 1N $H_2SO_4$ was added to 500ul supernatant and left at room temperature for 5 minutes. The sample was diluted 11x in distilled water and filtered through a 0.2$\mu$m filter before HPLC analysis. The HPLC (Thermo Scientific UltiMate 3000) run conditions were as follows: Phenomenex® Rezex™ Organic Acid (ROA), 300 x 7.80 mm column held at 65°C, run at 0.7 mL/min isocratic flow of 0.01N $H_2SO_4$ solvent, 20 $\mu$L injection volume, and 23 min elution runtime. Refractive index detection was used for quantification of ethanol and glycerol. Results are shown in Table 11 (glycerol) and Table 12 (ethanol).

End of fermentation materials were also analyzed at Midwest Laboratories (13611 B Street Omaha, Nebraska 68144) for acid hydrolyzed fat (AOAC 954.02 method was use with the following exceptions: 15mL PET ether and ethyl ether tubes instead of 25mL vessels, and digi tubes instead of mojonnier tubes) and crude protein analysis (protocol AOAC 990.03). Results are shown in Table 11.

Analysis of material obtained from the end of fermentation revealed that ME-3 treatment resulted in higher levels of crude protein and acid-hydrolyzed fat, the latter suggesting potentially higher corn oil yield in an ethanol plant. In addition, glycerol levels were found to be lower for ME-3 treated liquefacts, which is perceived as positive in the industry in combination with the higher ethanol yield.

Table 11 shows the results of measuring the acid hydrolyzed fat, crude protein, and glycerol levels at end of fermentation (54 hours) obtained using different ME-3 doses. ND indicates not determined.

**Table 11. Effect of ME-3 protease addition to liquefaction on residual fat, protein and glycerol content.**

| Urea concentration (ppm) | ME-3 concentration (ug/gDS) | Acid Hydrolyzed Fat (% dry weight) | Crude Protein (% dry weight) | % W/V Glycerol |
|---|---|---|---|---|
| 600 | 0 | 11.5 | 16.8 | 1.55 (+/-0.01) |
| 240 | 3 ME-3 | 12.3 | 17 | 1.51 (+/-0.02) |
| 240 | 4 ME-3 | ND | ND | 1.49 (+/-0.02) |
| 240 | 5 ME-3 | ND | ND | 1.47 (+/-0.01) |
| 240 | 6 ME-3 | 12.4 | 19.4 | 1.48 (+/-0.01) |

The ethanol yield was also determined (as described in Example 7) and results show that when ME-3 was added at various concentrations during liquefaction and subsequent SSF, a clear dose response effect and a faster rate of ethanol production was observed, as reported on Table 12. Furthermore, it was found that ethanol levels at the end of fermentation (54 hours, Table 12) were slightly higher for the ME-3-treated liquefacts.

| Table 12. Ethanol yield | | |
|---|---|---|
| Condition | hrs | % V/V Ethanol |
| 600 ppm urea - 0.07 SAPU/gDS FERMGEN™ 2.5X | 16 | 8.29 (+/-0.22) |
| 3 ug/gDS ME3 - 240 ppm urea | 16 | 8.44 (+/-0.21) |

(continued)

| Table 12. Ethanol yield | | |
|---|---|---|
| Condition | hrs | % V/V Ethanol |
| 4 ug/gDS ME3 - 240 ppm urea | 16 | 8.75 (+/-0.1) |
| 5 ug/gDS ME3 - 240 ppm urea | 16 | 8.93 (+/-0.11) |
| 6 ug/gDS ME3 - 240 ppm urea | 16 | 9.14 (+/-0.07) |
| | | |
| 600 ppm urea - 0.07 SAPU/gDS FERMGEN™ 2.5X | 24 | 11.8 (+/-0.06) |
| 3 ug/gDS ME3 - 240 ppm urea | 24 | 12.16 (+/-0.08) |
| 4 ug/gDS ME3 - 240 ppm urea | 24 | 12.6 (+/-0.14) |
| 5 ug/gDS ME3 - 240 ppm urea | 24 | 12.89 (+/-0.09) |
| 6 ug/gDS ME3 - 240 ppm urea | 24 | 13.13 (+/-0.05) |
| | | |
| 600 ppm urea - 0.07 SAPU/gDS FERMGEN™ 2.5X | 40 | 15.7 (+/-0.1) |
| 3 ug/gDS ME3 - 240 ppm urea | 40 | 15.78 (+/-0.08) |
| 4 ug/gDS ME3 - 240 ppm urea | 40 | 15.96 (+/-0.08) |
| 5 ug/gDS ME3 - 240 ppm urea | 40 | 16.13 (+/-0.03) |
| 6 ug/gDS ME3 - 240 ppm urea | 40 | 16.12 (+/-0.07) |
| | | |
| 600 ppm urea - 0.07 SAPU/gDS FERMGEN™ 2.5X | 48 | 16.16 (+/-0.09) |
| 3 ug/gDS ME3 - 240 ppm urea | 48 | 16.24 (+/-0.07) |
| 4 ug/gDS ME3 - 240 ppm urea | 48 | 16.34 (+/-0.07) |
| 5 ug/gDS ME3 - 240 ppm urea | 48 | 16.47 (+/-0.06) |
| 6 ug/gDS ME3 - 240 ppm urea | 48 | 16.43 (+/-0.08) |
| | | |
| 600 ppm urea - 0.07 SAPU/gDS FERMGEN™ 2.5X | 54 | 16.29 (+/-0.05) |
| 3 ug/gDS ME3 - 240 ppm urea | 54 | 16.4 (+/-0.05) |
| 4 ug/gDS ME3 - 240 ppm urea | 54 | 16.44 (+/-0.06) |
| 5 ug/gDS ME3 - 240 ppm urea | 54 | 16.41 (+/-0.05) |
| 6 ug/gDS ME3 - 240 ppm urea | 54 | 16.41 (+/-0.06) |

In another study, ME-3 dosage was doubled during liquefaction. It was found that subsequent SSF could be performed using the SSF protease at a normal level without the need for any urea. Thus, it was found that not only could all urea be replaced but the rate of ethanol formation as well as final ethanol yield (measured at 53 hours) were increased compared to the control as shown on Table 13.

| Table 13. ME-3 protease effect on ethanol yield when added twice during liquefaction step. | | | | | | |
|---|---|---|---|---|---|---|
| | | | Saccharification time (h) | | | |
| ME-3 (ug/gDS) | Urea (ppm) | FERMGEN™ (SAPU/gDS) | 24 | 30 | 47 | 53 |
| 0 | 600 | 0.07 | 12.6 | 15.1 | 15.4 | 15.7 |
| 3 | 240 | 0 | 12.6 | 15.1 | 15.6 | 15.7 |
| 6 | 0 | 0.07 | 12.8 | 15.4 | 16.1 | 16.4 |

In this study, corn kernels (Arie Blok Animal Nutrition, NL-3440 AA Woerden, Artnr: 3777) were milled using a Retsch ZM200 grinding machine with settings: 3mm screen, 10k rpm. The milled corn flour was used to generate a slurry at 32.64% dry solids by adding a 1:1 mixture of tap water/backset from a commercial ethanol plant to the flour. The pH was adjusted to 5.5 with $H_2SO_4$ and afterwards SPEZYME® RSL (DuPont commercial product containing a bacterial alpha amylase) was dosed at a relevant commercial dose. Subsequently, the ME-3 protease was added to a final concentration of 0, 3 or 6 ug/gDS and the mixture was incubated at 85°C for two hours under constantly stirring using an overhead mixer. A control sample was generated by adding no protease to the liquefaction. Following the incubation, the treated corn sample (Liquefact) was collected and used in a subsequent SSF experiment.
The pH of the liquefact samples (with and without ME-3 protease added to liquefaction reaction) was adjusted to 4.8 and a fungal alpha amylase, a fungal glucoamylase, and a fungal trehalase were added at a relevant dose to each SSF vessel containing 98grams of liquefact. Afterwards, 2ml of a propagated yeast culture (SYNERXIA® Thrive, Dupont) was added and subsequently urea and FERMGEN™ 2.5x (DuPont commercial product containing an acid fungal protease) were added at different concentrations as indicated in Table 13. The experiments were performed in duplicate.
The fermentation vessels were incubated at 32°C in a forced air incubator at 150rpm. Samples were collected at four different time points (24h, 30h, 47h and 53hours). These samples were analyzed for ethanol concentration using HPLC separation and quantitation as described below. Fermentation samples were centrifuged for 7 minutes 15,000g. Supernatant was diluted 11x in 0.01N $H_2SO_4$ and filtered through a 0.22μm filter before HPLC analysis. The HPLC (Agilent Technologies 1200 series) run conditions were as follows: Phenomenex Rezex™ RFQ-Fast Acid H+ column held at 80°C, run at 1.0mL/min isocratic flow of 0.01N $H_2SO_4$ solvent, a 10 μL injection volume, and 5.3 min elution runtime. Refractive index detection was used for quantification of ethanol, and the results are shown in Table 13.
The following numbered paragraphs (paras.) contain further statements of various aspects and embodiments of the present invention:-

1. A method for producing a thermostable serine protease comprising:

(a) transforming a production host with a recombinant construct encoding a thermostable serine protease having at least 80% sequence identity to SEQ ID NOs: 3, 8, or 10; and
(b) culturing the production host of step (a) at a temperature above 37.5°C to increase expression level of the thermostable serine protease.

2. The method according to para. 1 wherein the thermostable serine protease is optionally recovered from the production host.
3. A thermostable serine protease-containing culture supernatant obtained by the method of paras. 1 or 2.
4. A feed, feedstuff, feed additive composition, premix, food or grain product comprising a thermostable serine protease having at least 80% sequence identity to SEQ ID NOs: 3, 8, or 10 wherein said thermostable serine protease can be used alone or in combination with at least one direct fed microbial.
5. Animal feed comprising a thermostable serine protease having at least 80% sequence identity to SEQ ID NO: 1 wherein the thermostable serine protease is present in an amount from 1-20g/ton feed and further wherein said thermostable serine protease can be used alone or in combination with at least one direct fed microbial.
6. The feed additive composition of para. 4 for use in animal feed further comprising 10 and at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.
7. A granulated feed additive composition for use in animal feed comprising a thermostable serine protease having at least 80% sequence identity to SEQ ID NOs: 3, 8, or 10, used either alone or in combination with at least one direct fed microbial, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.
8. A granulated feed additive composition of para. 7, wherein the mean diameter of the particles is greater than 50 microns and less than 2000 microns
9. The feed additive composition of para. 7 wherein said composition is in a liquid form.
10. The feed additive composition of para. 9 wherein said composition is in a liquid form suitable for spray-drying on a feed pellet.
11. A method for hydrolyzing starch-containing material comprising:

(a) contacting starch-containing material with a liquid to form a mash; and
(b) hydrolyzing starch in the mash to form a liquefact by contacting the mash with an enzyme cocktail comprising a thermostable serine protease having at least 80% sequence identity to SEQ ID NOs: 3, 8, or 10.

12. The method of para. 11 wherein the enzyme cocktail further comprises at least one enzyme selected from the group consisting of alpha-amylase, amyloglucosidase, phytase, pullulanase, beta-glucanase, cellulase and xylanase.
13. A method for producing fermentation products from starch-containing material comprising:

(a) liquefying the starch-containing material with an enzyme cocktail comprising a thermostable serine protease having at least 80% sequence identity to SEQ ID NOs: 3, 8, or 10;
(b) saccharifying the product of step (a);
(c) fermenting with a suitable organism; and
(d) optionally, recovering the product produced in step (c).

14. The method of para. 13 wherein steps (b) and (c) are performed simultaneously.
15. The method of para. 13 or para. 14 wherein addition of a nitrogen source is eliminated or reduced by at least 60% by using 1-20 g thermostable serine protease/MT starch-containing material having at least 80% sequence identity to SEQ ID NOs:3, 8, or 10.
16. The method of para. 15 wherein when the fermentation product is ethanol then no acid proteolytic enzyme is needed in step (c) when using 1-20 g thermostable serine protease/MT starch-containing material having at least 80% sequence identity to SEQ ID NOs: 3, 8, or 10.
17. A method for reducing viscosity of a liquefied starch-containing material which comprises contacting a slurry of starch-containing material with a thermostable serine protease having at least 80% sequence identity to SEQ ID NOs: 3, 8, or 10.
18. A method for extracting oil from an oilseed crop comprising:

(a) contacting an oil-containing oilseed fraction with an aqueous extractant to form an extract oilseed fraction; and
(b) separating the extracted oilseed fraction into an aqueous phase, an oil-in-water emulsion, and an insoluble phase;
(c) contacting the oil-in-water emulsion with at least a thermostable serine protease having at least 80% sequence identity to SEQ ID NOs:3, 8, or 10, either alone or in combination with at least a phospholipase, under conditions permitting enzyme activity for a time sufficient to destabilize the emulsion; and
(d) separating the destabilizing emulsion of step (c) into an aqueous phase, an oil phase, and an insoluble phase to obtain oil from the oilseed.

19. The method of para. 18 wherein the oilseed fraction is from soybean, corn seed, rape seed, palm kernel, sunflower seed, safflower seed, coconut, peanut, cotton seed, sesame seed, flax seed, poppy seed, almond, hazelnut, walnut, evening primrose seed, grape seed, hemp seed, black currant seed, red raspberry seed, carrot seed, cumin seed, blueberry seed, cranberry seed, parsley seed, onion seed, pumpkin seed, apricot kernel, mustard seed, linseed, castor seed or jatropha.
20. The process of para. 18 wherein the oilseed fraction comprises a protein fraction that is useful as a food, food ingredient, a food additive or food supplement.
21. A plant-derived oil prepared by the process of para. 18.
22. A feed, feedstuff, feed additive composition, premix, food or grain product comprising the oil of para. 21.
23. A method for preparing a wort comprising:

a) contacting starch-containing material comprising a mix of milled grain with water and a thermostable serine protease having at least 80% sequence identity to SEQ ID NOs: 3, 8, or 10 to form a mash;
b) slowly heating the mash of step (a) to convert the starch to sugars; and
c) separating the heated mash of step (b) into residual grain and liquid wort

wherein the thermostable protease is present in an amount from 1-30 g/ton milled grain.
24. The method of para. 23 wherein at least one alpha- amylase is added in step (a) along with the thermostable serine protease.
25. A method for hydrolyzing at least one food or animal by-product comprising:

(a) contacting the food or animal by-product with a liquid; and

(b) hydrolyzing food or animal by-product in the mash to form a liquefact by contacting with an enzyme cocktail comprising a thermostable serine protease having at least 80% sequence identity to SEQ ID NOs: 3, 8, or 10.

26. The method of para. 25 wherein the food by-product is keratin rich material selected from the group consisting of feather, hair and wool.

27. A method for hydrolyzing proteins in lignocellulosic biomass comprising

(a) contacting the biomass with a liquid; and

(b) hydrolyzing the proteins in the biomass by contacting the biomass with an enzyme cocktail comprising a thermostable serine protease having at least 80% sequence identity to SEQ ID NOs: 3, 8, or 10.

## Claims

**1.** A feed, feedstuff, feed additive composition, premix, food or grain product comprising a thermostable serine protease having at least 80% sequence identity to SEQ ID NOs: 3, 8, or 10.

**2.** The feed, feedstuff, feed additive composition, premix, food or grain product according to claim 1, further comprising at least one direct fed microbial.

**3.** The feed additive composition of claim 1 or claim 2 for use in animal feed further comprising at least one component selected from the group consisting of a protein, a peptide, sucrose, lactose, sorbitol, glycerol, propylene glycol, sodium chloride, sodium sulfate, sodium acetate, sodium citrate, sodium formate, sodium sorbate, potassium chloride, potassium sulfate, potassium acetate, potassium citrate, potassium formate, potassium acetate, potassium sorbate, magnesium chloride, magnesium sulfate, magnesium acetate, magnesium citrate, magnesium formate, magnesium sorbate, sodium metabisulfite, methyl paraben and propyl paraben.

**4.** A granulated feed additive composition for use in animal feed comprising a thermostable serine protease having at least 80% sequence identity to SEQ ID NOs: 3, 8, or 10, wherein the granulated feed additive composition comprises particles produced by a process selected from the group consisting of high shear granulation, drum granulation, extrusion, spheronization, fluidized bed agglomeration, fluidized bed spray coating, spray drying, freeze drying, prilling, spray chilling, spinning disk atomization, coacervation, tableting, or any combination of the above processes.

**5.** A granulated feed additive composition of claim 4, wherein the mean diameter of the particles is greater than 50 microns and less than 2000 microns.

**6.** A composition comprising a granulated feed additive composition according to claim 4 or claim 5 and a direct fed microbial.

**7.** The feed additive composition of any one of claims 1 to 3 wherein said composition is in a liquid form.

**8.** The feed additive composition of claim 7 wherein said composition is in a liquid form suitable for spray-drying on a feed pellet.

**9.** A method of pelleting a feed composition, comprising pre-treating feed with a thermostable serine protease having at least 80% sequence identity to SEQ ID NOs: 3, 8, or 10 and steam pelleting the pre-treated feed.

Term - lambda
aprE promoter region
signal peptide
aprE (modified)
Tet R
Tce01961 pro-mature
pHYT-Tce01961
6828 bp
Terminator
Rep ori (pAMalpha-1)
RNA primer
(p15A ori - pACYC177)
Gram+ Origin 2
Amp R (Tn3 from pACYC177)

*FIG. 1*

0.6
0.5
0.4
0.3
0.2
0.1
0
Adsorbance at 340 nm
Control
500 ppm
1000 ppm
1500 ppm
Enzyme dose response in the presence of pepsin and pancreatin

*FIG. 2*

120
100
80
60
40
20
0
Residual activity of Tce01961, %
70
75
80
85
90
95
100
Temperature, °C

*FIG. 3*

Relative activity
100%
75%
50%
25%
0%
3.5
4.5
5.5
6.5
7.5
pH

*FIG. 4*

ME3 protease activity
100.00%
75.00%
50.00%
25.00%
0.00%
mash
90 degC
95 degC
100 degC

*FIG. 5*

Cumulative pressure
0
20
40
60
80
100
120
140
160
0
5
10
15
20
25
30
35
40
45
50
55
60
65
Time (h)

*FIG. 6*

220
200
180
160
140
120
100
FAN mg/L
137
165
179
199
0
2.5
4
6
ppm Protease ME-3

*FIG. 7*

```
              1        10        20        30        40        50        60
              |        |         |         |         |         |         |
       ME3    ADVIGGNPYYFG--GY-RCSIGFSVRKGSDTGFATAGHCGETGTLTRSP----EGVVAGS
      Tfpa    AAIIGGNPYYFG--NY-RCSIGFSVRQGSQTGFATAGHCGSTGTRVSSP----SGTVAGS
      Thpa    -DIIGGNPYYFD--GY-RCSIGFSVRRGSESGFATAGHCGEEGTETSDP----EGTVAGA
WP078763344   ADIIGGNKNSLNAAGTSYCSVGFAV----QGGFATAGHCGSVGATSYSP----AGTFAGS
WP091675221   -DIIGGNAYTFG--QG-RCSIGFSV----EGGFVTAGHCGRTGTRTQNP----SGTVAGS
WP017594871   GDIVGGNAYY---PGGSRCSIGFSV----QGGFATAGHCGSQGTRVTGGAG-ESGTVAGS
WP069846166   -DVRGGDAYYFG--GS-RCSIGFSV----NGGYVTAGHCGGVGTATQGSNRVASGSVAGS
WP067965505   AQIIGGNAYYIN--NSGRCSIGFAV----TGGFVTAGHCGRVGATATSSDG--RGTFRGS
WP033441214   -DVIGGNAYYIG--SGSRCSVGFAV----DGGFVTAGHCGRTGASTTQP----SGTFAGS

       ME3    YFPGRDMGWVRLTGADTVTPLVNRYDGGTVTVTGSQEAVTGSSVCRSGSTTGWRCGIIQS
      Tfpa    YFPGRDMGWVRITSADTVTPLVNRYNGGTVTVTGSQEAATGSSVCRSGATTGWRCGTIOS
      Thpa    YFPGRDMGWVRITDADTVTPLVNRYNGENVTVAGSREAATGSSVCRSGSTTGWRCGTIRS
WP078763344   IFPSRDMGWVRTTSAWVPRPYVNNYSGGTVTVRGSQEAAIGASVCRSGATTGWRCGVIQS
WP091675221   SFPGNDYAWVRTSSGNTPRPLVNRYPG-TVPVAGSQEAPVGSSVCRSGSTTGWRCGTIQQ
WP017594871   IFPGRDMGWVRVNSGWNPSPYVNNYSGGRVLVTGSQEASVGASICRSGSTTGWHCGTIQA
WP069916166   VFPCSDMGWVRTNSNWVPRGVVNRYNGGTVAVAGSTEAAIGASICRSGSTTGWHCGTIQA
WP067965505   IFPGRDMGWVGNTTNWTTTPYVNNYSGGRVTVTGSSEAATGSSICRSGSTTGWHCGSIQS
WP033441214   SFPGNDYAWVRTAAGNTGRPLVNRYPG-TVPVAGSTEAPVGASVCRSGSTTGWRCGTIQQ

       ME3    KNQTVRYAEGTVTGLTRTTACAEAGDSGGPWLTGSQAQGVTSGGSGNCRTGGITYFQPIN
      Tfpa    KNQTVRYAEGTVTGLTRTTACAEGGDSGGPWLTGSQAQGVTSGGTGDCRSGGITFFQPIN
      Thpa    KNQTVRYIEGTVTGLTRTTACAEGGDSGGPWLTGSQGQGVTSGGSGNCTLGGVTYFQPLN
WP078763344   KNQTVRYAQGAVYGLTRTTACAEPGDSGGSWLSGNQAQGVTSGGSGNCRTGGTTYFQPIN
WP091675221   KNASVTYPEGTJYGLTRTNACAEPGDSGGSWLTGDQAOGVTSGGSGNCRTGGTIYFQPVN
WP017594871   KNQTVRYPQGTVNGLTRTNVCAEPGDSGGSWISGSQAQGVTSGGSGNCSTGGTTFYQPIN
WP069846166   KNQTVNYAQGAVRGMTRTNACAEGGDSGGSWLSGSOAQGVTSGGSGNCSFGGTTYFQPVN
WP067965505   KNQTVTYPQGTVSGLTRTNVCAEPGDSGGSWVTGSQAQGVTSGGSGNCRTGGTTFYQPVN
WP033441214   KNASVTYPEGTITGLTRTNACAEPGDSGGSWLTGDQAQGVTSGGSGNCTSGGTIYFQPVN

       ME3    PLLSYFGLELVTG
      Tfpa    PLLSYFGLQLVTG
      Thpa    PLLSHFDLDLVTG
WP078763344   PILSQWGLTLVTG
WP091675221   EILSVYNLNLV--
WP017594871   PILSQWGLTLTTG
WP069846166   PILSRYGLSLVRG
WP067965505   PILSQWGLTLVRG
WP033441214   EILQVYGLRLV--
```

*FIG. 8*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62437340 A
- US 7932063 B
- US 6255076 B
- US 5955310 A
- WO 200151643 A
- WO 2013086219 A
- WO 200214490 A
- US 5264366 A
- EP 238023 A
- US 6022725 A
- US 6268328 B
- US 6255115 B
- WO 2012110778 A
- US 7754469 B
- US 20090280090 A
- WO 2013029013 A
- WO 2007044968 A
- WO 1997016076 A
- WO 1992012645 A
- US 8927250 B
- US 7354752 B
- US 8144046 B
- US 8673609 B
- US 8053221 B
- US 5817498 A
- EP 0063909 A
- US 5055403 A
- WO 201076113 A
- US 5000000 A
- US 5028539 A
- US 5424202 A
- US 5514583 A
- US 5554520 A
- WO 9513362 A
- US 4316956 A
- WO 2010113129 A
- WO 2010113130 A
- US 8545633 B
- WO 2012103220 A
- US 20080008783 A
- US 7781191 B
- US 7998713 B
- US 7915017 B
- WO 2011038019 A
- WO 2012125937 A
- US 20140106408
- WO 201517256 A1
- WO 201517255 A1
- WO 201517254 A1
- US 2014016408 A
- WO 03027306 A
- WO 200352118 A2
- WO 200352054 A2
- WO 200352057 A2
- WO 200352055 A2
- WO 200352056 A2
- WO 200416760 A2
- WO 9210581 A1
- WO 200448592 A2
- WO 200443980 A2
- WO 200528636 A2
- WO 200501065 A2
- WO 2005001036 A
- WO 2005093050 A
- WO 200593073 A1
- WO 200674005 A2
- WO 2009149202 A
- WO 2010141779 A
- WO 2011063308 A
- WO 2012125951 A
- WO 2012125925 A
- WO 2011153276 A
- WO 2014093275 A
- WO 2014070837 A
- WO 2014070841 A
- WO 2014070844 A
- WO 2014093281 A
- WO 2014093282 A
- WO 2014093287 A
- WO 2014093294 A
- WO 2015084596 A
- WO 2016069541 A
- US 5580389 A
- WO 2011002660 A
- US 7629156 B
- US 8247208 B
- US 8669076 B
- WO 2006110901 A2
- WO 20061019 A2
- WO 2006110901 A
- US 2010227042 A
- US 2015197783 A
- WO 2014202716 A

**Non-patent literature cited in the description**


- **JONES et al.** *EMBO J*, 1985, vol. 4, 2411-2418
- **DE ALMEIDA et al.** *Mol Gen Genetics*, 1989, vol. 218, 78-86
- Computational Molecular Biology. Oxford University Press, 1988
- Biocomputing: Informatics and Genome Projects. Academic Press, 1993
- Computer Analysis of Sequence Data. Humana Press, 1994
- Sequence Analysis in Molecular Biology. Academic Press, 1987
- Sequence Analysis Primer. Stockton Press, 1991
- **ALTSCHUL et al.** *J Mol Biol*, 1990, vol. 215, 403-410
- **KARLIN** ; **ALTSCHUL**. *Proc Natl Acad Sci USA*, 1993, vol. 90, 5873-5787
- **ALTSCHUL et al.** *Nucleic Acids Res*, 1997, vol. 25, 3389-3402
- **SCHAFFER et al.** *Nucleic Acids Res*, 2001, vol. 29, 2994-3005
- **ALTSCHUL SF** ; **MADDE TL** ; **SHAFFER AA** ; **ZHANG J** ; **ZHANG Z** ; **MILLER W** ; **LIPMAN DJ**. Gapped BLAST and PSI BLAST a new generation of protein database search programs. *Nucleic Acids Res*, 1997, vol. 25 (17), 3389-402
- **RICE et al.** *Trends in Genetics*, 2000, vol. 16 (6), 276-277
- **HIGGINS** ; **SHARP**. *CABIOS*, 1989, vol. 5, 151-153
- **HIGGINS et al.** *Nucleic Acids Res.*, 1994, vol. 22, 4673-4680
- **CHENNA et al.** *Nucleic Acids Res*, 2003, vol. 31 (13), 3497-500
- *Nucl. Acids Res.*, 2004, vol. 32 (5), 1792-1797
- **GRAESSLE et al.** *Appl. Environ. Microbiol.*, 1997, vol. 63, 753-756
- **LEUKER et al.** *Gene*, 1997, vol. 192, 235-240
- **LIU et al.** *Eukary. Cell*, 2006, vol. 5, 638-649
- **NUNBERG et al.** *Mol. Cell Biol.*, 1984, vol. 15 (4), 2306-2315
- **BOEL et al.** *EMBO J.*, 1984, vol. 3, 1581-1585
- **VILLA-KAMAROFF et al.** *Proceedings of the National Academy of Sciences USA*, 1978, vol. 75, 3727-3731
- **RYGUS** ; **HILLEN**. *J. Bacteriol.*, 1992, vol. 174, 3049-3055
- **KIM et al.** *Gene*, 1996, vol. 181, 71-76
- **DEBOER et al.** *Proceedings of the National Academy of Sciences USA*, 1983, vol. 80, 21-25
- *Scientific American*, 1980, vol. 242, 74-94
- **SAMBROOK** ; **FRITSCH** ; **MANIATIS**. Molecular Cloning, A Laboratory Manual. Cold Spring Harbor, 1989
- **VOSKUIL et al.** *Molecular Microbiology*, 1995, vol. 17, 271-279
- **HUE et al.** *Journal of Bacteriology*, 1995, vol. 177, 3465-3471
- **STEIGER et al.** *Appl. Environ. Microbiol.*, 2011, vol. 77, 114-121
- **LORITO** ; **HAYES** ; **DIPIETRO** ; **HARMAN**. *Curr. Genet.*, 1993, vol. 24, 349-356
- **GOLDMAN** ; **VANMONTAGU** ; **HERRERA-ESTRELLA**. *Curr. Genet*, 1990, vol. 17, 169-174
- **PENTTILA** ; **NEVALAINEN** ; **RATTO** ; **SALMINEN** ; **KNOWLES**. *Gene*, 1987, vol. 6, 155-164
- The Molecular Biology of Trichoderma and its Application to the Expression of Both Homologous and Heterologous Genes. **NEVALAINEN et al.** Molecular Industrial Mycology. Marcel Dekker Inc., 1992, 129-148
- **YELTON** ; **HAMER** ; **TIMBERLAKE**. *Proc. Natl. Acad. Sci. USA*, 1984, vol. 81, 1470-1474
- **BAJAR** ; **PODILA** ; **KOLATTUKUDY**. *Proc. Natl. Acad. Sci. USA*, 1991, vol. 88, 8202-8212
- **HOPWOOD et al.** Genetic Manipulation of Streptomyces: Laboratory Manual. The John Innes Foundation, 1985
- **FERNANDEZ-ABALOS et al.** *Microbiol*, 2003, vol. 149, 1623-1632
- **BRIGIDI** ; **DEROSSI** ; **BERTARINI** ; **RICCARDI** ; **MATTEUZZI**. *FEMS Microbiol. Lett.*, 1990, vol. 55, 135-138
- **SCOPES**. *Protein Purification*, 1982
- Proteinases. **WARD**. Microbial Enzymes and Biotechnology. Applied Science, 1983, 251-317
- **TWINING, S.S.** Fluorescein Isothiocyanate-Labeled Casein Assay for Proteolytic Enzymes. *Anal. Biochem.*, 1984, vol. 143, 30-34
- **WELLS et al.** *Nucleic Acids Res.*, 1983, vol. 11, 7911-7925
- **CHRISTIANSON et al.** *Anal. Biochem.*, 1994, vol. 223, 119-129
- **HSIA et al.** *Anal Biochem.*, 1999, vol. 242, 221-227
- TO THE ALCOHOL TEXTBOOK: A REFERENCE FOR THE BEVERAGE, FUEL AND INDUSTRIAL ALCOHOL INDUSTRIES 3rd ED. Nottingham University Press, 1999
- The Alcohol Textbook 3rd ED, A Reference for the Beverage. Fuel and Industrial Alcohol Industries. Nottingham University Press, 1999
- *Microbiol. Mol. Biol. Rev.*, 2002, vol. 66, 506-577
- Enzyme Nomenclature. Academic Press, 1992
- *Eur. J. Biochem.*, 1994, vol. 223 (1-5)
- *Eur. J. Biochem.*, 1995, vol. 232, 1-6
- *Eur. J. Biochem.*, 1996, vol. 237, 1-5
- *Eur. J. Biochem.*, 1997, vol. 250, 1-6

- *J. Biochem.*, 1999, vol. 264, 610-650
- **CANTAREL et al.** *Nucleic Acids Res.*, 2009, vol. 37, D233-238
- **SCHMIDT**. *Biochemistry*, 1999, vol. 38, 2403-2412
- **LO LEGGIO et al.** *FEBS Lett*, 2001, vol. 509, 303-308
- **HAKOUVAINEN et al.** *Biochemistry*, 1996, vol. 35, 9617-24
- **DUNSON et al.** *TREATMENT OF BIOMASS TO OBTAIN FERMENTABLE SUGARS*
- **FREITAS et al.** *Fett/Lipid*, 1997, vol. 99, 333-337
- **CAETANO et al.** *La Rivista Italiana Delle Sostanze Grasse*, 2002, vol. 79, 165-169
- **SINGLETON et al.** DICTIONARY OF MICROBIOLOGY AND MOLECULAR BIOLOGY, 2D ED.. John Wiley and Sons, 1994
- **HALE** ; **MARHAM**. THE HARPER COLLINS DICTIONARY OF BIOLOGY. Harper Perennial, N.Y., 1991
- **EMANUELSSON et al.** *Nature Protocols*, 2007, vol. 2, 953-971
- **SHAW et al.** *Acta Crystallogr Sect F Struct Biol Cryst Commun*, 2007, vol. 63, 266-269
- **KELCH BA** ; **AGARD DA**. *J Mol Biol.*, 2007, vol. 370, 784-795
- **P.M. NIELSEN** ; **D. PETERSEN** ; **C. DAMBMANN**. Improved Method for Determining Food Protein Degree of Hydrolysis. *Journal of Food Science*, 2001, vol. 66, 642-646
- **S. YU** ; **A. COWIESON** ; **C. GILBERT** ; **P. PLUMSTEAD** ; **S. DALSGAARD**. *J. Anim. Sci.*, 2012, vol. 90, 1824-1832
- **PEDERSEN et al.** Comparison of four feed proteases for improvement of nutritive value of poultry feather meal. *J. Anim. Sci.*, 2012, vol. 90, 350-352
- **KELCH,B.A** ; **AGARD,D.A.** *J. Mol. Biol.*, 2007, vol. 370, 784-795
- **ROBERT C. EDGAR**. *MUSCLE: multiple sequence alignment with high accuracy and high throughput Nucl. Acids Res*, 2004, vol. 32 (5), 1792-1797
- **ADESOGAN, A.T.** ; **KRUEGER, N.A.** ; **KIM, S.C.** *Anim. Feed Sci. Technol.*, 2005, vol. 123, 211-223
- **KRUEGER, N. A.** ; **A. T. ADESOGAN.** *Anim. Feed Sci. Tech.*, 2008, vol. 145, 84-94
- **GOERING, H. K.** ; **P. J. VAN SOEST.** Agric. Handbook. ARS USDA, 1970, 20